# EUROPÄISCHE PATENTSCHRIFT

(11) **EP 0 748 795 B1**
(45) Veröffentlichungstag und Bekanntmachung des Hinweises auf die Patenterteilung: **23.08.2000**
(21) Anmeldenummer: 96107586.8
(22) Anmeldetag: 13.05.1996
(51) Int. Cl.: C07C 279/24, C07D 213/30, C07D 215/20, C07C 317/22, C07C 317/32, A61K 31/27, A61K 31/17, A61K 31/155, A61K 31/44, A61K 31/47

(54) **Substituierte Benzyloxycarbonylguanidine als Natrium-protonen Antiporter-Inhibitoren, Verfahren zu ihrer Herstellung, ihre Verwendung als Medikament oder Diagnostikum, sowie sie enthaltendes Medikament**
Substituted benzyloxycarbonyl guanidines as Na+/H+ exchange inhibitors, process for their preparation, their use as medicinal or diagnostic agents as well as medicament containing them
Benzyloxyguanidines substituées comme inhibiteurs de l'échange Na+/H+, leur procédé de préparation, leur utilisation comme médicament ou agent diagnostique ainsi que médicament les contenant

(30) Priorität: 17.05.1995 DE 19518073
(43) Veröffentlichungstag der Anmeldung: 18.12.1996
(73) Patentinhaber: HOECHST AKTIENGESELLSCHAFT, 65929 Frankfurt am Main (DE)
(72) Erfinder: Kleemann, Heinz-Werner, Dr., 65474 Bischofsheim (DE); Brendel, Joachim, Dr., 61118 Bad Vilbel (DE); Schwark, Jan-Robert, Dr., 65929 Frankfurt (DE); Weichert, Andreas, Dr., 63329 Egelsbach (DE); Lang, Hans Jochen, Dr., 65719 Hofheim (DE); Albus, Udo, Dr., 61197 Florstadt (DE); Scholz, Wolfgang, Dr., 65760 Eschborn (DE)

(56) Entgegenhaltungen:
- EP-A- 0 416 499
- EP-A- 0 640 588
- BE-A- 844 832

## Beschreibung

Die Erfindung betrifft Benzyloxycarbonylguanidine der Formel I 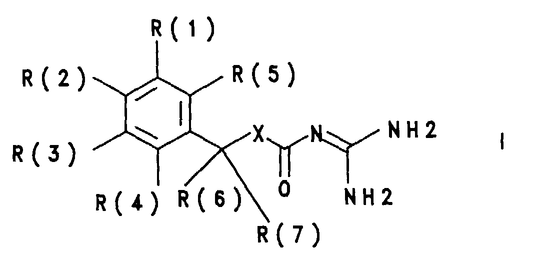 worin bedeuten:
R(1), R(2) und R(3)
unabhängig voneinander -Y-[4-R(8)-Phenyl], -Y-[3-R(8)-Phenyl] oder -Y-[2-R(8)-Phenyl],
wobei das Phenyl jeweils unsubstituiert oder substituiert ist mit 1 - 2 Substituenten aus der Gruppe bestehend aus F, Cl, -CF₃, Methyl, Hydroxy, Methoxy und -NR(96)R(97); R(96) und R(97)
unabhängig voneinander Wasserstoff oder -CH₃;
Y eine Bindung, CH₂, Sauerstoff, -S- oder -NR(9);
R(9) Wasserstoff oder Alkyl mit 1, 2, 3 oder 4 C-Atomen;
R(8) SOₐ[NR(98)]_{b}NR(99)R(10);
a 1 oder 2;
b 0 oder 1;
a + b = 2;
R(98), R(99) und R(10)
unabhängig voneinander Wasserstoff, -(C₁-C₈)-Alkyl, Benzyl, -(C₂-C₈)-Alkylen-NR(11)R(12), (C₂-C₈)-Alkylen-NR(13)-(C₂-C₈)-Alkylen-NR(37)R(38) oder (C₀-C₈)-Alkylen-CR(39)R(40)-CR(41)R(42)(C₀-C₈)-Alkylen-NR(43)R(44);
R(11), R(12), R(13), R(37), R(38), R(43) und R(44)
unabhängig voneinander Wasserstoff, -(C₁-C₈)-Alkyl oder Benzyl:
R(39), R(40), R(41) und R(42)
unabhängig voneinander Wasserstoff, -(C₁-C₈)-Alkyl oder -(C₀-C₃)-Alkylen-Phenyl,
wobei das Phenyl nicht substituiert oder substituiert ist mit 1 - 3 Substituenten ausgewählt aus der Gruppe bestehend aus F, Cl,-CF₃, Methyl und Methoxy;
oder
R(99) und R(10)
gemeinsam 4 - 6 Methylengruppen, von denen eine CH₂-Gruppe durch Sauerstoff, -S-, -NH-, -N-CH₃ oder -N-Benzyl ersetzt sein kann;
oder
R(8) SOₐ[NR(98)]_{b}NR(95)-C[=N-R(94)]-NR(93)R(92);
R(92), R(93), R(94) und R(95)
unabhängig voneinander Wasserstoff oder Alkyl mit 1, 2, 3 oder 4 C-Atomen;
oder
R(1), R(2) und R(3)
unabhängig voneinander Wasserstoff, -(C₁-C₈)-Alkyl, -(C₂-C₈)-Alkenyl oder -(CH₂)ₘR(14);
m Null, 1 oder 2;
R(14) -(C₃-C₈)-Cycloalkyl oder Phenyl,
welches nicht substituiert oder substituiert ist mit 1 - 3 Substituenten ausgewählt aus der Gruppe bestehend aus F und Cl, -CF₃, Methyl, Methoxy und -NR(15)R(16);
R(15) und R(16)
Wasserstoff oder -CH₃;
oder
R(1), R(2) und R(3)
unabhängig voneinander -Q-4-[(CH₂)ₖ-CHR(17)-(C=O)R(20)]-Phenyl, -Q-3[(CH₂)ₖ-CHR(17)-(C=O)R(20)]-Phenyl oder -Q-2-[(CH₂)ₖ-CHR(17)-(C=O)R(20)]-Phenyl,
wobei das Phenyl jeweils unsubstituiert oder substituiert ist mit 1 - 2 Substituenten aus der Gruppe F, Cl, -CF₃, Methyl, Hydroxy, Methoxy und -NR(35)R(36);
R(35) und R(36)
unabhängig voneinander Wasserstoff oder -CH₃;
Q eine Bindung, Sauerstoff, -S- oder -NR(18);
R(18) Wasserstoff oder -(C₁-C₄)-Alkyl;
R(17) -OR(21) oder -NR(21)R(22);
R(21) und R(22)
unabhängig voneinander Wasserstoff, -(C₁-C₈)-Alkyl, -(C₁-C₈)-Alkanoyl, -(C₁-C₈)-Alkoxycarbonyl, Benzyl, Benzyloxycarbonyl;
oder
R(21) Trityl;
R(20) -OR(23) oder -NR(23)R(24);
R(23) und R(24) unabhängig voneinander
Wasserstoff, -(C₁-C₈)-Alkyl oder Benzyl;
k Null, 1, 2, 3 oder 4;
oder
R(1), R(2) und R(3)
unabhängig voneinander (C₁-C₉)-Heteroaryl,
das über C oder N verknüpft ist und das unsubstituiert oder substituiert ist mit 1-3 Substituenten aus der Gruppe F, Cl, CF₃, CH₃, Methoxy, Hydroxy, Amino, Methylamino und Dimethylamino;
oder
R(1), R(2) und R(3)
-SR(25), -OR(25), -NR(25)R(26), -CR(25)R(26)R(27);
R(25) -C_{f}H_{2f}-(C₁-C₉)-Heteroaryl,
das unsubstituiert oder substituiert ist mit 1-3 Substituenten aus der Gruppe F, Cl, CF₃, CH₃, Methoxy, Hydroxy, Amino, Methylamino und Dimethylamino;
f Null, 1 oder 2;
R(26) und R(27)
unabhängig voneinander wie R(25) definiert oder Wasserstoff oder (C₁-C₄)-Alkyl;
oder
R(1), R(2) und R(3)
unabhängig voneinander (C₁-C₉)-Heteroaryl-N-Oxid,
das über C oder N verknüpft ist und das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
oder
R(1), R(2) und R(3)
unabhängig voneinander -SR(28), -OR(28), -NR(28)R(29) oder -CR(28)R(29)R(30);
R(28) -C_{g}H_{2g}-(C₁-C₉)-Heteroaryl-N-Oxid,
das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
g Null, 1 oder 2;
R(29) und R(30)
unabhängig voneinander wie R(28) definiert, Wasserstoff oder (C₁-C₄)-Alkyl;
oder
R(1), R(2) und R(3)
unabhängig voneinander Wasserstoff, F, Cl, Br, l, -CN, T-(CH₂)ₕ-(CᵢF₂ᵢ₊₁), R(31)SOₗ-, R(32)R(33)N-CO-, R(34)-CO- oder R(45)R(46)N-SO₂, wobei die Perfluoralkylgruppe geradkettig oder verzweigt ist;
T eine Bindung, Sauerstoff, -S- oder -NR(47);
l Null, 1 oder 2;
h Null, 1 oder 2;
i 1, 2, 3, 4, 5 oder 6;
R(31), R(32), R(34) und R(45)
unabhängig voneinander -(C₁-C₈)-Alkyl, -(C₃-C₆)Alkenyl, (CH₂)ₙR(48) oder -CF₃;
n Null, 1, 2, 3 oder 4;
R(47) Wasserstoff oder Alkyl mit 1, 2 oder 3 C-Atomen;
R(48) -(C₃-C₇)-Cycloalkyl oder Phenyl,
welches nicht substituiert ist oder substituiert mit 1 - 3 Substituenten ausgewählt aus der Gruppe bestehend aus F, Cl, -CF₃, Methyl, Methoxy und -NR(49)R(50);
R(49) und R(50)
Wasserstoff oder Alkyl mit 1, 2, 3 oder 4 C-Atomen;
oder
R(32), R(34) und R(45)
Wasserstoff;
R(33) und R(46)
unabhängig voneinander Wasserstoff oder Alkyl mit 1, 2, 3 oder 4 C-Atomen;
oder
R(32) und R(33) sowie R(45) und R(46)
gemeinsam 5 oder 6 Methylengruppen, von denen eine CH₂-Gruppe durch Sauerstoff, -S-, -NH-, -NCH₃ oder -N-Benzyl ersetzt sein kann;
oder
R(1), R(2) und R(3)
unabhängig voneinander R(51)-A-G-D-;
R(51) ein basischer protonierbarer Rest, d.h. eine Aminogruppe -NR(52)R(53), eine Amidinogruppe R(52)R(53)N-C[=N-R(54)]- oder eine Guanidinogruppe R(52)R(53)N-C[=N-R(54)]-NR(55)-;
R(52), R(53), R(54) und R(55)
unabhängig voneinander Wasserstoff oder Alkyl mit 1, 2, 3 oder 4 C-Atomen;
oder
R(52) und R(53)
eine Gruppe C_{α}H_{2α};
α 4, 5, 6 oder 7;
wobei im Falle von α = 5, 6 oder 7 ein C-Atom der Gruppe C_{α}H_{2α} durch eine Heteroatomgruppe O, SO_{d} oder NR(56) ersetzt sein kann,
oder
R(53) und R(54) oder R(54) und R(55) oder R(52) und R(55)
eine Gruppe C_{γ}H_{2γ};
γ 2, 3, 4 oder 5;
wobei im Falle von γ = 3, 4 oder 5 ein C-Atom der Gruppe C_{γ}H_{2γ} durch eine Heteroatomgruppe O, SO_{d} oder NR(56) ersetzt sein kann;
d Null, 1 oder 2;
R(56) Wasserstoff oder Methyl;
oder
R(51) ein basisches heteroaromatisches Ringsystem mit 1 - 9 C-Atomen;
A eine Gruppe CₑH₂ₑ;
e Null, 1, 2, 3, 4, 5, 6, 7, 8, 9 oder 10;
wobei in der Gruppe CₑH₂ₑ ein C-Atom durch eine der Gruppierungen -O-, -CO- oder SOᵣ- ersetzt sein kann;
r Null, 1 oder 2;
G einen Phenylenrest, 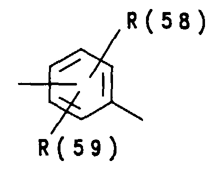 R(58) und R(59)
unabhängig voneinander Wasserstoff, Methyl, Methoxy, F, Cl, Br, J, CF₃ oder -SOₛ-R(60);
R(60) Methyl oder NR(61)R(62);
R(61) und R(62)
unabhängig voneinander Wasserstoff oder Alkyl mit 1, 2, 3 oder 4 C-Atomen;
D -CᵥH₂ᵥ-E_{w}-;
v Null, 1, 2, 3 oder 4;
E -O-, -CO-, -CH[OR(63)]-, -SOₐₐ- oder -NR(63)-;
w Null oder 1;
aa Null, 1 oder 2
R(63) Wasserstoff oder Methyl,
oder
R(1), R(2) und R(3)
unabhängig voneinander -CF₂R(64), -CF[R(65)][R(66)], -CF[(CF₂)_{q}-CF₃)][R(65)], -C[(CF₂)ₚ-CF₃]=CR(65)R(66);
R(64) Alkyl mit 1,2,3 oder 4 C-Atomen oder Cycloalkyl mit 3, 4, 5, 6 oder 7 C-Atomen;
R(65) und R(66) unabhängig voneinander Wasserstoff oder Alkyl mit 1, 2, 3 oder 4 C-Atomen;
q Null, 1 oder 2;
p Null, 1 oder 2;
oder
R(1), R(2) und R(3)
unabhängig voneinander -OR(67) oder -NR(67)R(68);
R(67) und R(68)
unabhängig voneinander Wasserstoff oder Alkyl mit 1, 2, 3, 4, 5 oder 6 C-Atomen;
oder
R(67) und R(68)
gemeinsam 4, 5, 6 oder 7 Methylengruppen, von denen eine CH₂-Gruppe durch Sauerstoff, -S-, SO₂, -NH-, -NCH₃ oder -N-Benzyl ersetzt sein kann;
R(4) und R(5)
unabhängig voneinander Wasserstoff, Alkyl mit 1, 2, 3 oder 4 C-Atomen, F, Cl, -OR(69), -NR(70)R(71) oder -C_{z}F_{2z+1};
R(69), R(70) und R(71)
unabhängig voneinander Wasserstoff oder Alkyl mit 1, 2 oder 3 C-Atomen;
z 1, 2, 3 oder 4;
R(6) und R(7)
unabhängig voneinander Wasserstoff oder Alkyl mit 1, 2, 3 oder 4 C-Atomen;
X Sauerstoff oder NR(72);
R(72) Wasserstoff oder Alkyl mit 1, 2, 3 oder 4 C-Atomen;
sowie deren pharmazeutisch verträgliche Salze;
wobei jedoch Verbindungen ausgenommen sind, in denen die Reste R(1) bis R(7) sowie R(72) gleich Wasserstoff sind.

Bevorzugt sind Verbindungen der Formel I, in denen bedeuten:
R(1), R(2) und R(3)
unabhängig voneinander -Y-[4-R(8)-Phenyl], -Y-[3-R(8)-Phenyl] oder -Y-[2-R(8)-Phenyl],
wobei das Phenyl jeweils unsubstituiert oder substituiert ist mit einem Substituenten aus der Gruppe bestehend aus F, Cl, -CF₃, Methyl, Methoxy und -NR(96)R(97);
R(96) und R(97)
unabhängig voneinander Wasserstoff oder -CH₃;
Y eine Bindung, Sauerstoff, -S- oder -NR(9);
R(9) Wasserstoff oder Methyl;
R(8) SOₐ[NR(98)]_{b}NR(99)R(10);
a 1 oder 2;
b 0 oder 1;
a + b = 2;
R(98) Wasserstoff, Alkyl mit 1, 2, 3, 4 oder 5 C-Atomen oder Benzyl;
R(99) und R(10)
unabhängig voneinander Wasserstoff, Alkyl mit 1 oder 2 C-Atomen, Benzyl, -(C₂-C₃)-Alkylen-NR(11)R(12), (C₂-C₃)-Alkylen-NR(13)-(C₂-C₃)-Alkylen-NR(37)R(38) oder (C₀-C₂)-Alkylen-CR(39)R(40)-CR(41)R(42)(C₀-C₂)-Alkylen-NR(43)R(44);
R(11), R(12), R(13), R(37), R(38), R(43) und R(44)
unabhängig voneinander Wasserstoff, Methyl oder Ethyl;
R(39), R(40), R(41) und R(42)
unabhängig voneinander Wasserstoff, Alkyl mit 1, 2, 3 oder 4 C-Atomen oder Benzyl,
wobei das Phenyl nicht substituiert oder substituiert ist mit einem Substituenten ausgewählt aus der Gruppe bestehend aus F, Cl,-CF₃, Methyl und Methoxy;
oder
R(99) und R(10)
gemeinsam 4, 5 oder 6 Methylengruppen, von denen eine CH₂-Gruppe durch Sauerstoff, -S-, -NH-, oder -N-CH₃ ersetzt sein kann;
oder
R(8) SOₐ[NR(98)]_{b}NR(95)-C[=N-R(94)]-NR(93)R(92);
R(95) Wasserstoff;
R(92), R(93) und R(94)
unabhängig voneinander Wasserstoff oder Alkyl mit 1, 2, 3 oder 4 C-Atomen;
oder
R(1), R(2) und R(3)
unabhängig voneinander Wasserstoff oder Alkyl mit 1, 2, 3 oder 4 C-Atomen;
oder
R(1), R(2) und R(3)
unabhängig voneinander -Q-4-[(CH₂)ₖ-CH(NR(21)R(22))-(C=O)R(20)]-Phenyl, -Q-3-[(CH₂)ₖ-CH(NR(21)R(22))-(C=O)R(20)]-Phenyl oder -Q-2-[(CH2)k-CH(NR(21)R(22))-(C=O)R(20)]-Phenyl,
wobei das Phenyl jeweils unsubstituiert oder substituiert ist mit 1 - 2 Substituenten aus der Gruppe F, Cl, -CF₃, Methyl, Hydroxy, Methoxy und -NR(35)R(36);
R(35) und R(36)
unabhängig voneinander Wasserstoff oder -CH₃;
Q eine Bindung, Sauerstoff, -S- oder -NR(18);
R(18) Wasserstoff oder -(C₁-C₄)-Alkyl;
R(21) und R(22)
unabhängig voneinander Wasserstoff, -(C₁-C₅)-Alkyl, -(C₁-C₅)-Alkanoyl, -(C₁-C₅)-Alkoxycarbonyl, Benzyl, Benzyloxycarbonyl;
oder
R(21) Trityl;
R(20) -OR(23) oder -NR(23)R(24);
R(23) und R(24)
unabhängig voneinander Wasserstoff, -(C₁-C₄)-Alkyl oder Benzyl;
k Null, 1 oder 2;
oder
R(1), R(2) und R(3)
unabhängig voneinander (C₁-C₉)-Heteroaryl, das über C oder N verknüpft ist und das unsubstituiert oder substituiert ist mit einem Substituenten aus der Gruppe F, Cl, CF₃, CH₃, Methoxy und Dimethylamino;
oder
R(1), R(2) und R(3)
-SR(25), -OR(25), -NR(25)R(26), -CR(25)R(26)R(27);
R(25) -C_{f}H_{2f}-(C₁-C₉)-Heteroaryl,
das unsubstituiert oder substituiert ist mit einem Substituenten aus der Gruppe F, Cl, CF₃, CH₃, Methoxy, Dimethylamino;
f Null, 1 oder 2;
R(26) und R(27)
unabhängig voneinander wie R(25) definiert oder Wasserstoff oder Methyl;
oder
R(1), R(2) und R(3)
unabhängig voneinander (C₁-C₉)-Heteroaryl-N-Oxid,
das über C oder N verknüpft ist und das unsubstituiert oder substituiert ist mit 1 - 2 Substituenten ausgewählt aus der Gruppe bestehend aus F, Cl, CF₃, CH₃, Methoxy, Hydroxy, Amino, Methylamino und Dimethylamino;
oder
R(1), R(2) und R(3)
unabhängig voneinander -SR(28), -OR(28), -NR(28)R(29) oder -CR(28)R(29)R(30);
R(28) -C_{g}H_{2g}-(C₁-C₉)-Heteroaryl-N-Oxid,
das unsubstituiert oder substituiert ist mit 1 - 2 Substituenten ausgewählt aus der Gruppe bestehend aus F, Cl, CF₃, CH₃, Methoxy, Hydroxy, Amino, Methylamino und Dimethylamino;
g Null oder 1;
R(29) und R(30)
unabhängig voneinander wie R(28) definiert, Wasserstoff oder Methyl;
oder
R(1) und R(3)
unabhängig voneinander Wasserstoff, F, Cl, CF₃, R(31)SO₂-, R(32)R(33)N-CO-, R(34)-CO- oder R(45)R(46)N-SO₂;
R(31) und R(34)
unabhängig voneinander Methyl oder -CF₃;
R(32), R(33), R(45) und R(46)
unabhängig voneinander Wasserstoff oder Methyl;
oder
R(1), R(2) und R(3)
unabhängig voneinander R(51)-A-G-D-;
R(51) -NR(52)R(53), eine Amidinogruppe R(52)R(53)N-C[=N-R(54)]- oder eine Guanidinogruppe R(52)R(53)N-C[=N-R(54)]-NR(55)-;
R(52), R(53), R(54) und R(55)
unabhängig voneinander Wasserstoff oder Alkyl mit 1, 2, 3 oder 4 C-Atomen;
oder
R(52) und R(53)
eine Gruppe C_{α}H_{2α};
α 4, 5, 6 oder 7;
wobei im Falle von α = 5, 6 oder 7 ein C-Atom der Gruppe C_{α}H_{2α} durch eine Heteroatomgruppe O, SO_{d} oder NR(56) ersetzt sein kann;
oder
R(53) und R(54)
eine Gruppe C_{γ}H_{2γ};
γ 2, 3, 4 oder 5;
wobei im Falle von γ = 3, 4 oder 5 ein C-Atom der Gruppe C_{γ}H_{2γ} durch eine Heteroatomgruppe O, SO_{d} oder NR(56) ersetzt sein kann;
d Null oder 2;
R(56) Wasserstoff oder Methyl;
oder
R(51) Imidazolyl, Pyridyl, Chinolinyl oder Isochinolinyl;
A eine Gruppe CₑH₂ₑ;
e Null, 1, 2, 3, 4 oder 5;
wobei in der Gruppe CₑH₂ₑ ein C-Atom durch eine der Gruppierungen -O-, -CO- oder -SOᵣ- ersetzt sein kann;
r Null oder 2;
G einen Phenylenrest, 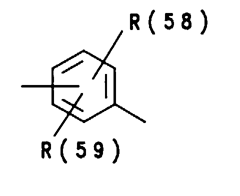 R(58) und R(59)
unabhängig voneinander Wasserstoff, Methyl, F, Cl, CF₃ oder -SO₂-R(60);
R(60) Methyl oder NR(61)R(62);
R(61) und R(62)
unabhängig voneinander Wasserstoff oder Methyl;
D -CᵥH₂ᵥ-E_{w}-;
v Null, 1, 2, 3 oder 4;
E -O-, -CO-, -CH[OR(63)]-, -SOₐₐ- oder -NR(63)-;
w Null oder 1;
aa Null oder 2
R(63) Wasserstoff oder Methyl;
oder
R(2) -CF₂R(64), -CF[R(65)][R(66)], -CF(CF₃)[R(65)], -C(CF₃)=CR(65)R(66);
R(64) Alkyl mit 1, 2, 3 oder 4 C-Atomen oder Cycloalkyl mit 3, 4, 5, 6 oder 7 C-Atomen;
R(65) und R(66)
unabhängig voneinander Wasserstoff oder Alkyl mit 1, 2, 3 oder 4 C-Atomen;
oder
R(1), R(2) und R(3)
unabhängig voneinander -OR(67) oder -NR(67)R(68);
R(67) und R(68)
unabhängig voneinander Wasserstoff oder Alkyl mit 1, 2 oder 3 C-Atomen;
oder
R(67) und R(68)
gemeinsam 4, 5 oder 6 Methylengruppen, von denen eine CH₂-Gruppe durch Sauerstoff, -S-, SO₂, -NH- oder -NCH₃ ersetzt sein kann;
R(4) und R(5)
unabhängig voneinander Wasserstoff, Alkyl mit 1, 2 oder 3 C-Atomen, F, Cl, -OR(69), -NR(70)R(71) oder -CF₃;
R(69), R(70) und R(71)
unabhängig voneinander Wasserstoff oder Methyl;
R(6) und R(7)
unabhängig voneinander Wasserstoff oder Methyl;
X Sauerstoff oder NR(72);
R(72) Wasserstoff oder Methyl;
sowie deren pharmazeutisch verträgliche Salze.

Besonders bevorzugt sind Verbindungen der Formel I, in denen bedeuten:
R(1), R(2) und R(3)
unabhängig voneinander -O-[4-R(8)-Phenyl],
das Phenyl jeweils unsubstituiert oder substituiert ist mit einem Substituenten aus der Gruppe bestehend aus F, Cl, -CF₃, Methyl und Methoxy;
R(8) SOₐ[NR(98)]_{b}NR(99)R(10);
a 1 oder 2;
b 0 oder 1;
a + b = 2;
R(98) Wasserstoff oder Alkyl mit 1, 2, 3 oder 4 C-Atomen;
R(99) und R(10)
unabhängig voneinander Wasserstoff, Alkyl mit 1 oder 2 C-Atomen, Benzyl, -(C₂-C₃)-Alkylen-NR(11)R(12), (C₂-C₃)-Alkylen-NR(13)-(C₂-C₃)-Alkylen-NR(37)R(38) oder (C₀-C₂)-Alkylen-CR(39)R(40)-CR(41)R(42)(C₀-C₂)-Alkylen-NR(43)R(44);
R(11), R(12), R(13), R(37), R(38), R(43) und R(44)
unabhängig voneinander Wasserstoff, Methyl oder Ethyl;
R(39), R(40), R(41) und R(42)
unabhängig voneinander Wasserstoff, Alkyl mit 1, 2, 3 oder 4 C-Atomen oder Benzyl,
wobei das Phenyl nicht substituiert oder substituiert ist mit einem Substituenten ausgewählt aus der Gruppe bestehend aus F, Cl,-CF₃, Methyl und Methoxy;
oder
R(99) und R(10)
gemeinsam 4, 5 oder 6 Methylengruppen, von denen eine CH₂-Gruppe durch -NH-, oder -N-CH₃ ersetzt sein kann;
oder
R(8) SOₐ[NR(98)]_{b}NR(95)-C[=N-R(94)]-NR(93)R(92);
R(95) Wasserstoff;
R(92), R(93) und R(94)
unabhängig voneinander Wasserstoff oder Alkyl mit 1, 2, 3 oder 4 C-Atomen;
oder
R(1), R(2) und R(3)
unabhängig voneinander Wasserstoff oder Alkyl mit 1, 2, 3 oder 4 C-Atomen;
oder
R(1), R(2) und R(3)
unabhängig voneinander -Q-4-[(CH₂)ₖ-CH(NR(21)R(22))-(C=O)R(20)]-Phenyl,
wobei das Phenyl jeweils unsubstituiert oder substituiert ist mit einem Substituenten aus der Gruppe F, Cl, -CF₃, Methyl, Hydroxy und Methoxy;
Q eine Bindung oder Sauerstoff;
R(21) und R(22)
unabhängig voneinander Wasserstoff, Methyl, -(C₁-C₅)-Alkanoyl, -(C₁-C₅)-Alkoxycarbonyl, Benzyl oder Benzyloxycarbonyl;
R(20) -OR(23) oder -NR(23)R(24);
R(23) und R(24)
unabhängig voneinander Wasserstoff, -(C₁-C₄)-Alkyl oder Benzyl;
k Null, 1 oder 2;
oder
R(1), R(2) und R(3)
unabhängig voneinander Imidazolyl,
das über C oder N verknüpft ist und das unsubstituiert oder substituiert ist mit einem Substituenten aus der Gruppe-F, Cl, CF₃, CH₃ oder Methoxy;
oder
R(1), R(2) und R(3)
-SR(25), -OR(25), -NR(25)R(26), -CR(25)R(26)R(27);
R(25) -(C₁-C₉)-Heteroaryl,
das unsubstituiert oder substituiert ist mit einem Substituenten aus der Gruppe F, Cl, CF₃, CH₃ oder Methoxy;
R(26) und R(27)
unabhängig voneinander Wasserstoff oder Methyl;
oder
R(1), R(2) und R(3)
unabhängig voneinander -SR(28), -OR(28), -NR(28)R(29) oder -CR(28)R(29)R(30);
R(28) -(C₁-C₉)-Heteroaryl-N-Oxid,
das unsubstituiert oder substituiert ist mit einem Substituenten ausgewählt aus der Gruppe bestehend F, Cl, CF₃, CH₃ und Methoxy;
R(29) und R(30)
unabhängig voneinander Wasserstoff oder Methyl;
oder
R(1) und R(3)
unabhängig voneinander Wasserstoff, F, Cl, CF₃, R(31)SO₂-, R(32)R(33)N-CO-, R(34)-CO- oder R(45)R(46)N-SO₂;
R(31) und R(34)
unabhängig voneinander Methyl oder -CF₃;
R(32), R(33), R(45) und R(46)
unabhängig voneinander Wasserstoff oder Methyl;
oder
R(2) R(51)-A-G-D-;
R(51) -NR(52)R(53), eine Amidinogruppe R(52)R(53)N-C[=N-R(54)]- oder eine Guanidinogruppe R(52)R(53)N-C[=N-R(54)]-NR(55)-;
R(52), R(53), R(54) und R(55)
unabhängig voneinander Wasserstoff oder Alkyl mit 1, 2, 3 oder 4 C-Atomen;
oder
R(52) und R(53)
eine Gruppe C_{α}H_{2α};
α 4, 5, 6 oder 7;
wobei im Falle von α = 5, 6 oder 7 ein C-Atom der Gruppe C_{α}H_{2α} durch eine Heteroatomgruppe O, SO_{d} oder NR(56) ersetzt sein kann,
oder
R(53) und R(54)
eine Gruppe C_{γ}H_{2γ};
γ 2, 3, 4 oder 5;
wobei im Falle von γ = 3, 4 oder 5 ein C-Atom der Gruppe C_{γ}H_{2γ} durch eine Heteroatomgruppe O, SOd oder NR(56) ersetzt sein kann;
d Null oder 2;
R(56) Wasserstoff oder Methyl;
oder
R(51) Imidazolyl, Pyridyl, Chinolinyl oder Isochinolinyl;
A CₑH₂ₑ;
e Null, 1, 2, 3, 4 oder 5;
wobei in der Gruppe CₑH₂ₑ ein C-Atom durch eine der Gruppierungen -O-, -CO- oder -SOᵣ ersetzt sein kann;
r Null oder 2;
G einen Phenylenrest, 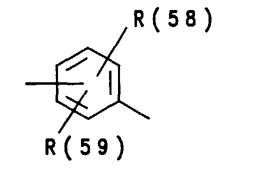 R(58) und R(59)
unabhängig voneinander Wasserstoff, Methyl, F, Cl, CF₃ oder -SO₂-R(60);
R(60) Methyl oder NR(61)R(62);
R(61) und R(62)
unabhängig voneinander Wasserstoff oder Methyl;
D -CᵥH₂ᵥ-E_{w}-;
v Null, 1, 2, 3 oder 4;
E -O-, -CO-, -CH[OR(63)]-, -SOₐₐ- oder -NR(63)-;
w Null oder 1;
aa Null oder 2
R(63) Wasserstoff oder Methyl,
oder
R(2) -CF₂R(64), -CF[R(65)][R(66)], -CF(CF₃)[R(65)], -C(CF₃)=CR(65)R(66);
R(64) Alkyl mit 1, 2, 3 oder 4 C-Atomen oder Cycloalkyl mit 3, 4, 5, 6 oder 7 C-Atomen;
R(65) und R(66)
unabhängig voneinander Wasserstoff oder Alkyl mit 1, 2, 3 oder 4 C-Atomen;
oder
R(1), R(2) und R(3)
unabhängig voneinander -OR(67) oder -NR(67)R(68);
R(67) und R(68)
unabhängig voneinander Wasserstoff oder Alkyl mit 1, 2 oder 3 C-Atomen;
oder
R(67) und R(68)
gemeinsam 4, 5 oder 6 Methylengruppen, von denen eine CH₂-Gruppe durch Sauerstoff, -S-, SO₂, -NH- oder -NCH₃ ersetzt sein kann;
R(4) und R(5)
unabhängig voneinander Wasserstoff, Alkyl mit 1, 2 oder 3 C-Atomen, F, Cl, -OR(69), -NR(70)R(71) oder -CF₃;
R(69), R(70) und R(71)
unabhängig voneinander Wasserstoff oder Methyl;
R(6) und R(7)
unabhängig voneinander Wasserstoff oder Methyl;
X Sauerstoff oder NR(72);
R(72) Wasserstoff oder Methyl;
sowie deren pharmazeutisch verträgliche Salze.

Ganz besonders bevorzugt sind Verbindungen der Formel I, in denen bedeuten:
R(2) -O-[4-R(8)-Phenyl],
R(8) SOₐ[NR(98)]_{b}NR(99)R(10);
a 1 oder 2;
b 0 oder 1;
a + b = 2;
R(98) Wasserstoff oder Alkyl mit 1, 2, 3 oder 4 C-Atomen;
R(99) und R(10)
unabhängig voneinander Wasserstoff, Alkyl mit 1 oder 2 C-Atomen oder -(C₂-C₃)-Alkylen-NR(11)R(12);
R(11) und R(12)
unabhängig voneinander Wasserstoff, Methyl oder Ethyl;
oder
R(99) und R(10)
gemeinsam 5 oder 6 Methylengruppen, von denen eine CH₂-Gruppe durch -NH-, oder -N-CH₃ ersetzt sein kann;
oder
R(8) SOₐ[NR(98)]_{b}NR(95)-C[=N-R(94)]-NR(93)R(92);
R(95) Wasserstoff;
R(92), R(93) und R(94)
unabhängig voneinander Wasserstoff oder Alkyl mit 1, 2, 3 oder 4 C-Atomen;
oder
R(1), R(2) und R(3)
unabhängig voneinander Wasserstoff oder Alkyl mit 1, 2, 3 oder 4 C-Atomen;
oder
R(2) -O-4-[CH₂-CH(NR(21)R(22))-(C=O)R(20)]-Phenyl,
R(21) und R(22)
unabhängig voneinander Wasserstoff, Methyl, -(C₁-C₅)-Alkanoyl,-(C₁-C₅)-Alkoxycarbonyl, Benzyl oder Benzyloxycarbonyl;
R(20) -OR(23) oder -NR(23)R(24);
R(23) und R(24)
unabhängig voneinander Wasserstoff oder -(C₁-C₄)-Alkyl;
oder
R(2) Imidazolyl,
das über C oder N verknüpft ist,
oder
R(2) -SR(25) oder -OR(25);
R(25) Pyridyl, Chinolinyl oder Isochinolinyl, die jeweils unsubstituiert oder substituiert sind mit einem Substituenten aus der Gruppe bestehend aus F, Cl, CF₃, CH₃ und Methoxy;
oder
R(2) -SR(28) oder -OR(28);
R(28) Pyridyl-N-Oxid, Chinolinyl-N-Oxid oder Isochinolinyl-N-Oxid,
die jeweils unsubstituiert oder substituiert sind mit einem Substituenten ausgewählt aus der Gruppe bestehend aus F, Cl, CF₃, CH₃ und Methoxy;
oder
R(1) Wasserstoff, F, Cl, CF₃, R(31)SO₂- oder R(45)R(46)N-SO₂;
R(31)
unabhängig voneinander Methyl oder -CF₃;
R(45) und R(46)
unabhängig voneinander Wasserstoff oder Methyl;
oder
R(2) R(51 )-A-G-O-;
R(51) -NR(52)R(53),
R(52) und R(53)
unabhängig voneinander Wasserstoff oder Alkyl mit 1 oder 2 C-Atomen;
oder
R(52) und R(53)
C_{α}H_{2α};
α 5 oder 6;
wobei ein C-Atom der Gruppe C_{α}H_{2α} durch NR(56) ersetzt sein kann;
R(56) Wasserstoff oder Methyl;
oder
R(51) Imidazolyl, Pyridyl, Chinolinyl oder Isochinolinyl;
A CₑH₂ₑ;
e Null, 1, 2 oder 3;
wobei in der Gruppe CₑH₂ₑ ein C-Atom durch eine der Gruppierungen -O-, -CO- oder -SOᵣ- ersetzt sein kann;
r Null oder 2;
G einen Phenylenrest, 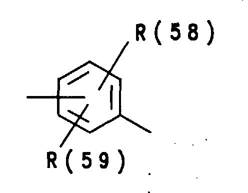 R(58) und R(59)
unabhängig voneinander Wasserstoff, Methyl, F, Cl, CF₃ oder -SO₂-R(60);
R(60) Methyl oder NR(61)R(62);
R(61) und R(62)
unabhängig voneinander Wasserstoff oder Methyl;
oder
R(2) -CF₂R(64), -CF[R(65)][R(66)], -CF(CF₃)[R(65)], -C(CF₃)=CR(65)R(66);
R(64) Alkyl mit 1, 2, 3 oder 4 C-Atomen oder Cycloalkyl mit 5 oder 6 C-Atomen;
R(65) und R(66)
unabhängig voneinander Wasserstoff oder Alkyl mit 1, 2, 3 oder 4 C-Atomen;
oder
R(1), R(2) und R(3)
unabhängig voneinander -OR(67) oder -NR(67)R(68);
R(67) und R(68)
unabhängig voneinander Wasserstoff, Methyl oder Ethyl;
oder
R(67) und R(68)
gemeinsam 4 oder 5 Methylengruppen, von denen eine CH₂-Gruppe durch Sauerstoff, -NH- oder -NCH₃ ersetzt sein kann;
R(4) und R(5)
unabhängig voneinander Wasserstoff, Alkyl mit 1, 2 oder 3 C-Atomen, F, Cl oder -CF₃;
R(6) und R(7)
unabhängig voneinander Wasserstoff oder Methyl;
X Sauerstoff oder NR(72)
R(72) Wasserstoff oder Methyl;
sowie ihre pharmazeutisch verträglichen Salze.

Insbesondere bevorzugt sind Verbindungen der Formel I, ausgewählt aus der Gruppe bestehend aus
4-(3-Pyridyloxy)-3-trifluormethyl-benzyloxycarbonylguanidin Dihydrochlorid,
4-(6-Chinaldinyloxy)-3-methylsulfonyl-benzyloxycarbonylguanidin Dihydrochlorid,
4-(6-Chinaldinyloxy)-3-trifluormethyl-benzyloxycarbonylguanidin Dihydrochlorid,
4-lsopropyl-3-methylsulfonyl-benzyloxycarbonylguanidin Hydrochlorid,
3-lsopropyl-benzyloxycarbonylguanidin Hydrochlorid,
2-Chlor-5-trifluormethyl-benzyloxycarbonylguanidin Hydrochlorid,
4-(6-Chinolinyloxy)-3-methylsulfonyl-benzyloxycarbonylguanidin Dihydrochlorid,
3-Brom-5-fluor-benzylamino-carbonylguanidid-hydrochlorid,
3,5-Dimethylbenzylamino-carbonylguanidid-hydrochlorid,
2-Fluorbenzylamino-carbonylguanidid-hydrochlorid,
3-Fluorbenzylamino-carbonylguanidid-hydrochlorid,
2,6-Difluorbenzylamino-carbonylguanidid-hydrochlorid,
2,5-Difluorbenzylamino-carbonylguanidid-hydrochlorid,
3-Fluor-5-trifluormethylbenzylamino-carbonylguanidid-hydrochlorid,
4-Dimethylaminobenzylamino-carbonylguanidid-hydrochlorid,
3,5-Difluorbenzylamino-carbonylguanidid-hydrochlorid,
3-Methylbenzylamino-carbonylguanidid-hydrochlorid,
N-3,5-Difluorbenzyl-N-methyl-amino-carbonylguanidid-hydrochlorid,
3,5-Difluorbenzyloxy-carbonylguanidid-hydrochlorid,
2,5-Difluorbenzyloxy-carbonylguanidid-hydrochlorid,
2,3,6-Trifluorbenzyloxy-carbonylguanidid-hydrochlorid,
N-(3,4-Dichlorbenzyl)-N-methyl-aminocarbonylguanidin-hydrochlorid,
N-(2-Chlor-5-trifluormethyl-benzyl)-N-methyl-aminocarbonylguanidin-hydrochlorid,
N-Methyl-N-(3-methylsulfonyl-4-isopropyl-benzyl)-aminocarbonylguanidinmethansulfonat,
N-(4-Fluor-3-trifluormethyl-benzyl)-N-methyl-aminocarbonylguanidin-hydrochlorid,
4-lsopropyl-3-methylsulfonyl-benzyloxycarbonylguanidin-hydrochlorid,
2-Chlor-5-trifluormethyl-benzyloxycarbonylguanidin-hydrochlorid,
3-Isopropyl-benzyloxycarbonylguanidin-hydrochlorid,
4-(6-Chinolyloxy)-3-methylsulfonyl-benzyloxycarbonylguanidin-hydrochlorid und
3-Trifluormethyl-4-methoxy-benzyloxycarbonylguanidin-hydrochlorid.

Enthält eine der Verbindungen der Formel (I) ein oder mehrere Asymmetriezentren, so können diese unabhängig voneinander sowohl S als auch R konfiguriert sein. Die Verbindungen können als optische Isomere, als Diastereomere, als Racemate oder als Gemische derselben vorliegen.

Die bezeichneten Alkyl- und Perfluoralkylreste können sowohl geradkettig als auch verzweigt vorliegen.

Unter (C₁-C₉)-Heteroaryl werden insbesondere Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bicyclischer Reste (wie im Indolizinyl) N-Atome sein.

Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl und Cinnolinnyl.

Die Verbindungen I werden erhalten, nach einem Verfahren, bei dem man zunächst eine Verbindung der Formel II 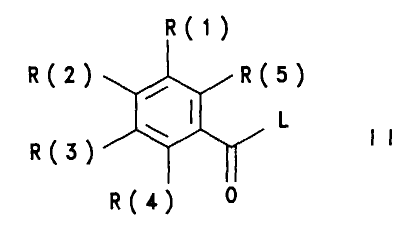 worin R(1) bis R(5) die oben angegebenen Bedeutungen haben, reduziert oder mit einem C-Nucleophil umsetzt. Man erhält Vorstufen der Formel III, 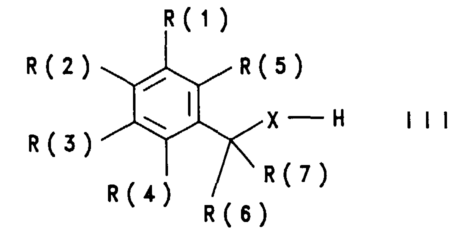 wobei R(1) bis R(7) sowie X die oben angegebene Bedeutung haben.

Die Säurederivate der Formel II, worin L eine Amino-, Alkylamino- oder Guanidinogruppe, oder eine Alkoxy-, vorzugsweise eine Methoxygruppe oder Phenoxygruppe, eine Phenylthio-, Methylthio-, 2-Pyridylthiogruppe, oder einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere Säurederivate der Formel II in an sich bekannter Weise direkt aus den zugrundeliegenden Benzoesäurederivaten (Formel II, L = OH) herstellen, wie beispielsweise die Methylester der Formel II mit L = OCH₃ durch Behandeln mit gasförmigem HCI in Methanol, die Imidazolide der Formel II durch Behandeln mit Carbonyldiimidazol [L = 1-Imidazolyl, Staab, Angew. Chem. Int. Ed. Engl. 1, 351 - 367 (1962)], die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)-methylen)amino]-1,1,3,3-tetramethyluroniumtetrafluoroborat] ("TOTU") [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der allgemeinen Formel II sind unter Angabe von Quellenliteratur in J. March, Advanced Organic Chemistry, Third Edition (John Wiley & Sons, 1985), S. 350 angegeben.

Die Umsetzung der Vorstufen der Formel III zu den Acylguanidinen der Formel I erfolgt über die Umsetzung mit einem geeigneten Kohlensäurederivat, bevorzugt Phosgen, Diphosgen (Chlorameisensäuretrichlormethylester), Triphosgen (Kohlensäure-bis-trichlormethylester), Chlorameisensäureethylester, Chlorameisensäure-i-butylester, Bis-(1-hydroxy-1-H-benzotriazolyl)carbonat und N,N'-Carbonyldiimidazol, in einem gegenüber den verwendeten Reagentien inerten Lösungsmitteln, bevorzugt DMF, THF oder Toluol, bei einer Temperatur zwischen - 20°C und dem Siedepunkt des Lösungsmittels, bevorzugt zwischen 0°C und 60°C, zunächst zu einem substituierten Kohlensäurederivat der Formel IV, 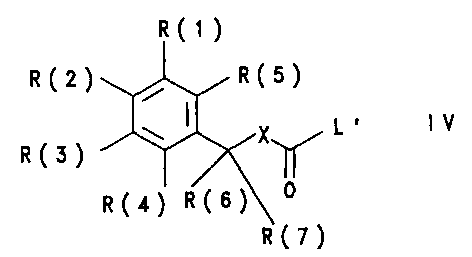 worin R(1) bis R(7) sowie X die oben angegebene Bedeutung haben und L' je nach verwendetem Kohlensäurederivat Chlor, Ethoxy, Isobutoxy, Benzotriazol-1-oxy oder 1-Imidazolyl bedeutet. Die Guanylierung der Kohlensäurederivate der Formel IV zu den erfindungsgemäßen Verbindungen der Formel I erfolgt bevorzugt im selben Lösungsmittel bei einer Temperatur zwischen 0°C und 60°C ohne vorangegangene Reinigung.
Die unbekannten Verbindungen der Formel II können nach literatur-bekannten Methoden hergestellt werden, indem man beispielsweise 4-Halogen-3-chlorsulfonylbenzoesäuren mit Ammoniak oder Aminen in 3-Aminosulfonyl-4-Halogen-Benzoesäuren bzw. mit einem schwachen Reduktionsmittel wie Natriumbisulfit und anschließende Alkylierung in 3-Alkylsulfonyl-4-Halogen-Benzoesäuren überführt und nach einer der oben beschriebenen Verfahrensvarianten zu erfindungsgemäßen Verbindungen I umsetzt.

Die Einführung der im Phenylteil mit Schwefel-, Sauerstoff- oder Stickstoffnucleophilen substituierten Benzolsulfonamid-Derivate gelingt durch literaturbekannte Methoden der nucleophilen Substitution am Aromaten. Als Abgangsgruppe am Benzoesäurederivat haben sich bei dieser Substitution Halogenide und Trifluormethansulfonate bewährt. Man arbeitet vorteilhaft in einem dipolar aprotischen Lösungsmittel, wie DMF oder TMU, bei einer Temperatur von 0°C bis zum Siedepunkt des Lösungsmittels, bevorzugt von 80°C bis zum Siedepunkt des Lösungsmittels. Als Säurefänger dient vorteilhaft ein Alkali- oder Erdalkalisalz mit einem Anion hoher Basizität und geringer Nucleophilie, zum Beispiel K₂CO₃ oder CsCO₃.

Die Einführung der Alkyl- oder Arylsubstituenten gelingt durch literaturbekannte Methoden des Palladium-vermittelten cross-couplings von Arylhalogeniden mit beispielsweise Organozinkverbindungen, Organostannanen, Organoboronsäuren oder Organoboranen.

Acylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Ascorbate, Lactate, Sulfate, Citrate, Tartrate, Acetate, Phosphate, Methylsulfonate, p-Toluolsulfonate.

In der US-Patentschrift 5 091 394 (HOE 89/F 288) sind ausschließlich Benzoylguanidine beschrieben. Alkanoylguanidine sind dagegen nirgendwo erwähnt, ebenso wenig eine Hemmung des zellulären Na⁺/H⁺-Austauschmechanismus durch sie.

Es war daher überraschend, daß die Verbindungen der Formel I potente Inhibitoren dieses Systems sind.

Gegenüber dem Stand der Technik zeichnen sich die Verbindungen der Formel I durch eine erhöhte Solvolysestabilität aus.

Die Verbindungen I sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe, beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnem, Suppositoriengrundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z.B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage: Wasser, physiologische Kochsalzlösung oder Alkohole, z.B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z.B. Lösungen, Suspensionen oder Emulsionen des Wirkstoffes der Formel I in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg Körpergewicht, vorzugsweise mindestens 0,01 mg/kg Körpergewicht, bis höchstens 10 mg/kg Körpergewicht, vorzugsweise bis höchstens 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z.B. bis zu 4 Einzeldosen pro Tag. Insbesondere bei i.v. Anwendung, etwa bei einem Infarktpatienten auf der Intensivstation können bis zu 100 mg pro Tag notwendig werden.

### Liste der Abkürzungen:

- MeOH: Methanol
- DMF: N,N-Dimethylformamid
- TMU: N, N, N', N'-Tetramethylharnstoff
- NBS: N-Bromsuccinimid
- AIBN: α,α-Azo-bis-isobutyronitril
- El: electron impact
- DCI: Desorption-Chemical Ionisation
- RT: Raumtemperatur
- EE: Ethylacetat (EtOAc)
- DIP: Diisopropylether
- MTB: Methyltertiärbutylether
- mp: Schmelzpunkt
- HEP: n-Heptan
- DME: Dimethoxyethan
- FAB: Fast Atom Bombardment
- CH₂Cl₂: Dichlormethan
- THF: Tetrahydrofuran
- eq: Äquivalent
- ES: Elektrospray-lonisation
- Me: Methyl
- Et: Ethyl
- Bn: Benzyl
- ZNS: Zentralnervensystem
- Brine: gesättigte wäßrige NaCl-Lösung
- CDI: N,N'-Carbonyldiimidazol

### Beispiel 1: 4-(3-Pyridyloxy)-3-trifluormethyl-benzyloxycarbonylguanidin, Dihydrochlorid

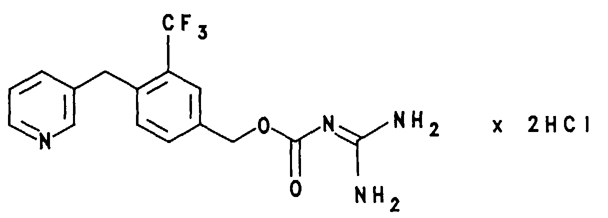

a) 4-(3-Pyridyloxy)-3-trifluoromethyl-benzoesäuremethylester
   2 mmol 4-Fluor-3-trifluoromethyl-benzoesäuremethylester, 2 mmol 3-Hydroxypyridin und 4 mmol K₂CO₃ werden in 15 ml DMF (wasserfrei) 1.5 h bei 110°C gerührt. Das Gemisch wird anschließend auf 100 ml Wasser gegossen und 3 x mit je 50 ml EE extrahiert. Über Na₂SO₄ wird getrocknet, das Lösungsmittel im Vakuum entfernt und das Produkt ohne weitere Reinigung weiter umgesetzt.
   500 mg farbloses Öl.
   R_{f}(MTB) = 0.33 MS (ES) : 298 (M+1)⁺
b) 4-(3-Pyridyloxy)-3-trifluormethyl-benzylalkohol
   0.9 g 4-(3-Pyridyloxy)-3-trifluormethyl-benzoesäuremethylester werden in 10 ml THF gelöst und bei 0°C 235 mg LiAlH₄ zugegeben. 3 h wird bei RT gerührt, das Gemisch auf 50 ml 1 N Na₂CO₃ gegossen und 3 mal mit 50 ml EE extrahiert. Über Na₂SO₄ wird getrocknet und das Solvens im Vakuum entfernt. Man erhält 780 mg eines weißen Feststoffs, der ohne weitere Reinigung verwendet wird. mp 96°C R_{f} (MTB) = 0.22MS (EI): 269 (M+1)⁺
c) 4-(3-Pyridyloxy)-3-trifluormethyl-benzyloxycarbonylguanidin, Dihydrochlorid 600 mg 4-(3-Pyridyloxy)-3-trifluormethyl-benzylalkohol und 360 mg CDI werden in 10 ml DMF gelöst und 24 h bei RT gerührt. Anschließend wird 660 mg Guanidin zugegeben, und es wird weitere 24 h bei RT gerührt. Das Reaktionsgemisch wird auf 100 ml Wasser gegossen eine Stunde bei RT gerührt und das Produkt abfiltriert. Anschließend wird in 50 ml 0.1 N wäßriger HCI-Lösung aufgenommen und Wasser sowie überschüssiges HCI im Vakuum entfernt.
   Man erhält 750 mg des Dihydrochlorids,
   mp 130°C (Zersetzung).
   R_{f} (EE/MeOH 10:1) = 0.08 MS (ES) 355 (M+H)⁺

Beispiele 2 bis 7 wurden analog Beispiel 1 synthetisiert:

### Beispiel 2: 4-(6-Chinaldinyloxy)-3-methylsulfonyl-benzyloxycarbonylguanidin, Dihydrochlorid

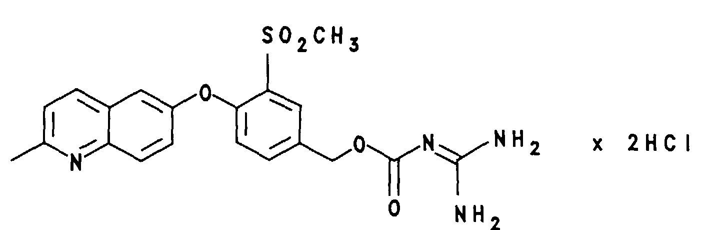

R_{f} (EE/MeOH 3:1) = 0.25 MS (ES): 429 (M+H)⁺

### Beispiel 3: 4-(6-Chinaldinyloxy)-3-trifluormethyl-benzyloxycarbonylguanidin, Dihydrochlorid

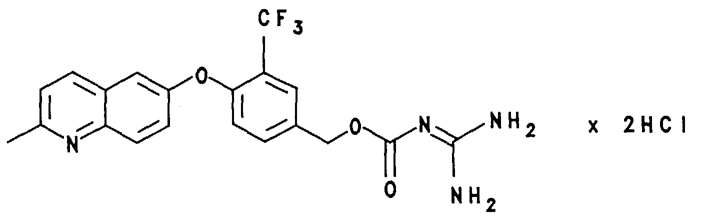

R_{f} (EE/MeOH 3:1) = 0.44 MS (ES) 419 (M+1)⁺

### Beispiel 4: 4-Isopropyl-3-methylsulfonyl-benzyloxycarbonylguanidin, Hydrochlorid

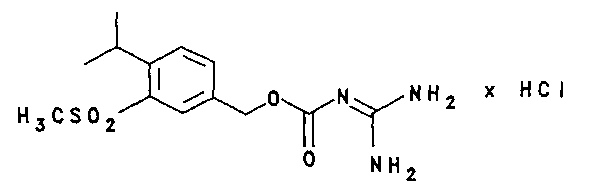

MS (ES): 314 (M+1)⁺

### Beispiel 5: 3-Isopropyl-benzyloxycarbonylguanidin, Hydrochlorid

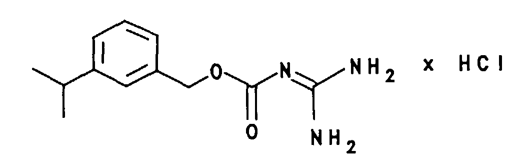

MS (ES) 236 (M+1)⁺

### Beispiel 6: 2-Chlor-5-trifluormethyl-benzyloxycarbonylguanidin, Hydrochlorid

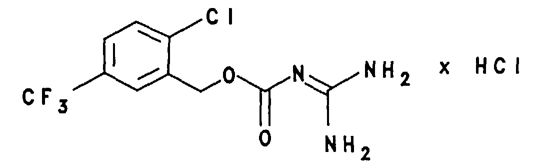

MS (ES): 296 (M+1)⁺

### Beispiel 7: 4-(6-Chinolinyloxy)-3-methylsulfonyl-benzyloxycarbonylguanidin, Dihydrochlorid

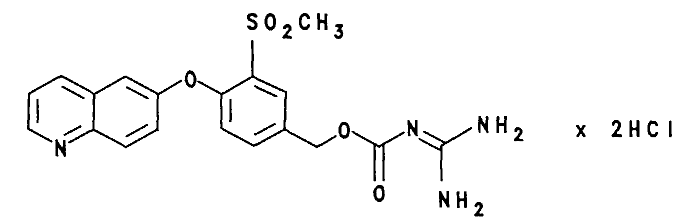

MS (ES): 415 (M+1)⁺

Allgemeine Vorschrift A: 1 eq. des entsprechenden Benzylamins in THF (1 ml/mmol)wurden bei 0°C zu einer Lösung von Carbonyldiimidazol in THF (1.1eq CDI, 3 ml/mmol THF) hinzugetropft. Die Reaktionslösung wurde bis zur vollständigen Umsetzung bei RT (DC-Kontrolle) gerührt. Anschließend wurden 4 eq. Guanidin hinzugegeben. Nach dem Rühren über Nacht destilliert man das THF unter vermindertem Druck ( im Rotationsverdampfer) ab, versetzt mit Wasser, stellt mit 2 N HCI auf pH 6 bis 8 und filtriert das entsprechende Guanidid ab. Die so erhaltenen Benzylaminocarbonylguanidide können durch Behandeln mit wäßriger, methanolischer oder etherischer Salzsäure oder anderen pharmakologisch verträglichen Säuren in die entsprechenden Salze übergeführt werden.

### Beispiel 8: 3-Brom-5-fluor-benzylamino-carbonylguanidid-hydrochlorid

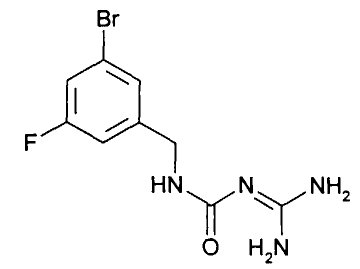

3-Brom-5-fluor-benzylamin wurde gemäß der allgemeinen Vorschrift A zunächst mit CDI und anschließend mit Guanidin umgesetzt und als Hydrochlorid isoliert.
mp: 125°C MS (ES ): 289 (M+1)⁺

### Beispiel 9: 3,5-Dimethylbenzylamino-carbonylguanidid-hydrochlorid

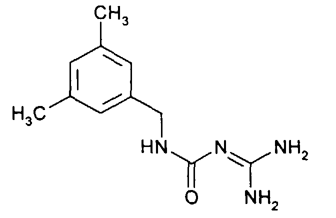

3,5-Dimethylbenzylamin wurde gemäß der allgemeinen Vorschrift A umgesetzt und als Hydrochlorid isoliert.
mp: 97°C MS ( FAB ) 221 (M+1)⁺

### Beispiel 10: 2-Fluorbenzylamino-carbonylguanidid-hydrochlorid

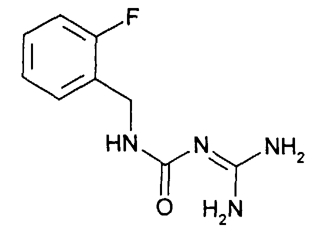

2-Fluorbenzylamin wurde gemäß der allgemeinen Vorschrift A zunächst mit CDI und anschließend mit Guanidin umgesetzt und als Hydrochlorid isoliert.
mp: 125°C MS ( ES ): 211 (M+1)⁺

### Beispiel 11: 3-Fluorbenzylamino-carbonylguanidid-hydrochlorid

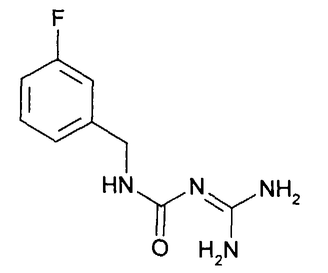

3-Fluorbenzylamin wurde gemäß der allgemeinen Vorschrift A umgesetzt und als Hydrochlorid (zähes Öl )isoliert.
MS ( ES ): 211 (M+1)⁺
R_{F}: 0.42 ( EE/Cyclohexan/Methylenchlorid/MeOH/Ammoniak = 10/5/5/5/1)

### Beispiel 12: 2,6-Difluorbenzylamino-carbonylguanidid-hydrochlorid

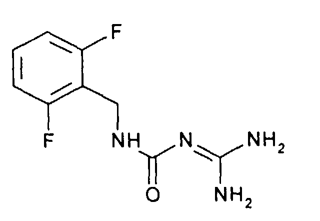

2,6-Difluorbenzylamin wurde gemäß der allgemeinen Vorschrift A zunächst mit CDI und anschließend mit Guanidin umgesetzt und als Hydrochlorid isoliert.
mp: 150°C MS ( ES ): 229 (M+1)⁺

### Beispiel 13: 2,5-Difluorbenzylamino-carbonylguanidid-hydrochlorid

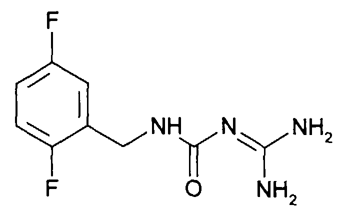

2,5-Difluorbenzylamin wurde gemäß der allgemeinen Vorschrift A zunächst mit CDI und anschließend mit Guanidin umgesetzt und als Hydrochlorid isoliert.
mp: 103°C MS ( FAB ): 229 (M+1)⁺

### Beispiel 14: 3-Fluor-5-trifluormethylbenzylamino-carbonylguanidid-hydrochlorid

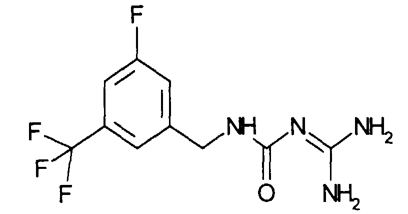

3-Fluor-5-trifluormethylbenzylamin wurde gemäß der allgemeinen Vorschrift A zunächst mit CDI und anschließend mit Guanidin umgesetzt und als Hydrochlorid isoliert.
mp: 133°C MS ( ES ): 279 (M+1)⁺

### Beispiel 15: 4-Dimethytaminobenzylamino-carbonylguanidid-hydrochlorid

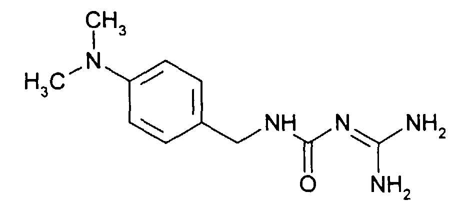

4-Dimethylaminobenzylamin wurde gemäß der allgemeinen Vorschrift A zunächst mit CDI und anschließend mit Guanidin umgesetzt und als Hydrochlorid isoliert.
mp: 187°C MS ( ES ): 236 (M+1)⁺

### Beispiel 16: 3,5-Difluorbenzylamino-carbonylguanidid-hydrochlorid

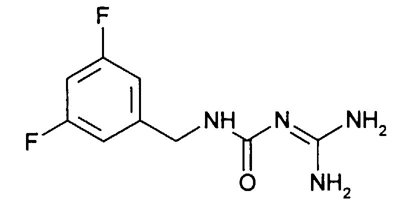

3,5-Difluorbenzylamin wurde gemäß der allgemeinen Vorschrift A zunächst mit CDI und anschließend mit Guanidin umgesetzt und als Hydrochlorid isoliert.
mp: 120°C MS ( ES ): 229 (M+1)⁺

### Beispiel 17: 3-Methylbenzylamino-carbonylguanidid-hydrochlorid

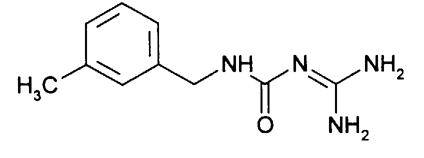

3-Methylbenzylamin wurde gemäß der allgemeinen Vorschrift A zunächst mit CDI und anschließend mit Guanidin umgesetzt und als Hydrochlorid (zähes hygroskopisches Öl) isoliert.
MS ( ES ): 207 (M+1)⁺
R_{F}: 0.48 (EE/Cyclohexan/Methylenchlorid/MeOH/Ammoniak = 10/5/5/5/1)

### Beispiel 18: N-3,5-Difluorbenzyl-N-methyl-amino-carbonylguanidid-hydrochlorid

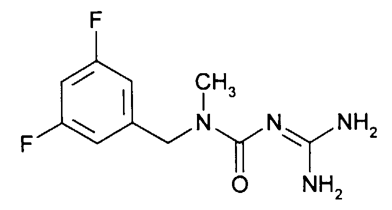

3,5-Difluorbenzyl-methylamin (dargestellt nach Standardverfahren: N-Formylierung, Reduktion) wurde gemäß der allgemeinen Vorschrift A zunächst mit CDI und anschließend mit Guanidin umgesetzt und als Hydrochlorid isoliert.
mp: 126°C MS ( ES ): 243 (M+1)⁺

### Beispiel 19: 3,5-Difluorbenzyloxy-carbonylguanidid-hydrochlorid

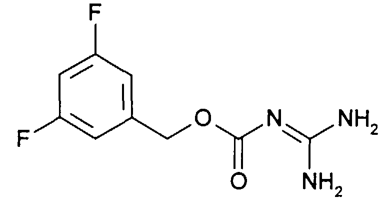

3,5-Difluorbenzylalkohol wurde gemäß der allgemeinen Vorschrift B zunächst mit CDI und anschließend mit Guanidin umgesetzt und als Hydrochlorid isoliert.
mp: 177°C MS (Cl): 230 (M+1)⁺

### Beispiel 20: 2,5-Difluorbenzyloxy-carbonylguanidid-hydrochlorid

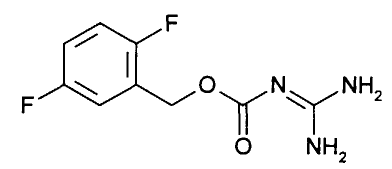

2,5-Difluorbenzylalkohol wurde gemäß der allgemeinen Vorschrift B zunächst mit CDI und anschließend mit Guanidin umgesetzt und als Hydrochlorid isoliert.
mp: 169°C MS (ES): 230 (M+1)⁺

### Beispiel 21: 2,3,6-Trifluorbenzyloxy-carbonylguanidid-hydrochlorid

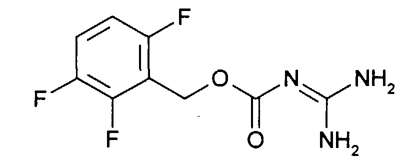

2,3,6-Trifluorbenzylalkohol wurde gemäß der allgemeinen Vorschrift B zunächst mit CDI und anschließend mit Guanidin umgesetzt und als Hydrochlorid isoliert.
mp: 180°C MS ( ES ): 248 (M+1)⁺

### Beispiel 22: N-(3,4-Dichlorbenzyl)-N-methyl-aminocarbonylguanidin-hydrochlorid

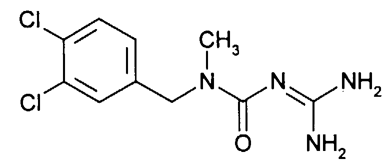

Zu einer Lösung von 3.0 g 3,4-Dichlorbenzylmethylamin in 110 ml THF werden 3.5 g CDI zugefügt, und es wird über Nacht bei RT gerührt. Nach Zugabe von 4.6 g Guanidin wird erneut über Nacht gerührt, die Reaktionsmischung am Rotationsverdampfer eingeengt und der Rückstand mit 120 ml Wasser verrührt. Der hierbei ausgefallene Niederschlag wird abgesaugt, im Vakuum getrocknet und in 50 ml Essigester und 7 ml Methanol gelöst. Nach Zugabe von etherischer Salzsäure und Absaugen des ausgefallenen Produktes werden 3.8 g N-(3,4-Dichlorbenzyl)-N-methyl-aminocarbonylguanidin-hydrochlorid erhalten;
mp: 198-199°C.
¹H-NMR (DMSO-d6): δ [ppm] = 3.05 (3H), 4.6 (2H), 7.3 (1H), 7.6 (1H), 7.7 (1H), 8.25 (2H), 8.65 (2H), 10.7 (1H).

### Beispiel 23: N-(2-Chlor-5-trifluormethyl-benzyl)-N-methyl-aminocarbonylguanidinhydrochlorid

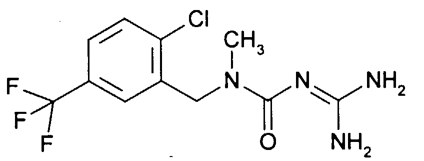

a) 2.1 g 2-Chlor-5-trifluormethylbenzaldehyd, 15 ml Methylamin und 2 g Magnesiumsulfat werden 2 h bei Raumtemperatur gerührt, wobei das überschüssige Methylamin größtenteils verdunstet. Die Reaktionsmischung wird mit Ether verdünnt, filtriert und im Vakuum eingeengt. Das so erhaltene Methylbenzimin wird in 15 ml THF gelöst und zu einer Suspension von 1.5 g Natriumboranat in 15 ml THF getropft. Nach Rühren über Nacht versetzt man mit 30 ml Methanol, rührt noch 30 min und stellt dann mit verd. Salzsäure sauer. Man extrahiert mit tert.-Butylmethylether, stellt die wäßrige Phase alkalisch und extrahiert erneut 2x mit tert.-Butylmethylether. Nach Einengen dieses Extraktes erhält man 1.0 g N-(2-Chlor-5-trifluormethyl-benzyl)-N-methyl-amin.
b) 1.0 g N-(2-Chlor-5-trifluormethyl-benzyl)-N-methyl-amin wird analog Beispiel 23 mit 0.75 g CDI und 1.1 g Guanidin umgesetzt. Man erhält 0.6 g N-(2-Chlor-5-trifluormethyl-benzyl)-N-methyl-aminocarbonylguanidin-hydrochlorid; mp: 178 - 179°C.
   ¹H-NMR (DMSO-d6): δ [ppm] = 3.1 (3H), 4.7 (2H), 7.6 (1H), 7.75 (2H), 8.2 (2H), 8.6 (2H), 10.6 (1H).

### Beispiel 24: N-Methyl-N-(3-methylsulfonyl-4-isopropyl-benzyl)-aminocarbonylguanidin-methansulfonat

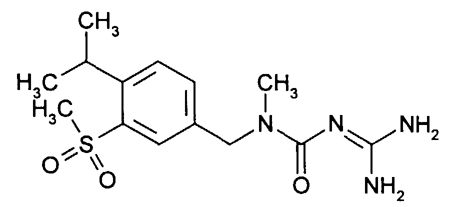

Aus 4-lsopropyl-3-methylsulfonylbenzoesäure wird auf dem bei Beispiel 23 beschriebenen Weg N-Methyl-N-(3-methylsulfonyl-4-isopropyl-benzyl)-aminocarbonylguanidin-methansulfonat erhalten;
mp: 180°C.
¹H-NMR (DMSO-d6): δ [ppm] = 1.3 (6H), 2.4 (3H), 3.0 (3H), 3.25 (3H), 3.8 (1H), 4.6 (2H), 7.6 (1H), 7.7 (1H), 7.8 (1H), 8.15 (4H), 9.8 (1H).

### Beispiel 25: N-(4-Fluor-3-trifluormethyl-benzyl)-N-methyl-aminocarbonylguanidinhydrochlorid

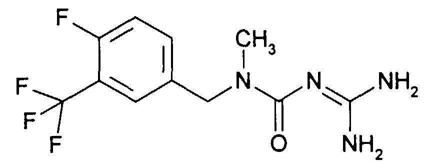

a) 1.9 g 4-Fluor-3-trifluormethylbenzaldehyd, 30 ml Methylamin und 2 g Magnesiumsulfat werden 2 h bei Raumtemperatur gerührt, wobei das überschüssige Methylamin größtenteils verdunstet. Die Reaktionsmischung wird mit Ether verdünnt, filtriert und im Vakuum eingeengt. Das so erhaltene Methylbenzimin wird in 30 ml Methanol gelöst und nach Zugabe von 2.0 g Natriumboranat wird über Nacht gerührt. Man engt im Vakuum ein, versetzt den Rückstand mit 20 ml 10proz. Salzsäure und extrahiert 2mal mit tert.-Butylmethylether. Dann stellt man die wäßrige Phase alkalisch und extrahiert erneut 2mal mit tert.-Butylmethylether. Nach Einengen dieses Extraktes erhält man 1.0 g N-(4-Fluor-3-trifluormethyl-benzyl)-N-methyl-amin.
b) 1.0 g N-(4-Fluor-3-trifluormethyl-benzyl)-N-methyl-amin und 1.0 g CDI werden in THF 2 h gerührt. Dann fügt man 1.4 g Guanidin hinzu, rührt über Nacht, engt am Rotationsverdampfer ein und verrührt mit Wasser. Der ausgefallene Niederschlag wird abgesaugt und mit Essigester/HCI in das Hydrochlorid überführt. Man erhält 1.2 g N-(4-Fluor-3-trifluormethyl-benzyl)-N-methyl-aminocarbonylguanidin-hydrochlorid ; mp: 150-152°C
   ¹H-NMR (DMSO-d6): δ [ppm] = 3.05 (3H), 4.65 (2H), 7.5 (1H), 7.7 (2H), 8.2 (2H), 8.6 (2H), 10.5 (1H).

### Beispiel 26: 4-lsopropyl-3-methylsulfonyl-benzyloxycarbonylguanidin-hydrochlorid

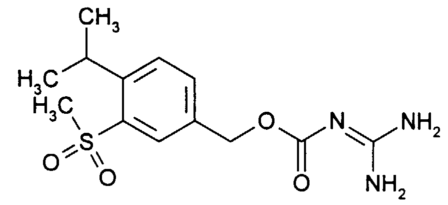

Syntheseweg:
a) 4-Isopropyl-benzoesäure durch Oxidation von 4-lsopropyl-benzaldehyd mit Natriumperborat in Essigsäure bei 50°C,
   mp. 118°C.
b) 4-Isopropyl-3-chlorsulfonyl-benzoesäure aus a) durch Erhitzen in Chlorschwefelsäure bei 95°C für 3 h,
   mp 203 - 4°C.
c) 2-lsopropyl-5-carboxy-benzolsulfinsäure aus b) durch Reduktion mit Natriumsulfit bei 60°C in wäßriger Natronlauge (pH ∼ 9 - 10),
   mp: 205 - 7°C.
d) 4-lsopropyl-3-methylsulfonyl-benzoesäure aus c) durch Alkylierung mit Methylbromid in Gegenwart von NaOH in DMF bei 60°C für 3 h,
   mp: 209 - 11°C.
e) Zu einer Lösung von 4.8 g 4-lsopropyl-3-methylsulfonyl-benzoesäure und 2.1 g Triethylamin in 40 ml THF werden bei -10°C 2.3 g Chlorameisensäureethylester gelöst in 5 ml THF zugetropft. Nach 1-stündigem Rühren wird vom ausgefallenen Niederschlag abgesaugt, und das Filtrat wird bei 5°C in eine Lösung von 2.3 g Natriumboranat in 25 ml Wasser eingetropft. Man rührt 5 h nach, säuert die Reaktionsmischung mit Salzsäure an, extrahiert mit Ether und wäscht die organische Phase mit 10proz. Natronlauge. Nach dem Einengen erhält man 3.1 g 4-Isopropyl-3-methylsulfonyl-benzylalkohol.
f) 2.3 g 4-lsopropyl-3-methylsulfonyl-benzylalkohol und 1.95 g CDI werden in 50 ml DMF über Nacht bei RT gerührt. Man fügt 3 g Guanidin hinzu, rührt 4 h nach und engt dann die Reaktionsmischung am Rotationsverdampfer ein. Der Rückstand wird auf 250 ml Wasser gegossen, und nach 1stündigem Rühren wird das kristalline Produkt abgesaugt. Nach Überführung in das Hydrochlorid erhält man 1.5 g 4-lsopropyl-3-methylsulfonyl-benzyloxycarbonylguanidin-hydrochlorid; mp: 159 - 160°C.
   ¹H-NMR (DMSO-d6): δ [ppm] = 1.3 (6H), 3.3 (3H), 3.8 (1H), 5.3 (2H), 7.75 (2H), 7.95 (1H), 8.1 (2H), 8.5 (2H), 11.6 (1H).

### Beispiel 27: 2-Chlor-5-trifluormethyl-benzyloxycarbonylguanidin-hydrochlorid

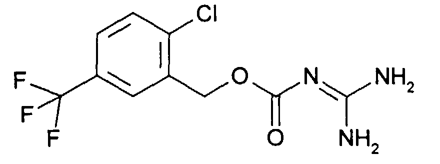

Analog Beispiel 26 wird ausgehend von 2-Chlor-5-trifluormethylbenzoesäure 2-Chlor-5-trifluormethyl-benzyloxycarbonylguanidin-hydrochlorid erhalten;
mp: 170°C.
¹H-NMR (DMSO-d6): δ [ppm] = 5.4 (2H), 7.8 (2H), 7.95 (1H), 8.15 (2H), 8.65 (2H), 11.9 (1H).

### Beispiel 28: 3-lsopropyl-benzyloxycarbonylguanidin-hydrochlorid

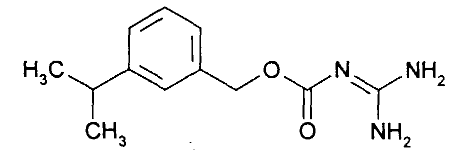

a. Trifluormethansulfonsäure-3-isopropylphenylester
   100 g 3-lsopropylphenol werden in 1 l CH₂Cl₂ gelöst, bei -30°C zunächst 95 g Lutidin, dann 18 g 4-Dimethylaminopyridin zugegeben. Anschließend wird bei dieser Temperatur 150 ml Trifluormethansulfonsäure-Anhydrid langsam zugetropft. Das Reaktionsgemisch wird auf RT erwärmt, auf 2 l einer gesättigten wäßrigen NaHCO₃-Lösung gegossen, das CH₂Cl₂ abgetrennt und noch 2 mal mit 300 ml desselben Lösungsmittels extrahiert. Über Na₂SO₄ wird getrocknet und das Solvens im Vakuum entfernt. Chromatographie an Kieselgel mit EE/Hep 1:8 liefert 181 g eines farblosen öls.
   R_{f}(EE/HEP 1:8) = 0.56
b. 3-lsopropylbenzoesäure-methylester
   180 g Trifluormethansulfonsäure-3-isopropylphenylester werden in 340 ml Methanol und 670 ml DMF gelöst, 187 ml Triethylamin, 1.5 g Pd(II)-acetat sowie 2.8 g 1,3-Bis-(diphenylphosphino)propan zugegeben und unter einer CO-Atmosphäre 8 h zum Rückfluß erhitzt. Anschließend werden die Solventien im Vakuum entfernt, mit 1 l einer gesättigten wäßrigen NaHCO₃-Lösung und 1 l Wasser aufgenommen und 3 mal mit je 500 ml MTB extrahiert. Über Na₂SO₄ wird getrocknet und das Solvens im Vakuum entfernt. Chromatographie an Kieselgel mit EE/HEP 1:8 liefert 25 g eines farblosen Öls.
   R_{f}(EE/HEP 1:8) = 0.22
c. Aus 1.0 g 3-lsopropylbenzoesäuremethylester werden durch Reduktion mit Lithiumaluminiumhydrid 0.8 g 3-lsopropylbenzylalkohol erhalten.
d. Aus 0.7 g 3-lsopropylbenzylalkohol, 1 g CDI und 1.3 g Guanidin werden analog Beispiel 26 b 0.6 g 3-lsopropyl-benzyloxycarbonylguanidin-hydrochlorid erhalten; mp: 112°C.
   ¹H-NMR (DMSO-d6): δ [ppm] = 1.2 (6H), 2.9 (1H), 5.2 (2H), 7.3 (4H), 8.15 (2H), 8.55 (2H), 11.65.

### Beispiel 29: 4-(6-Chinolyloxy)-3-methylsulfonyl-benzyloxycarbonylguanidinhydrochlorid

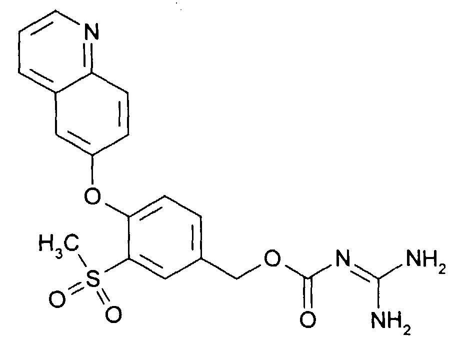

Analog Beispiel 22 wird ausgehend von 4-(6-Chinolyloxy)-3-methylsulfonylbenzoesäuremethylester 4-(6-Chinolyloxy)-3-methylsulfonylbenzyloxycarbonylguanidin-hydrochlorid erhalten; m.p.: 170°C.
a. 4-(6-Chinolyloxy)-3-methylsulfonyl-benzoesäuremethylester 2 mmol 4-Chlor-3-methylsulfonyl-benzoesäuremethylester, 2 mmol 6-Hydroxychinolin sowie 6 mmol K₂CO₃ wurden in 20 ml DMF (wasserfrei) bei 130°C 2 h gerührt. Das Gemisch wird anschließend in 100 ml gesättigte wäßrige NaHCO₃-Lösung gegossen und 3 x mit 100 ml EE extrahiert. Über Na₂SO₄ wird getrocknet, das Lösungsmittel im Vakuum entfernt und das Produkt ohne weitere Reinigung weiter umgesetzt.
R_{f}(MTB) = 0,15 MS (DCI): 358 (M+1)⁺

### Beispiel 30: 3-Trifluormethyl-4-methoxy-benzyloxycarbonylguanidin-hydrochlorid

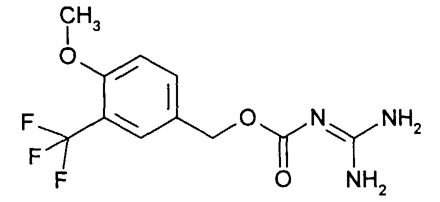

a) 9.7 g 3-lod-4-methoxybenzoesäuremethylester, 9.4 g Kaliumtrifluoracetat und 12.9 g Kupfer(I)-iodid werden in 250 ml DMF 5 h auf 160°C erhitzt. Man gießt auf 500 ml NaHCO₃-Lösung, saugt vom ausgefallenen Niederschlag ab und extrahiert sowohl den Niederschlag als auch die wäßrige Phase mit Essigester. Die vereinigten organischen Phasen werden eingeengt, und der Rückstand wird durch Flash-Chromatographie mit Hexan/Essigester 3:1 gereinigt. Man erhält 6 g 3-Trifluormethyl-4-methoxy-benzoesäuremethylester; mp: 77 - 79°C.
b) Der 3-Trifluormethyl-4-methoxy-benzoesäuremethylester wird analog Beispiel 28 in 3-Trifluormethyl-4-methoxy-benzyloxycarbonylguanidin-hydrochlorid überführt; mp: 143 - 144°C.
   ¹H-NMR (DMSO-d6): δ [ppm] = 3.9 (3H), 5.25 (2H), 7.3 (1H), 7.75 (2H), 8.1 (2H), 8.5 (2H), 11.6 (1H).

### Pharmakologische Daten:

Inhibition des Na⁺/H⁺-Exchangers von Kaninchenerythrocyten Weiße Neuseeland-Kaninchen (Ivanovas) erhielten eine Standard-Diät mit 2% Cholesterin für sechs Wochen, um den Na⁺/H⁺-Austausch zu aktivieren und so den Na⁺-Influx in die Erythrocyten via Na⁺/H⁺-Austausch flammenphotometrisch bestimmen zu können. Das Blut wurde den Ohrarterien entnommen und durch 25 IE Kalium-Heparin ungerinnbar gemacht. Ein Teil jeder Probe wurde zur Doppelbestimmung des Hämatokrits durch Zentrifugieren benutzt. Aliquots von jeweils 100 µl dienten zur Messung des Na⁺-Ausgangsgehalts der Erythrocyten.

Um den Amilorid-sensitiven Natrium-Influx zu bestimmen, wurden 100 µl jeder Blutprobe in jeweils 5 ml eines hyperosmolaren Salz-Sucrose-Mediums (mmol/l: 140 NaCl, 3 KCI, 150 Sucrose, 0,1 Ouabain, 20 Tris-hydroxymethyl-aminomethan) bei pH 7,4 und 37°C inkubiert. Die Erythrocyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrocyten nach Inkubation errechnet. Der Amiloridhemmbare Natrium-Influx ergab sich aus der Differenz des Natriumgehalts der Erythrocyten nach Inkubation mit und ohne Amilorid 3 x 10⁻⁴ mol/l. Auf diese Weise wurde auch bei den erfindungsgemäßen Verbindungen verfahren.

Ergebnisse

| Inhibition des Na⁺/H⁺-Exchangers: | |
|---|---|
| Beispiel | IC₅₀ [µmol/l] |
| 1 | <1 |

## Patentansprüche

1. Verbindung der Formel I 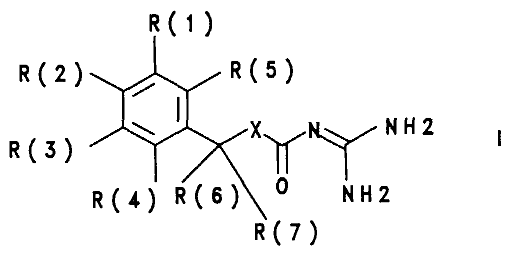 worin bedeuten:
R(1), R(2) und R(3)
unabhängig voneinander -Y-[4-R(8)-Phenyl], -Y-[3-R(8)-Phenyl] oder -Y-[2-R(8)-Phenyl],
wobei das Phenyl jeweils unsubstituiert oder substituiert ist mit 1 - 2 Substituenten aus der Gruppe bestehend aus F, Cl, -CF₃, Methyl, Hydroxy, Methoxy und -NR(96)R(97);
R(96) und R(97)
unabhängig voneinander Wasserstoff oder -CH₃;
Y eine Bindung, CH₂, Sauerstoff, -S- oder -NR(9);
R(9) Wasserstoff oder Alkyl mit 1, 2, 3 oder 4 C-Atomen;
R(8) SOₐ[NR(98)]_{b}NR(99)R(10);
a 1 oder 2;
b 0 oder 1;
a + b = 2;
R(98), R(99) und R(10)
unabhängig voneinander Wasserstoff, -(C₁-C₈)-Alkyl, Benzyl, -(C₂-C₈)-Alkylen-NR(11)R(12), (C₂-C₈)-Alkylen-NR(13)-(C₂-C₈)-Alkylen-NR(37)R(38) oder (C₀-C₈)-Alkylen-CR(39)R(40)-CR(41)R(42)(C₀-C₈)-Alkylen-NR(43)R(44);
R(11), R(12), R(13), R(37), R(38), R(43) und R(44)
unabhängig voneinander Wasserstoff, -(C₁-C₈)-Alkyl oder Benzyl:
R(39), R(40), R(41) und R(42)
unabhängig voneinander Wasserstoff, -(C₁-C₈)-Alkyl oder -(C₀-C₃)-Alkylen-Phenyl,
wobei das Phenyl nicht substituiert oder substituiert ist mit 1 - 3 Substituenten ausgewählt aus der Gruppe bestehend aus F, Cl,-CF₃, Methyl und Methoxy;
oder
R(99) und R(10)
gemeinsam 4 - 6 Methylengruppen, von denen eine CH₂-Gruppe durch Sauerstoff, -S-, -NH-, -N-CH₃ oder -N-Benzyl ersetzt sein kann;
oder
R(8) SOₐ[NR(98)]_{b}NR(95)-C[=N-R(94)]-NR(93)R(92);
R(92), R(93), R(94) und R(95)
unabhängig voneinander Wasserstoff oder Alkyl mit 1, 2, 3 oder 4 C-Atomen;
oder
R(1), R(2) und R(3)
unabhängig voneinander Wasserstoff, -(C₁-C₈)-Alkyl, -(C₂-C₈)-Alkenyl oder -(CH₂)ₘR(14);
m Null, 1 oder 2;
R(14) -(C₃-C₈)-Cycloalkyl oder Phenyl,
welches nicht substituiert oder substituiert ist mit 1 - 3 Substituenten ausgewählt aus der Gruppe bestehend aus F und Cl, -CF₃, Methyl, Methoxy und -NR(15)R(16);
R(15) und R(16)
Wasserstoff oder -CH₃;
oder
R(1), R(2) und R(3)
unabhängig voneinander -Q-4-[(CH₂)ₖ-CHR(17)-(C=O)R(20)]-Phenyl, -Q-3[(CH₂)ₖ-CHR(17)-(C=O)R(20)]-Phenyl oder -Q-2-[(CH₂)ₖ-CHR(17)-(C=O)R(20)]-Phenyl,
wobei das Phenyl jeweils unsubstituiert oder substituiert ist mit 1 - 2 Substituenten aus der Gruppe F, Cl, -CF₃, Methyl, Hydroxy, Methoxy und -NR(35)R(36);
R(35) und R(36)
unabhängig voneinander Wasserstoff oder -CH₃;
Q eine Bindung, Sauerstoff, -S- oder -NR(18);
R(18) Wasserstoff oder -(C₁-C₄)-Alkyl;
R(17) -OR(21) oder -NR(21)R(22);
R(21) und R(22)
unabhängig voneinander Wasserstoff, -(C₁-C₈)-Alkyl, -(C₁-C₈)-Alkanoyl, -(C₁-C₈)-Alkoxycarbonyl, Benzyl, Benzyloxycarbonyl;
oder
R(21) Trityl;
R(20) -OR(23) oder -NR(23)R(24);
R(23) und R(24) unabhängig voneinander
Wasserstoff, -(C₁-C₈)-Alkyl oder Benzyl;
k Null, 1, 2, 3 oder 4;
oder
R(1), R(2) und R(3)
unabhängig voneinander (C₁-C₉)-Heteroaryl,
das über C oder N verknüpft ist und das unsubstituiert oder substituiert ist mit 1-3 Substituenten aus der Gruppe F, Cl, CF₃, CH₃, Methoxy, Hydroxy, Amino, Methylamino und Dimethylamino;
oder
R(1), R(2) und R(3)
-SR(25), -OR(25), -NR(25)R(26), -CR(25)R(26)R(27);
R(25) -C_{f}H_{2f}-(C₁-C₉)-Heteroaryl,
das unsubstituiert oder substituiert ist mit 1-3 Substituenten aus der Gruppe F, Cl, CF₃, CH₃, Methoxy, Hydroxy, Amino, Methylamino und Dimethylamino;
f Null, 1 oder 2;
R(26) und R(27)
unabhängig voneinander wie R(25) definiert oder Wasserstoff oder (C₁-C₄)-Alkyl;
oder
R(1), R(2) und R(3)
unabhängig voneinander (C₁-C₉)-Heteroaryl-N-Oxid,
das über C oder N verknüpft ist und das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
oder
R(1), R(2) und R(3)
unabhängig voneinander -SR(28), -OR(28), -NR(28)R(29) oder -CR(28)R(29)R(30);
R(28) -C_{g}H_{2g}-(C₁-C₉)-Heteroaryl-N-Oxid,
das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
g Null, 1 oder 2;
R(29) und R(30)
unabhängig voneinander wie R(28) definiert, Wasserstoff oder (C₁-C₄)-Alkyl;
oder
R(1), R(2) und R(3)
unabhängig voneinander Wasserstoff, F, Cl, Br, l, -CN, T-(CH₂)ₕ-(CᵢF₂ᵢ₊₁), R(31)SOₗ-, R(32)R(33)N-CO-, R(34)-CO- oder R(45)R(46)N-SO₂, wobei die Perfluoralkylgruppe geradkettig oder verzweigt ist;
T eine Bindung, Sauerstoff, -S- oder -NR(47);
l Null, 1 oder 2;
h Null, 1 oder 2;
i 1, 2, 3, 4, 5 oder 6;
R(31), R(32), R(34) und R(45)
unabhängig voneinander -(C₁-C₈)-Alkyl, -(C₃-C₆)Alkenyl, (CH₂)ₙR(48) oder -CF₃;
n Null, 1, 2, 3 oder 4;
R(47) Wasserstoff oder Alkyl mit 1, 2 oder 3 C-Atomen;
R(48) -(C₃-C₇)-Cycloalkyl oder Phenyl,
welches nicht substituiert ist oder substituiert mit 1 - 3 Substituenten ausgewählt aus der Gruppe bestehend aus F, Cl, -CF₃, Methyl, Methoxy und -NR(49)R(50);
R(49) und R(50)
Wasserstoff oder Alkyl mit 1, 2, 3 oder 4 C-Atomen;
oder
R(32), R(34) und R(45)
Wasserstoff;
R(33) und R(46)
unabhängig voneinander Wasserstoff oder Alkyl mit 1, 2, 3 oder 4 C-Atomen;
oder
R(32) und R(33) sowie R(45) und R(46)
gemeinsam 5 oder 6 Methylengruppen, von denen eine CH₂-Gruppe durch Sauerstoff, -S-, -NH-, -NCH₃ oder -N-Benzyl ersetzt sein kann;
oder
R(1), R(2) und R(3)
unabhängig voneinander R(51)-A-G-D-;
R(51) ein basischer protonierbarer Rest, d.h. eine Aminogruppe -NR(52)R(53), eine Amidinogruppe R(52)R(53)N-C[=N-R(54)]- oder eine Guanidinogruppe R(52)R(53)N-C[=N-R(54)]-NR(55)-;
R(52), R(53), R(54) und R(55)
unabhängig voneinander Wasserstoff oder Alkyl mit 1, 2, 3 oder 4 C-Atomen;
oder
R(52) und R(53)
eine Gruppe C_{α}H_{2α};
α 4, 5, 6 oder 7;
wobei im Falle von α = 5, 6 oder 7 ein C-Atom der Gruppe C_{α}H_{2α} durch eine Heteroatomgruppe O, SO_{d} oder NR(56) ersetzt sein kann,
oder
R(53) und R(54) oder R(54) und R(55) oder R(52) und R(55)
eine Gruppe C_{γ}H_{2γ};
γ 2, 3, 4 oder 5;
wobei im Falle von γ = 3, 4 oder 5 ein C-Atom der Gruppe C_{γ}H_{2γ} durch eine Heteroatomgruppe O, SO_{d} oder NR(56) ersetzt sein kann;
d Null, 1 oder 2;
R(56) Wasserstoff oder Methyl;
oder
R(51) ein basisches heteroaromatisches Ringsystem mit 1 - 9 C-Atomen;
A eine Gruppe CₑH₂ₑ;
e Null, 1, 2, 3, 4, 5, 6, 7, 8, 9 oder 10;
wobei in der Gruppe CₑH₂ₑ ein C-Atom durch eine der Gruppierungen -O-, -CO- oder SOᵣ- ersetzt sein kann;
r Null, 1 oder 2;
G einen Phenylenrest, 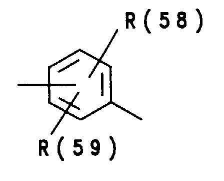 R(58) und R(59)
unabhängig voneinander Wasserstoff, Methyl, Methoxy, F, Cl, Br, J, CF₃ oder -SOₛ-R(60);
R(60) Methyl oder NR(61)R(62);
R(61) und R(62)
unabhängig voneinander Wasserstoff oder Alkyl mit 1, 2, 3 oder 4 C-Atomen;
D -CᵥH₂ᵥ-E_{w}-;
v Null, 1, 2, 3 oder 4;
E -O-, -CO-, -CH[OR(63)]-, -SOₐₐ- oder -NR(63)-;
w Null oder 1;
aa Null, 1 oder 2
R(63) Wasserstoff oder Methyl,
oder
R(1), R(2) und R(3)
unabhängig voneinander -CF₂R(64), -CF[R(65)][R(66)], -CF[(CF₂)_{q}-CF₃)][R(65)], -C[(CF₂)ₚ-CF₃]=CR(65)R(66);
R(64) Alkyl mit 1,2,3 oder 4 C-Atomen oder Cycloalkyl mit 3, 4, 5, 6 oder 7 C-Atomen;
R(65) und R(66) unabhängig voneinander Wasserstoff oder Alkyl mit 1, 2, 3 oder 4 C-Atomen;
q Null, 1 oder 2;
p Null, 1 oder 2;
oder
R(1), R(2) und R(3)
unabhängig voneinander -OR(67) oder -NR(67)R(68);
R(67) und R(68)
unabhängig voneinander Wasserstoff oder Alkyl mit 1, 2, 3, 4, 5 oder 6 C-Atomen;
oder
R(67) und R(68)
gemeinsam 4, 5, 6 oder 7 Methylengruppen, von denen eine CH₂-Gruppe durch Sauerstoff, -S-, SO₂, -NH-, -NCH₃ oder -N-Benzyl ersetzt sein kann;
R(4) und R(5)
unabhängig voneinander Wasserstoff, Alkyl mit 1, 2, 3 oder 4 C-Atomen, F, Cl, -OR(69), -NR(70)R(71) oder -C_{z}F_{2z+1};
R(69), R(70) und R(71)
unabhängig voneinander Wasserstoff oder Alkyl mit 1, 2 oder 3 C-Atomen;
z 1, 2, 3 oder 4;
R(6) und R(7)
unabhängig voneinander Wasserstoff oder Alkyl mit 1, 2, 3 oder 4 C-Atomen;
X Sauerstoff oder NR(72);
R(72) Wasserstoff oder Alkyl mit 1, 2, 3 oder 4 C-Atomen;
sowie deren pharmazeutisch verträgliche Salze;
wobei jedoch Verbindungen ausgenommen sind, in denen die Reste R(1) bis R(7) sowie R(72) gleich Wasserstoff sind.

2. Verbindung der Formel I nach Anspruch 1, dadurch gekennzeichnet, daß darin bedeuten:
R(1), R(2) und R(3)
unabhängig voneinander -Y-[4-R(8)-Phenyl], -Y-[3-R(8)-Phenyl] oder -Y-[2-R(8)-Phenyl],
wobei das Phenyl jeweils unsubstituiert oder substituiert ist mit einem Substituenten aus der Gruppe bestehend aus F, Cl, -CF₃, Methyl, Methoxy und -NR(96)R(97);
R(96) und R(97)
unabhängig voneinander Wasserstoff oder -CH₃;
Y eine Bindung, Sauerstoff, -S- oder -NR(9);
R(9) Wasserstoff oder Methyl;
R(8) SOₐ[NR(98)]_{b}NR(99)R(10);
a 1 oder 2;
b 0 oder 1;
a + b = 2;
R(98) Wasserstoff, Alkyl mit 1, 2, 3, 4 oder 5 C-Atomen oder Benzyl;
R(99) und R(10)
unabhängig voneinander Wasserstoff, Alkyl mit 1 oder 2 C-Atomen, Benzyl, -(C₂-C₃)-Alkylen-NR(11)R(12), (C₂-C₃)-Alkylen-NR(13)-(C₂-C₃)-Alkylen-NR(37)R(38) oder (C₀-C₂)-Alkylen-CR(39)R(40)-CR(41)R(42)(C₀-C₂)-Alkylen-NR(43)R(44);
R(11), R(12), R(13), R(37), R(38), R(43) und R(44)
unabhängig voneinander Wasserstoff, Methyl oder Ethyl;
R(39), R(40), R(41) und R(42)
unabhängig voneinander Wasserstoff, Alkyl mit 1, 2, 3 oder 4 C-Atomen oder Benzyl,
wobei das Phenyl nicht substituiert oder substituiert ist mit einem Substituenten ausgewählt aus der Gruppe bestehend aus F, Cl,-CF₃, Methyl und Methoxy;
oder
R(99) und R(10)
gemeinsam 4, 5 oder 6 Methylengruppen, von denen eine CH₂-Gruppe durch Sauerstoff, -S-, -NH-, oder -N-CH₃ ersetzt sein kann;
oder
R(8) SOₐ[NR(98)]_{b}NR(95)-C[=N-R(94)]-NR(93)R(92);
R(95) Wasserstoff;
R(92), R(93) und R(94)
unabhängig voneinander Wasserstoff oder Alkyl mit 1, 2, 3 oder 4 C-Atomen;
oder
R(1), R(2) und R(3)
unabhängig voneinander Wasserstoff oder Alkyl mit 1, 2, 3 oder 4 C-Atomen;
oder
R(1), R(2) und R(3)
unabhängig voneinander -Q-4-[(CH₂)ₖ-CH(NR(21)R(22))-(C=O)R(20)]-Phenyl, -Q-3-[(CH₂)ₖ-CH(NR(21)R(22))-(C=O)R(20)]-Phenyl oder -Q-2-[(CH₂)ₖ-CH(NR(21)R(22))-(C=O)R(20)]-Phenyl,
wobei das Phenyl jeweils unsubstituiert oder substituiert ist mit 1 - 2 Substituenten aus der Gruppe F, Cl, -CF₃, Methyl, Hydroxy, Methoxy und -NR(35)R(36);
R(35) und R(36)
unabhängig voneinander Wasserstoff oder -CH₃;
Q eine Bindung, Sauerstoff, -S- oder -NR(18);
R(18) Wasserstoff oder -(C₁-C₄)-Alkyl;
R(21) und R(22)
unabhängig voneinander Wasserstoff, -(C₁-C₅)-Alkyl, -(C₁-C₅)-Alkanoyl, -(C₁-C₅)-Alkoxycarbonyl, Benzyl, Benzyloxycarbonyl;
oder
R(21) Trityl;
R(20) -OR(23) oder -NR(23)R(24);
R(23) und R(24)
unabhängig voneinander Wasserstoff, -(C₁-C₄)-Alkyl oder Benzyl;
k Null, 1 oder 2;
oder
R(1), R(2) und R(3)
unabhängig voneinander (C₁-C₉)-Heteroaryl, das über C oder N verknüpft ist und das unsubstituiert oder substituiert ist mit einem Substituenten aus der Gruppe F, Cl, CF₃, CH₃, Methoxy und Dimethylamino;
oder
R(1), R(2) und R(3)
-SR(25), -OR(25), -NR(25)R(26), -CR(25)R(26)R(27);
R(25) -C_{f}H_{2f}-(C₁-C₉)-Heteroaryl,
das unsubstituiert oder substituiert ist mit einem Substituenten aus der Gruppe F, Cl, CF₃, CH₃, Methoxy, Dimethylamino;
f Null, 1 oder 2;
R(26) und R(27)
unabhängig voneinander wie R(25) definiert oder Wasserstoff oder Methyl;
oder
R(1), R(2) und R(3)
unabhängig voneinander (C₁-C₉)-Heteroaryl-N-Oxid,
das über C oder N verknüpft ist und das unsubstituiert oder substituiert ist mit 1 - 2 Substituenten ausgewählt aus der Gruppe bestehend aus F, Cl, CF₃, CH₃, Methoxy, Hydroxy, Amino, Methylamino und Dimethylamino;
oder
R(1), R(2) und R(3)
unabhängig voneinander -SR(28), -OR(28), -NR(28)R(29) oder -CR(28)R(29)R(30);
R(28) -C_{g}H_{2g}-(C₁-C₉)-Heteroaryl-N-Oxid,
das unsubstituiert oder substituiert ist mit 1 - 2 Substituenten ausgewählt aus der Gruppe bestehend aus F, Cl, CF₃, CH₃, Methoxy, Hydroxy, Amino, Methylamino und Dimethylamino;
g Null oder 1;
R(29) und R(30)
unabhängig voneinander wie R(28) definiert, Wasserstoff oder Methyl;
oder
R(1) und R(3)
unabhängig voneinander Wasserstoff, F, Cl, CF₃, R(31)SO₂-, R(32)R(33)N-CO-, R(34)-CO- oder R(45)R(46)N-SO₂;
R(31) und R(34)
unabhängig voneinander Methyl oder -CF₃;
R(32), R(33), R(45) und R(46)
unabhängig voneinander Wasserstoff oder Methyl;
oder
R(1), R(2) und R(3)
unabhängig voneinander R(51)-A-G-D-;
R(51) -NR(52)R(53), eine Amidinogruppe R(52)R(53)N-C[=N-R(54)]- oder eine Guanidinogruppe R(52)R(53)N-C[=N-R(54)]-NR(55)-;
R(52), R(53), R(54) und R(55)
unabhängig voneinander Wasserstoff oder Alkyl mit 1, 2, 3 oder 4 C-Atomen;
oder
R(52) und R(53)
eine Gruppe C_{α}H_{2α};
α 4, 5, 6 oder 7;
wobei im Falle von α = 5, 6 oder 7 ein C-Atom der Gruppe C_{α}H_{2α} durch eine Heteroatomgruppe O, SO_{d} oder NR(56) ersetzt sein kann;
oder
R(53) und R(54)
eine Gruppe C_{γ}H_{2γ};
γ 2, 3, 4 oder 5;
wobei im Falle von γ = 3, 4 oder 5 ein C-Atom der Gruppe C_{γ}H_{2γ} durch eine Heteroatomgruppe O, SO_{d} oder NR(56) ersetzt sein kann;
d Null oder 2;
R(56) Wasserstoff oder Methyl;
oder
R(51) Imidazolyl, Pyridyl, Chinolinyl oder Isochinolinyl;
A eine Gruppe CₑH₂ₑ;
e Null, 1, 2, 3, 4 oder 5;
wobei in der Gruppe CₑH₂ₑ ein C-Atom durch eine der Gruppierungen -O-, -CO- oder -SOᵣ- ersetzt sein kann;
r Null oder 2;
G einen Phenylenrest, 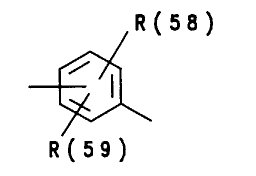 R(58) und R(59)
unabhängig voneinander Wasserstoff, Methyl, F, Cl, CF₃ oder -SO₂-R(60);
R(60) Methyl oder NR(61)R(62);
R(61) und R(62)
unabhängig voneinander Wasserstoff oder Methyl;
D -CᵥH₂ᵥ-E_{w}-;
v Null, 1, 2, 3 oder 4;
E -O-, -CO-, -CH[OR(63)]-, -SOₐₐ- oder -NR(63)-;
w Null oder 1;
aa Null oder 2
R(63) Wasserstoff oder Methyl;
oder
R(2) -CF₂R(64), -CF[R(65)][R(66)], -CF(CF₃)[R(65)], -C(CF₃)=CR(65)R(66); R(64) Alkyl mit 1, 2, 3 oder 4 C-Atomen oder Cycloalkyl mit 3, 4, 5, 6 oder 7 C-Atomen;
R(65) und R(66)
unabhängig voneinander Wasserstoff oder Alkyl mit 1, 2, 3 oder 4 C-Atomen;
oder
R(1), R(2) und R(3)
unabhängig voneinander -OR(67) oder -NR(67)R(68);
R(67) und R(68)
unabhängig voneinander Wasserstoff oder Alkyl mit 1, 2 oder 3 C-Atomen;
oder
R(67) und R(68)
gemeinsam 4, 5 oder 6 Methylengruppen, von denen eine CH₂-Gruppe durch Sauerstoff, -S-, SO₂, -NH- oder -NCH₃ ersetzt sein kann;
R(4) und R(5)
unabhängig voneinander Wasserstoff, Alkyl mit 1, 2 oder 3 C-Atomen, F, Cl, -OR(69), -NR(70)R(71) oder -CF₃;
R(69), R(70) und R(71)
unabhängig voneinander Wasserstoff oder Methyl;
R(6) und R(7)
unabhängig voneinander Wasserstoff oder Methyl;
X Sauerstoff oder NR(72);
R(72) Wasserstoff oder Methyl;
sowie deren pharmazeutisch verträgliche Salze.

3. Verbindung der Formel I nach einem oder mehreren der Ansprüche 1 bis 2, dadurch gekennzeichnet, daß darin bedeuten:
R(1), R(2) und R(3)
unabhängig voneinander -O-[4-R(8)-Phenyl],
das Phenyl jeweils unsubstituiert oder substituiert ist mit einem Substituenten aus der Gruppe bestehend aus F, Cl, -CF₃, Methyl und Methoxy;
R(8) SOₐ[NR(98)]_{b}NR(99)R(10);
a 1 oder 2;
b 0 oder 1;
a + b = 2;
R(98) Wasserstoff oder Alkyl mit 1, 2, 3 oder 4 C-Atomen;
R(99) und R(10)
unabhängig voneinander Wasserstoff, Alkyl mit 1 oder 2 C-Atomen, Benzyl, -(C₂-C₃)-Alkylen-NR(11)R(12), (C₂-C₃)-Alkylen-NR(13)-(C₂-C₃)-Alkylen-NR(37)R(38) oder (C₀-C₂)-Alkylen-CR(39)R(40)-CR(41)R(42)(C₀-C₂)-Alkylen-NR(43)R(44);
R(11), R(12), R(13), R(37), R(38), R(43) und R(44)
unabhängig voneinander Wasserstoff, Methyl oder Ethyl;
R(39), R(40), R(41) und R(42)
unabhängig voneinander Wasserstoff, Alkyl mit 1, 2, 3 oder 4 C-Atomen oder Benzyl,
wobei das Phenyl nicht substituiert oder substituiert ist mit einem Substituenten ausgewählt aus der Gruppe bestehend aus F, Cl,-CF₃, Methyl und Methoxy;
oder
R(99) und R(10)
gemeinsam 4, 5 oder 6 Methylengruppen, von denen eine CH₂-Gruppe durch -NH-, oder -N-CH₃ ersetzt sein kann;
oder
R(8) SOₐ[NR(98)]_{b}NR(95)-C[=N-R(94)]-NR(93)R(92);
R(95) Wasserstoff;
R(92), R(93) und R(94)
unabhängig voneinander Wasserstoff oder Alkyl mit 1, 2, 3 oder 4 C-Atomen;
oder
R(1), R(2) und R(3)
unabhängig voneinander Wasserstoff oder Alkyl mit 1, 2, 3 oder 4 C-Atomen;
oder
R(1), R(2) und R(3)
unabhängig voneinander -Q-4-[(CH₂)ₖ-CH(NR(21)R(22))-(C=O)R(20)]-Phenyl,
wobei das Phenyl jeweils unsubstituiert oder substituiert ist mit einem Substituenten aus der Gruppe F, Cl, -CF₃, Methyl, Hydroxy und Methoxy;
Q eine Bindung oder Sauerstoff;
R(21) und R(22)
unabhängig voneinander Wasserstoff, Methyl, -(C₁-C₅)-Alkanoyl, -(C₁-C₅)-Alkoxycarbonyl, Benzyl oder Benzyloxycarbonyl;
R(20) -OR(23) oder -NR(23)R(24);
R(23) und R(24)
unabhängig voneinander Wasserstoff, -(C₁-C₄)-Alkyl oder Benzyl;
k Null, 1 oder 2;
oder
R(1), R(2) und R(3)
unabhängig voneinander Imidazolyl,
das über C oder N verknüpft ist und das unsubstituiert oder substituiert ist mit einem Substituenten aus der Gruppe F, Cl, CF₃, CH₃ oder Methoxy;
oder
R(1), R(2) und R(3)
-SR(25), -OR(25), -NR(25)R(26), -CR(25)R(26)R(27);
R(25) -(C₁-C₉)-Heteroaryl,
das unsubstituiert oder substituiert ist mit einem Substituenten aus der Gruppe F, Cl, CF₃, CH₃ oder Methoxy;
R(26) und R(27)
unabhängig voneinander Wasserstoff oder Methyl;
oder
R(1), R(2) und R(3)
unabhängig voneinander -SR(28), -OR(28), -NR(28)R(29) oder -CR(28)R(29)R(30);
R(28) -(C₁-C₉)-Heteroaryl-N-Oxid,
das unsubstituiert oder substituiert ist mit einem Substituenten ausgewählt aus der Gruppe bestehend F, Cl, CF₃, CH₃ und Methoxy;
R(29) und R(30)
unabhängig voneinander Wasserstoff oder Methyl;
oder
R(1) und R(3)
unabhängig voneinander Wasserstoff, F, Cl, CF₃, R(31)SO₂-, R(32)R(33)N-CO-, R(34)-CO- oder R(45)R(46)N-SO₂;
R(31) und R(34)
unabhängig voneinander Methyl oder -CF₃;
R(32), R(33), R(45) und R(46)
unabhängig voneinander Wasserstoff oder Methyl;
oder
R(2) R(51)-A-G-D-;
R(51) -NR(52)R(53), eine Amidinogruppe R(52)R(53)N-C[=N-R(54)]- oder eine Guanidinogruppe R(52)R(53)N-C[=N-R(54)]-NR(55)-;
R(52), R(53), R(54) und R(55)
unabhängig voneinander Wasserstoff oder Alkyl mit 1, 2, 3 oder 4 C-Atomen;
oder
R(52) und R(53)
eine Gruppe C_{α}H_{2α};
α 4, 5, 6 oder 7;
wobei im Falle von α = 5, 6 oder 7 ein C-Atom der Gruppe C_{α}H_{2α} durch eine Heteroatomgruppe O, SO_{d} oder NR(56) ersetzt sein kann,
oder
R(53) und R(54)
eine Gruppe C_{γ}H_{2γ};
γ 2, 3, 4 oder 5;
wobei im Falle von γ = 3, 4 oder 5 ein C-Atom der Gruppe C_{γ}H_{2γ} durch eine Heteroatomgruppe O, SO_{d} oder NR(56) ersetzt sein kann;
d Null oder 2;
R(56) Wasserstoff oder Methyl;
oder
R(51) Imidazolyl, Pyridyl, Chinolinyl oder Isochinolinyl;
A CₑH₂ₑ;
e Null, 1, 2, 3, 4 oder 5;
wobei in der Gruppe CₑH₂ₑ ein C-Atom durch eine der Gruppierungen -O-, -CO- oder -SOr ersetzt sein kann;
r Null oder 2;
G einen Phenylenrest, 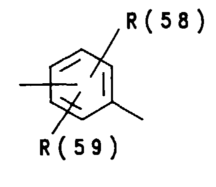 R(58) und R(59)
unabhängig voneinander Wasserstoff, Methyl, F, Cl, CF₃ oder -SO₂-R(60);
R(60) Methyl oder NR(61)R(62);
R(61) und R(62)
unabhängig voneinander Wasserstoff oder Methyl;
D -CᵥH₂ᵥ-E_{w}-;
v Null, 1, 2, 3 oder 4;
E -O-, -CO-, -CH[OR(63)]-, -SOₐₐ- oder -NR(63)-;
w Null oder 1;
aa Null oder 2
R(63) Wasserstoff oder Methyl,
oder
R(2) -CF₂R(64), -CF[R(65)][R(66)], -CF(CF₃)[R(65)], -C(CF₃)=CR(65)R(66);
R(64) Alkyl mit 1, 2, 3 oder 4 C-Atomen oder Cycloalkyl mit 3, 4, 5, 6 oder 7 C-Atomen;
R(65) und R(66)
unabhängig voneinander Wasserstoff oder Alkyl mit 1, 2, 3 oder 4 C-Atomen;
oder
R(1), R(2) und R(3)
unabhängig voneinander -OR(67) oder -NR(67)R(68);
R(67) und R(68)
unabhängig voneinander Wasserstoff oder Alkyl mit 1, 2 oder 3 C-Atomen;
oder
R(67) und R(68)
gemeinsam 4, 5 oder 6 Methylengruppen, von denen eine CH₂-Gruppe durch Sauerstoff, -S-, SO₂, -NH- oder -NCH₃ ersetzt sein kann;
R(4) und R(5)
unabhängig voneinander Wasserstoff, Alkyl mit 1, 2 oder 3 C-Atomen, F, Cl, -OR(69), -NR(70)R(71) oder -CF₃;
R(69), R(70) und R(71)
unabhängig voneinander Wasserstoff oder Methyl;
R(6) und R(7)
unabhängig voneinander Wasserstoff oder Methyl;
X Sauerstoff oder NR(72);
R(72) Wasserstoff oder Methyl.

4. Verbindung der Formel I nach einem oder mehreren der Ansprüche 1 bis 3, dadurch gekennzeichnet, daß darin bedeuten:
R(2) -O-[4-R(8)-Phenyl],
R(8) SOₐ[NR(98)]_{b}NR(99)R(10);
a 1 oder 2;
b 0 oder 1;
a + b = 2;
R(98) Wasserstoff oder Alkyl mit 1, 2, 3 oder 4 C-Atomen;
R(99) und R(10)
unabhängig voneinander Wasserstoff, Alkyl mit 1 oder 2 C-Atomen oder -(C₂-C₃)-Alkylen-NR(11)R(12);
R(11) und R(12)
unabhängig voneinander Wasserstoff, Methyl oder Ethyl;
oder
R(99) und R(10)
gemeinsam 5 oder 6 Methylengruppen, von denen eine CH₂-Gruppe durch -NH-, oder -N-CH₃ ersetzt sein kann;
oder
R(8) SOₐ[NR(98)]_{b}NR(95)-C[=N-R(94)]-NR(93)R(92);
R(95) Wasserstoff;
R(92), R(93) und R(94)
unabhängig voneinander Wasserstoff oder Alkyl mit 1, 2, 3 oder 4 C-Atomen;
oder
R(1), R(2) und R(3)
unabhängig voneinander Wasserstoff oder Alkyl mit 1, 2, 3 oder 4 C-Atomen;
oder
R(2) -O-4-[CH₂-CH(NR(21)R(22))-(C=O)R(20)]-Phenyl,
R(21) und R(22)
unabhängig voneinander Wasserstoff, Methyl, -(C₁-C₅)-Alkanoyl,-(C₁-C₅)-Alkoxycarbonyl, Benzyl oder Benzyloxycarbonyl;
R(20) -OR(23) oder -NR(23)R(24);
R(23) und R(24)
unabhängig voneinander Wasserstoff oder -(C₁-C₄)-Alkyl;
oder
R(2) Imidazolyl,
das über C oder N verknüpft ist,
oder
R(2) -SR(25) oder -OR(25);
R(25) Pyridyl, Chinolinyl oder Isochinolinyl, die jeweils unsubstituiert oder substituiert sind mit einem Substituenten aus der Gruppe bestehend aus F, Cl, CF₃, CH₃ und Methoxy;
oder
R(2) -SR(28) oder -OR(28);
R(28) Pyridyl-N-Oxid, Chinolinyl-N-Oxid oder Isochinolinyl-N-Oxid, die jeweils unsubstituiert oder substituiert sind mit einem Substituenten ausgewählt aus der Gruppe bestehend aus F, Cl, CF₃, CH₃ und Methoxy;
oder
R(1) Wasserstoff, F, Cl, CF₃, R(31)SO₂- oder R(45)R(46)N-SO₂;
R(31)
unabhängig voneinander Methyl oder -CF₃;
R(45) und R(46)
unabhängig voneinander Wasserstoff oder Methyl;
oder
R(2) R(51)-A-G-O-;
R(51) -NR(52)R(53),
R(52) und R(53)
unabhängig voneinander Wasserstoff oder Alkyl mit 1 oder 2 C-Atomen;
oder
R(52) und R(53)
C_{α}H_{2α};
α 5 oder 6;
wobei ein C-Atom der Gruppe C_{α}H_{2α} durch NR(56) ersetzt sein kann;
R(56) Wasserstoff oder Methyl;
oder
R(51) Imidazolyl, Pyridyl, Chinolinyl oder Isochinolinyl;
A CₑH₂ₑ;
e Null, 1, 2 oder 3;
wobei in der Gruppe CₑH₂ₑ ein C-Atom durch eine der Gruppierungen -O-, -CO- oder -SOᵣ- ersetzt sein kann;
r Null oder 2;
G einen Phenylenrest, 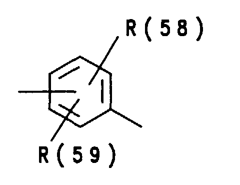 R(58) und R(59)
unabhängig voneinander Wasserstoff, Methyl, F, Cl, CF₃ oder -SO₂-R(60);
R(60) Methyl oder NR(61)R(62);
R(61) und R(62)
unabhängig voneinander Wasserstoff oder Methyl;
oder
R(2) -CF₂R(64), -CF[R(65)][R(66)], -CF(CF₃)[R(65)], -C(CF₃)=CR(65)R(66);
R(64) Alkyl mit 1, 2, 3 oder 4 C-Atomen oder Cycloalkyl mit 5 oder 6 C-Atomen;
R(65) und R(66)
unabhängig voneinander Wasserstoff oder Alkyl mit 1, 2, 3 oder 4 C-Atomen;
oder
R(1), R(2) und R(3)
unabhängig voneinander -OR(67) oder -NR(67)R(68);
R(67) und R(68)
unabhängig voneinander Wasserstoff, Methyl oder Ethyl;
oder
R(67) und R(68)
gemeinsam 4 oder 5 Methylengruppen, von denen eine CH₂-Gruppe durch Sauerstoff, -NH- oder -NCH₃ ersetzt sein kann;
R(4) und R(5)
unabhängig voneinander Wasserstoff, Alkyl mit 1, 2 oder 3 C-Atomen, F, Cl oder -CF₃;
R(6) und R(7)
unabhängig voneinander Wasserstoff oder Methyl;
X Sauerstoff oder NR(72)
R(72) Wasserstoff oder Methyl.

5. Verbindung der Formel I nach Anspruch 1, ausgewählt aus der Gruppe bestehend aus
4-(3-Pyridyloxy)-3-trifluormethyl-benzyloxycarbonylguanidin Dihydrochlorid,
4-(6-Chinaldinyloxy)-3-methylsulfonyl-benzyloxycarbonylguanidin Dihydrochlorid,
4-(6-Chinaldinyloxy)-3-trifluormethyl-benzyloxycarbonylguanidin Dihydrochlorid,
4-lsopropyl-3-methylsulfonyl-benzyloxycarbonylguanidin Hydrochlorid,
3-Isopropyl-benzyloxycarbonylguanidin Hydrochlorid,
2-Chlor-5-trifluormethyl-benzyloxycarbonylguanidin Hydrochlorid,
4-(6-Chinolinyloxy)-3-methylsulfonyl-benzyloxycarbonylguanidin Dihydrochlorid,
3-Brom-5-fluor-benzylamino-carbonylguanidid-hydrochlorid,
3,5-Dimethylbenzylamino-carbonylguanidid-hydrochlorid,
2-Fluorbenzylamino-carbonylguanidid-hydrochlorid,
3-Fluorbenzylamino-carbonylguanidid-hydrochlorid,
2,6-Difluorbenzylamino-carbonylguanidid-hydrochlorid,
2,5-Difluorbenzylamino-carbonylguanidid-hydrochlorid,
3-Fluor-5-trifluormethylbenzylamino-carbonylguanidid-hydrochlorid,
4-Dimethylaminobenzylamino-carbonylguanidid-hydrochlorid,
3,5-Difluorbenzylamino-carbonylguanidid-hydrochlorid,
3-Methylbenzylamino-carbonylguanidid-hydrochlorid,
N-3,5-Difluorbenzyl-N-methyl-amino-carbonylguanidid-hydrochlorid,
3,5-Difluorbenzyloxy-carbonylguanidid-hydrochlorid,
2,5-Difluorbenzyloxy-carbonylguanidid-hydrochlorid,
2,3,6-Trifluorbenzyloxy-carbonylguanidid-hydrochlorid,
N-(3,4-Dichlorbenzyl)-N-methyl-aminocarbonylguanidin-hydrochlorid,
N-(2-Chlor-5-trifluormethyl-benzyl)-N-methyl-aminocarbonylguanidin-hydrochlorid,
N-Methyl-N-(3-methylsulfonyl-4-isopropyl-benzyl)-aminocarbonylguanidinmethansulfonat,
N-(4-Fluor-3-trifluormethyl-benzyl)-N-methyl-aminocarbonylguanidin-hydrochlorid,
4-lsopropyl-3-methylsulfonyl-benzyloxycarbonylguanidin-hydrochlorid,
2-Chlor-5-trifluormethyl-benzyloxycarbonylguanidin-hydrochlorid,
3-lsopropyl-benzyloxycarbonylguanidin-hydrochlorid,
4-(6-Chinolyloxy)-3-methylsulfonyl-benzyloxycarbonylguanidin-hydrochlorid und
3-Trifluormethyl-4-methoxy-benzyloxycarbonylguanidin-hydrochlorid.

6. Verfahren zum Herstellen einer Verbindung der Formel I nach Anspruch 1,
dadurch gekennzeichnet, daß man
eine Verbindung der Formel IV 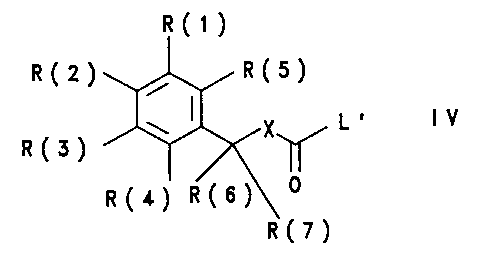 worin R(1) bis R(7) und X die in Anspruch 1 angegebenen Bedeutungen haben, und worin L' Chlor, Ethoxy, Isobutoxy, Benzotriazol-1-oxy oder 1-Imidazolyl bedeutet, mit Guanidin umsetzt.

7. Verwendung einer Verbindung I nach Anspruch 1 zur Herstellung eines Medikaments zu Behandlung von Arrhythmien.

8. Verwendung einer Verbindung I nach Anspruch 1 zur Herstellung eines Medikaments zur Behandlung oder Prophylaxe des Herzinfarkts.

9. Verwendung einer Verbindung I nach Anspruch 1 zur Herstellung eines Medikaments zur Behandlung oder Prophylaxe der Angina Pectoris.

10. Verwendung einer Verbindung I nach Anspruch 1 zur Herstellung eines Medikaments zur Behandlung oder Prophylaxe von ischämischen Zuständen des Herzens.

11. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

12. Verwendung einer Verbindung I nach Anspruch 1 zur Herstellung eines Medikaments zur Behandlung oder Prophylaxe von ischämischen Zuständen peripherer Organe und Gliedmaßen.

13. Verwendung einer Verbindung I nach Anspruch 1 zur Herstellung eines Medikaments zur Behandlung von Schockzuständen.

14. Verwendung einer Verbindung I nach Anspruch 1 zur Herstellung eines Medikaments zum Einsatz bei chirurgischen Operationen und Organtransplantationen.

15. Verwendung einer Verbindung I nach Anspruch 1 zur Herstellung eines Medikaments zur Konservierung und Lagerung von Transplantaten für chirurgische Maßnahmen.

16. Verwendung einer Verbindung I nach Anspruch.1 zur Herstellung eines Medikaments zur Behandlung von Krankheiten, bei denen die Zellproliferation eine primäre oder sekundäre Ursache darstellt.

17. Heilmittel, enthaltend eine wirksame Menge einer Verbindung I nach einem oder mehreren der Ansprüche 1 bis 3.

## Claims

1. A compound of the formula I 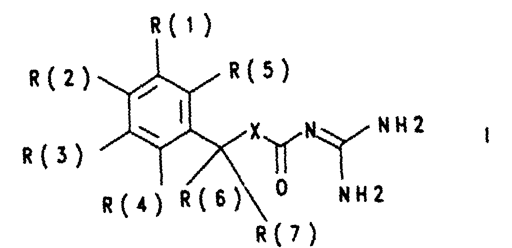 in which:
R(1), R(2) and R(3)
independently of one another are -Y-[4-R(8)-phenyl], -Y-[3-R(8)-phenyl] or -Y-[2-R(8)-phenyl],
where the phenyl in each case is unsubstituted or substituted by 1 - 2 substituents from the group consisting of F, Cl, -CF₃, methyl, hydroxyl, methoxy and -NR(96)R(97);
R(96) and R(97)
independently of one another are hydrogen or -CH₃;
Y is a bond, CH₂, oxygen, -S- or -NR(9);
R(9) is hydrogen or alkyl having 1, 2, 3 or 4 carbon atoms;
R(8) is SOₐ[NR(98)]_{b}NR(99)R(10);
a is 1 or 2;
b is 0 or 1;
a + b = 2;
R(98), R(99) and R(10)
independently of one another are hydrogen, -(C₁-C₃)-alkyl, benzyl, -(C₂-C₈)-alkylene-NR(11)R(12), (C₂-C₈)-alkylene-NR(13)-(C₂-C₈)-alkylene-NR(37)R(38) or (C₀-C₈)-alkylene-CR(39)R-(40)-CR(41)R(42) (C₀-C₈)-alkylene-NR(43)R(44);
R(11), R(12), R(13), R(37), R(38), R(43) and R(44)
independently of one another are hydrogen, -(C₁-C₈)-alkyl or benzyl:
R(39), R(40), R(41) and R(42)
independently of one another are hydrogen, -(C₁-C₈)-alkyl or - (C₀-C₃)-alkylenephenyl,
where the phenyl is unsubstituted or substituted by 1 - 3 substituents selected from the group consisting of F, Cl, -CF₃, methyl and methoxy;
or
R(99) and R(10)
together are 4 - 6 methylene groups, of which one CH₂ group can be replaced by oxygen, -S-, -NH-, -N-CH₃ or -N-benzyl;
or
R(8) is SOₐ[NR(98)]_{b}NR(95)-C[=N-R(94)]-NR(93)R(92);
R(92), R(93), R(94) and R(95)
independently of one another are hydrogen or alkyl having 1, 2, 3 or 4 carbon atoms;
R(1), R(2) and R(3)
independently of one another are hydrogen, -(C₁-C₈)-alkyl, -(C₂-C₈)-alkenyl or -(CH₂)ₘR(14);
m is zero, 1 or 2;
R(14) is -(C₃-C₈)-cycloalkyl or phenyl,
which is unsubstituted or substituted by 1 - 3 substituents selected from the group consisting of F, Cl, -CF₃, methyl, methoxy and -NR(15)R(16);
R(15) and R(16)
are hydrogen or -CH₃;
or
R(1), R(2) and R(3)
independently of one another are -Q-4-[(CH₂)ₖ-CHR-(17)-(C=O)R(20)]-phenyl, -Q-3-[(CH₂)ₖ-CHR(17)-(C=O)R(20)]-phenyl or -Q-2-[(CH₂)ₖ-CHR(17)-(C=O)R(20)]-phenyl,
where the phenyl in each case is unsubstituted or substituted by 1 - 2 substituents from the group consisting of F, Cl, -CF₃, methyl, hydroxyl, methoxy and -NR(35)R(36);
R(35) and R(36)
independently of one another are hydrogen or -CH₃;
Q is a bond, oxygen, -S- or -NR(18);
R(18) is hydrogen or -(C₁-C₄)-alkyl;
R(17) is -OR(21) or -NR(21)R(22);
R(21) and R(22)
independently of one another are hydrogen, -(C₁-C₈)-alkyl, -(C₁-C₈)-alkanoyl, - (C₁-C₈)-alkoxycarbonyl, benzyl, benzyloxycarbonyl;
or
R(21) is trityl;
R(20) is -OR(23) or -NR(23)R(24);
R(23) and R(24) independently of one another are hydrogen, -(C₁-C₈)-alkyl or benzyl;
k is zero, 1, 2, 3 or 4;
or
R(1), R(2) and R(3)
independently of one another are (C₁-C₉)-heteroaryl,
which is linked via C or N and which is unsubstituted or substituted by 1-3 substituents from the group consisting of F, Cl, CF₃, CH₃, methoxy, hydroxyl, amino, methylamino and dimethylamino;
or
R(1), R(2) and R(3) are
-SR(25), -OR(25), -NR(25)R(26), -CR(25)R(26)R(27);
R(25) is -C_{f}H_{2f}- (C₁-C₉)-heteroaryl,
which is unsubstituted or substituted by 1-3 substituents from the group consisting of F, Cl, CF₃, CH₃, methoxy, hydroxyl, amino, methylamino and dimethylamino;
f is zero, 1 or 2;
R(26) and R(27)
independently of one another are defined as R(25) or are hydrogen or (C₁-C₄)-alkyl;
or
R(1), R(2) and R(3)
independently of one another are (C₁-C₉)-heteroaryl N-oxide,
which is linked via C or N and which is unsubstituted or substituted by 1 - 3 substituents selected from the group consisting of F, Cl, CF₃, CH₃, methoxy, hydroxyl, amino, methylamino and dimethylamino;
or
R(1), R(2) and R(3)
independently of one another are -SR(28), -OR(28), -NR(28)R(29) or -CR(28)R(29)R(30);
R(28) is -C_{g}H_{2g}-(C₁-C₉)-heteroaryl N-oxide,
which is unsubstituted or substituted by 1 - 3 substituents selected from the group consisting of F, Cl, CF₃, CH₃, methoxy, hydroxyl, amino, methylamino and dimethylamino;
g is zero, 1 or 2;
R(29) and R(30)
independently of one another are defined as R(28), or are hydrogen or (C₁-C₄)-alkyl;
or
R(1), R(2) and R(3)
independently of one another are hydrogen, F, Cl, Br, I, -CN, T-(CH₂)ₕ-(CᵢF₂ᵢ₊ₗ), R(31)SO₁-, R(32)R(33)N-CO-, R(34)-CO- or R(45)R(46)N-SO₂, where the perfluoroalkyl group is straight-chain or branched;
T is a bond, oxygen, -S- or -NR(47);
l is zero, 1 or 2;
h is zero, 1 or 2;
i is 1, 2, 3, 4, 5 or 6;
R(31), R(32), R(34) and R(45)
independently of one another are -(C₁-C₈)-alkyl, -(C₃-C₆)-alkenyl, (CH₂)ₙR(48) or -CF₃;
n is zero, 1, 2, 3 or 4;
R(47) is hydrogen or alkyl having 1, 2 or 3 carbon atoms;
R(48) is -(C₃-C₇)-cycloalkyl or phenyl,
which is unsubstituted or substituted by 1 - 3 substituents selected from the group consisting of F, Cl, -CF₃, methyl, methoxy and -NR(49)R(50);
R(49) and R(50) are
hydrogen or alkyl having 1, 2, 3 or 4 carbon atoms;
or
R(32), R(34) and R(45) are
hydrogen;
R(33) and R(46)
independently of one another are hydrogen or alkyl having 1, 2, 3 or 4 carbon atoms;
or
R(32) and R(33) and also R(45) and R(46)
together are 5 or 6 methylene groups, of which one CH₂ group can be replaced by oxygen, -S-, -NH-, -NCH₃ or -N-benzyl;
or
R(1), R(2) and R(3)
independently of one another are R(51)-A-G-D-;
R(51) is a basic protonatable radical, i.e. an amino group -NR(52)R(53), an amidino group R(52)R(53)N-C[=N-R(54)]- or a guanidino group R(52)R(53)N-C[=N-R(54)]-NR(55)-;
R(52), R(53), R(54) and R(55)
independently of one another are hydrogen or alkyl having 1, 2, 3 or 4 carbon atoms;
or
R(52) and R(53) are
a group C_{α}H_{2α};
α is 4, 5, 6 or 7;
where if α = 5, 6 or 7 a carbon atom of the group C_{α}H_{2α} can be replaced by a heteroatom group O, SO_{d} or NR(56),
or
R(53) and R(54) or R(54) and R(55) or R(52)
and R(55) are
a group C_{γ}H_{2γ};
γ is 2, 3, 4 or 5;
where if γ = 3, 4 or 5 a carbon atom of the group C_{γ}H_{2γ} can be replaced by a heteroatom group O, SO_{d} or NR(56);
d is zero, 1 or 2;
R(56) is hydrogen or methyl;
or
R(51) is a basic heteroaromatic ring system having 1 - 9 carbon atoms;
A is a group CₑH₂ₑ;
e is zero, 1, 2, 3, 4, 5, 6, 7, 8, 9 or 10;
where in the group CₑH₂ₑ a carbon atom can be replaced by one of the groups -O-, -CO- or SOᵣ-;
r is zero, 1 or 2;
G is a phenylene radical 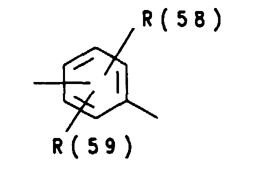 R(58) and R(59)
independently of one another are hydrogen, methyl, methoxy, F, Cl, Br, I,
CF₃ or -SOₛ-R(60);
R(60) is methyl or NR(61)R(62);
R(61) and R(62)
independently of one another are hydrogen or alkyl having 1, 2, 3 or 4 carbon atoms;
D is -CᵥH₂ᵥ-E_{w}-;
v is zero, 1, 2, 3 or 4;
E is -O-, -CO-, -CH[OR(63)]-, -SOₐₐ- or -NR(63)-;
w is zero or 1;
aa is zero, 1 or 2
R(63) is hydrogen or methyl,
or
R(1), R(2) and R(3)
independently of one another are -CF₂R(64), -CF[R(65)][R(66)], -CF[(CF₂)_{q}-CF₃)] [R(65)], -C[(CF₂)ₚ-CF₃]=CR(65)R(66);
R(64) is alkyl having 1, 2, 3 or 4 carbon atoms or cycloalkyl having 3, 4, 5, 6 or 7 carbon atoms;
R(65) and R(66) independently of one another are hydrogen or alkyl having 1, 2, 3 or 4 carbon atoms;
q is zero, 1 or 2;
p is zero, 1 or 2;
or
R(1), R(2) and R(3)
independently of one another are -OR(67) or -NR(67)R(68);
R(67) and R(68)
independently of one another are hydrogen or alkyl having 1, 2, 3, 4, 5 or 6 carbon atoms;
or
R(67) and R(68)
together are 4, 5, 6 or 7 methylene groups, of which one CH₂ group can be replaced by oxygen, -S-, SO ₂,
-NH-, -NCH₃ or -N-benzyl;
R(4) and R(5)
independently of one another are hydrogen, alkyl having 1, 2, 3 or 4 carbon atoms, F, Cl, -OR(69), -NR(70)R(71) or -C_{z}F_{2z+1};
R(69), R(70) and R(71)
independently of one another are hydrogen or alkyl having 1, 2 or 3 carbon atoms;
z is 1, 2, 3 or 4;
R(6) and R(7)
independently of one another are hydrogen or alkyl having 1, 2, 3 or 4 carbon atoms;
X is oxygen or NR(72);
R(72) is hydrogen or alkyl having 1, 2, 3 or 4 carbon atoms;
or its pharmaceutically tolerable salts;
but where compounds are excluded in which the radicals R(1) to R(7) and also R(72) are equal to hydrogen.

2. A compound of the formula I as claimed in claim 1, wherein:
R(1), R(2) and R(3)
independently of one another are -Y-[4-R(8)-phenyl], -Y-[3-R(8)-phenyl] or -Y-[2-R(8)-phenyl],
where the phenyl is in each case unsubstituted or substituted by a substituent from the group consisting of F, Cl, -CF₃, methyl, methoxy and -NR(96)R(97);
R(96) and R(97)
independently of one another are hydrogen or -CH₃;
Y is a bond, oxygen, -S- or -NR(9);
R(9) is hydrogen or methyl;
R(8) is SOₐ[NR(98)]_{b}NR(99)R(10);
a is 1 or 2;
b is 0 or 1;
a + b = 2;
R(98) is hydrogen, alkyl having 1, 2, 3, 4 or 5 carbon atoms or benzyl;
R(99) and R(10)
independently of one another are hydrogen, alkyl having 1 or 2 carbon atoms, benzyl, -(C₂-C₃)-alkylene-NR(11) R(12), (C₂-C₃)-alkylene-NR(13)-(C₂-C₃₎-alkylene-NR(37)R(38) or -(C₀-C₂)-alkylene-CR(39)R (40)-CR(41)R(42)(-Cₒ-C₂₎-alkylene-NR (43)R(44);
R(11), R(12), R(13), R(37), R(38), R(43) and R(44)
independently of one another are hydrogen, methyl or ethyl;
R(39), R(40), R(41) and R(42)
independently of one another are hydrogen, alkyl having 1, 2, 3 or 4 carbon atoms or benzyl,
where the phenyl is unsubstituted or substituted by a substituent selected from the group consisting of F, Cl, -CF₃, methyl and methoxy;
or
R(99) and R(10)
together are 4, 5 or 6 methylene groups, of which one CH₂ group can be replaced by oxygen, -S-, -NH- or -N-CH₃;
or
R(8) is SOₐ[NR(98)]_{b}NR(95)-C[=N-R(94)]-NR(93)R(92);
R(95) is hydrogen;
R(92), R(93) and R(94)
independently of one another are hydrogen or alkyl having 1, 2, 3 or 4 carbon atoms;
or
R(1), R(2) and R(3)
independently of one another are hydrogen or alkyl having 1, 2, 3 or 4 carbon atoms;
or
R(1), R(2) and R(3)
independently of one another are -Q-4-[(CH₂)ₖ-CH(NR(21)R(22))-(C=O)R(20)]-phenyl, -Q-3-[(CH₂)ₖ-CH(NR(21)R(22))-(C=O)R(20)]-phenyl or -Q-2-[(CH₂)ₖ-CH(NR(21)R(22))-(C=O)R(20)]-phenyl,
where the phenyl in each case is unsubstituted or substituted by 1 - 2 substituents from the group consisting of F, Cl, -CF₃, methyl, hydroxyl, methoxy and -NR(35)R(36);
R(35) and R(36)
independently of one another are hydrogen or -CH₃;
Q is a bond, oxygen, -S- or -NR(18);
R(18) is hydrogen or -(C₁-C₄)-alkyl;
R(21) and R(22)
independently of one another are hydrogen, - (C₁-C₅)-alkyl, - (C₁-C₅)-alkanoyl, - (C₁-C₅)-alkoxycarbonyl, benzyl, benzyloxycarbonyl;
or
R(21) is trityl;
R(20) is -OR(23) or -NR(23)R(24);
R(23) and R(24)
independently of one another are hydrogen, -(C₁-C₄)-alkyl or benzyl;
k is zero, 1 or 2;
or
R(1), R(2) and R(3)
independently of one another are (C₁-C₉)-heteroaryl, which is linked via C or N and which is unsubstituted or substituted by a substituent from the group consisting of F, Cl, CF₃, CH₃, methoxy and dimethylamino;
or
R(1), R(2) and R(3) are
-SR(25), -OR(25), -NR(25)R(26), -CR(25)R(26)R(27);
R(25) is -C_{f}H_{2f} -(C₁-C₉)-heteroaryl,
which is unsubstituted or substituted by a substituent from the group consisting of F, Cl, CF₃, CH₃, methoxy, dimethylamino;
f is zero, 1 or 2;
R(26) and R(27)
independently of one another are defined as R(25) or are hydrogen or methyl;
or
R(1), R(2) and R(3)
independently of one another are (C₁-C₉)-heteroaryl N-oxide,
which is linked via C or N and which is unsubstituted or substituted by 1 - 2 substituents selected from the group consisting of F, Cl, CF₃, CH₃, methoxy, hydroxyl, amino, methylamino and dimethylamino;
or
R(1), R(2) and R(3)
independently of one another are -SR(28), -OR(28), -NR(28)R(29) or -CR(28)R(29)R(30);
R(28) is -C_{g}H_{2g}-(C₁-C₉)-heteroaryl N-oxide,
which is unsubstituted or substituted by 1 - 2 substituents selected from the group consisting of F, Cl, CF₃, CH₃, methoxy, hydroxyl, amino, methylamino and dimethylamino;
g is zero or 1;
R(29) and R(30)
independently of one another are defined as R(28) or are hydrogen or methyl;
or
R(1), R(2) and R(3)
independently of one another are hydrogen, F, Cl, CF₃, R(31)SO₂-, R(32)R(33)N-CO-, R(34)-CO- or R(45)R(46)N-SO₂;
R(31) and R(34)
independently of one another are methyl or -CF₃;
R(32), R(33), R(45) and R(46)
independently of one another are hydrogen or methyl;
or
R(1), R(2) and R(3)
independently of one another are R(51)-A-G-D-;
R(51) is -NR(52)R(53), an amidino group R(52)R(53)N-C[=N-R(54)]- or a guanidino group R(52)R(53)N-C[=N-R(54)]-NR(55)-;
R(52), R(53), R(54) and R(55)
independently of one another are hydrogen or alkyl having 1, 2, 3 or 4 carbon atoms;
or
R(52) and R(53) are
a group C_{α}H_{2α};
α is 4, 5, 6 or 7;
where if α = 5, 6 or 7 a carbon atom of the group C_{α}H_{2α} can be replaced by a heteroatom group O, SO_{d} or NR(56);
or
R(53) and R(54) are
a group C_{γ}H_{2γ};
γ is 2, 3, 4 or 5;
where if γ = 3, 4 or 5 a carbon atom of the group C_{γ}H_{2γ} can be replaced by a heteroatom group O, SO_{d} or NR(56);
d is zero or 2;
R(56) is hydrogen or methyl;
or
R(51) is imidazolyl, pyridyl, quinolinyl or isoquinolinyl;
A is a group CₑH₂ₑ;
e is zero, 1, 2, 3, 4 or 5;
where in the group CₑH₂ₑ a carbon atom can be replaced by one of the groups -O-, -CO- or -SOᵣ-;
r is zero or 2;
G is a phenylene radical 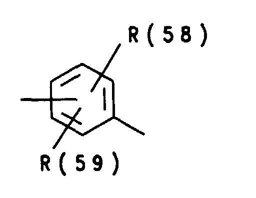 R(58) and R(59)
independently of one another are hydrogen, methyl, F, Cl, CF₃ or -SO₂-R(60);
R(60) is methyl or NR(61)R(62);
R(61) and R(62)
independently of one another are hydrogen or methyl;
D is -CᵥH₂ᵥ-E_{w}-;
v is zero, 1, 2, 3 or 4;
E is -O-, -CO-, -CH[OR(63)]-, -SOₐₐ- or -NR(63)-;
w is zero or 1;
aa is zero or 2
R(63) is hydrogen or methyl;
or
R(2) is -CF₂R(64), -CF[R(65)][R(66)], -CF(CF₃)[R(65)], -C(CF₃)=CR(65)R(66);
R(64) is alkyl having 1, 2, 3 or 4 carbon atoms or cycloalkyl having 3, 4, 5, 6 or 7 carbon atoms;
R(65) and R(66)
independently of one another are hydrogen or alkyl having 1, 2, 3 or 4 carbon atoms;
or
R(1), R(2) and R(3)
independently of one another are -OR(67) or -NR(67)R(68);
R(67) and R(68)
independently of one another are hydrogen or alkyl having 1, 2 or 3 carbon atoms;
or
R(67) and R(68)
together are 4, 5 or 6 methylene groups, of which one CH₂ group can be replaced by oxygen, -S-, SO ₂, -NH- or -NCH₃;
R(4) and R(5)
independently of one another are hydrogen, alkyl having 1, 2 or 3 carbon atoms, F, Cl, -OR(69), -NR(70)R(71) or -CF₃;
R(69), R(70) and R(71)
independently of one another are hydrogen or methyl;
R(6) and R(7)
independently of one another are hydrogen or methyl;
X is oxygen or NR(72);
R(72) is hydrogen or methyl;
or its pharmaceutically tolerable salts.

3. A compound of the formula I as claimed in one or more of claims 1 and 2, wherein:
R(1), R(2) and R(3)
independently of one another are -O-[4-R(8)-phenyl], the phenyl in each case is unsubstituted or substituted by a substituent from the group consisting of F, Cl, -CF₃, methyl and methoxy;
R(8) is SOₐ[NR(98)]_{b}NR(99)R(10);
a is 1 or 2;
b is 0 or 1;
a + b = 2;
R(98) is hydrogen or alkyl having 1, 2, 3 or 4 carbon atoms;
R(99) and R(10)
independently of one another are hydrogen, alkyl having 1 or 2 carbon atoms, benzyl, -(C₂-C₃)-alkylene-NR(11)R(12), (C₂-C₃) -alkylene-NR(13)-(C₂-C₃) -alkylene-NR(37)R(38) or (C₀-C₂)-alkylene-CR(39)R(40)-CR(41)R(42) (C₀-C₂)-alkylene-NR(43)R(44);
R(11), R(12), R(13), R(37), R(38), R(43) and R(44)
independently of one another are hydrogen, methyl or ethyl;
R(39), R(40), R(41) and R(42)
independently of one another are hydrogen, alkyl having 1, 2, 3 or 4 carbon atoms or benzyl,
where the phenyl is unsubstituted or substituted by a substituent selected from the group consisting of F, Cl, -CF₃, methyl and methoxy;
or
R(99) and R(10)
together are 4, 5 or 6 methylene groups, of which one CH₂ group can be replaced by -NH- or -N-CH₃;
or
R(8) is SOₐ[NR(98)]_{b}NR(95)-C[=N-R(94)]-NR(93)R(92);
R(95) is hydrogen;
R(92), R(93) and R(94)
independently of one another are hydrogen or alkyl having 1, 2, 3 or 4 carbon atoms;
or
R(1), R(2) and R(3)
independently of one another are hydrogen or alkyl having 1, 2, 3 or 4 carbon atoms;
or
R(1), R(2) and R(3)
independently of one another are -Q-4-[(CH₂)ₖ-CH (NR(21)R(22))-(C=O)R(20)]-phenyl,
where the phenyl in each case is unsubstituted or substituted by a substituent from the group consisting of F, Cl, -CF₃, methyl, hydroxyl and methoxy;
Q is a bond or oxygen;
R(21) and R(22)
independently of one another are hydrogen, methyl,-(C₁-C₅)-alkanoyl, -(C₁-C₅)-alkoxycarbonyl, benzyl or benzyloxycarbonyl;
R(20) is -OR(23) or -NR(23)R(24);
R(23) and R(24)
independently of one another are hydrogen, -(C₁-C₄)-alkyl or benzyl;
k is zero, 1 or 2;
or
R(1), R(2) and R(3)
independently of one another are imidazolyl,
which is linked via C or N and which is unsubstituted or substituted by a substituent from the group consisting of F, Cl, CF₃, CH₃ and methoxy;
or
R(1), R(2) and R(3) are
-SR(25), -OR(25), -NR(25)R(26), -CR(25)R(26)R(27);
R(25) is- (C₁-C₉)-heteroaryl,
which is unsubstituted or substituted by a substituent from the group consisting of F, Cl, CF₃, CH₃ and methoxy;
R(26) and R(27)
independently of one another are hydrogen or methyl;
or
R(1), R(2) and R(3)
independently of one another are -SR(28), -OR(28), -NR(28)R(29) or -CR(28)R(29)R(30);
R(28) is -(C₁-C₉)-heteroaryl N-oxide,
which is unsubstituted or substituted by a substituent selected from the group consisting of F, Cl, CF₃, CH₃ and methoxy;
R(29) and R(30)
independently of one another are hydrogen or methyl;
or
R(1), R(2) and R(3)
independently of one another are hydrogen, F, Cl, CF₃, R(31)SO₂-, R(32)R(33)N-CO-, R(34)-CO- or R(45)R(46)N-SO₂;
R(31) and R(34)
independently of one another are methyl or -CF₃;
R(32), R(33), R(45) and R(46)
independently of one another are hydrogen or methyl;
or
R(2) is R(51)-A-G-D-;
R(51) is -NR(52)R(53), an amidino group R(52)R(53)N-C[=N-R(54)]- or a guanidino group R(52)R(53)N-C[=N-R(54)]-NR(55)-;
R(52), R(53), R(54) and R(55)
independently of one another are hydrogen or alkyl having 1, 2, 3 or 4 carbon atoms;
or
R(52) and R(53) are
a group C_{α}H_{2α};
α is 4, 5, 6 or 7;
where if α = 5, 6 or 7 a carbon atom of the group C_{α}H_{2α} can be replaced by a heteroatom group O, SO_{d} or NR(56),
or
R(53) and R(54) are
a group C_{γ} H_{2γ};
γ is 2, 3, 4 or 5;
where if γ = 3, 4 or 5 a carbon atom of the group C_{γ}H_{2γ} can be replaced by a heteroatom group O, SO_{d} or NR(56);
d is zero or 2;
R(56) is hydrogen or methyl; or
R(51) is imidazolyl, pyridyl, quinolinyl or isoquinolinyl;
A is CₑH₂ₑ;
e is zero, 1, 2, 3, 4 or 5;
where in the group CₑH₂ₑ a carbon atom can be replaced by one of the groups -O-, -CO- or -SOᵣ-;
r is zero or 2;
G is a phenylene radical 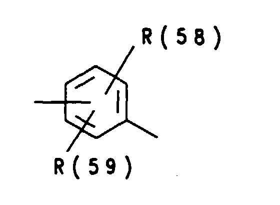 R(58) and R(59)
independently of one another are hydrogen, methyl, F, Cl, CF₃ or -SO₂-R(60);
R(60) is methyl or NR(61)R(62);
R(61) and R(62)
independently of one another are hydrogen or methyl;
D is -CᵥH₂ᵥ-E_{w}-;
v is zero, 1, 2, 3 or 4;
E is -O-, -CO-, -CH[OR(63)]-, -SOₐₐ- or -NR(63)-;
w is zero or 1;
aa is zero or 2
R(63) is hydrogen or methyl,
or
R(2) is -CF₂R(64), -CF[R(65)][R(66)], -CF(CF₃)[R(65)], -C(CF₃)=CR(65)R(66);
R(64) is alkyl having 1, 2, 3 or 4 carbon atoms or cycloalkyl having 3, 4, 5, 6 or 7 carbon atoms;
R(65) and R(66)
independently of one another are hydrogen or alkyl having 1, 2, 3 or 4 carbon atoms;
or
R(1), R(2) and R(3)
independently of one another are -OR(67) or -NR(67)R(68);
R(67) and R(68)
independently of one another are hydrogen or alkyl having 1, 2 or 3 carbon atoms;
or
R(67) and R(68)
together are 4, 5 or 6 methylene groups, of which one CH₂ group can be replaced by oxygen, -S-, SO₂, -NH- or -NCH₃;
R(4) and R(5)
independently of one another are hydrogen, alkyl having 1, 2 or 3 carbon atoms, F, Cl, -OR(69), -NR(70)R(71) or -CF₃;
R(69), R(70) and R(71)
independently of one another are hydrogen or methyl;
R(6) and R(7)
independently of one another are hydrogen or methyl;
X is oxygen or NR(72);
R(72) is hydrogen or methyl.

4. A compound of the formula I as claimed in one or more of claims 1 to 3, wherein:
R(2) is -0-[4-R(8)-phenyl],
R(8) is SOₐ[NR(98)]_{b}NR(99)R(10);
a is 1 or 2;
b is 0 or 1;
a + b = 2;
R(98) is hydrogen or alkyl having 1, 2, 3 or 4 carbon atoms;
R(99) and R(10)
independently of one another are hydrogen, alkyl having 1 or 2 carbon atoms or -(C₂-C₃)-alkylene-NR(11)R(12);
R(11) and R(12)
independently of one another are hydrogen, methyl or ethyl;
or
R(99) and R(10)
together are 5 or 6 methylene groups, of which one CH₂ group can be replaced by -NH- or -N-CH₃;
or
R(8) is SOₐ[NR(98)]_{b}NR(95)-C[=N-R(94)]-NR(93)R(92);
R(95) is hydrogen;
R(92), R(93) and R(94)
independently of one another are hydrogen or alkyl having 1, 2, 3 or 4 carbon atoms;
or
R(1), R(2) and R(3)
independently of one another are hydrogen or alkyl having 1, 2, 3 or 4 carbon atoms;
or
R(2) is -0-4-[CH₂-CH (NR(21)R(22))-(C=O)R(20)]-phenyl,
R(21) and R(22)
independently of one another are hydrogen, methyl, -(C₁-C₅)-alkanoyl,-(C₁-C₅)-alkoxycarbonyl, benzyl or benzyloxycarbonyl;
R(20) is -OR(23) or -NR(23)R(24);
R(23) and R(24)
independently of one another are hydrogen or -(C₁-C₄)-alkyl;
or
R(2) is imidazolyl,
which is linked via C or N;
or
R(2) is -SR(25) or -OR(25);
R(25) is pyridyl, quinolinyl or isoquinolinyl, which is unsubstituted or substituted by a substituent from the group consisting of F, Cl, CF₃, CH₃ and methoxy;
or
R(2) is -SR(28) or -OR(28);
R(28) is pyridyl N-oxide, quinolinyl N-oxide or isoquinolinyl N-oxide, which is unsubstituted or substituted by a substituent selected from the group consisting of F, Cl, CF₃, CH₃ and methoxy;
or
R(1) is hydrogen, F, Cl, CF₃, R(31)SO₂- or R(45)R(46)N-SO₂;
R(31) is methyl or -CF₃;
R(45) and R(46)
independently of one another are hydrogen or methyl;
or
R(2) is R(51)-A-G-O-;
R(51) is -NR(52)R(53);
R(52) and R(53)
independently of one another are hydrogen or alkyl having 1 or 2 carbon atoms;
or
R(52) and R(53) are
C_{α}H_{2α};
α is 5 or 6;
where a carbon atom of the group C_{α}H_{2α} can be replaced by NR(56);
R(56) is hydrogen or methyl;
or
R(51) is imidazolyl, pyridyl, quinolinyl or isoquinolinyl;
A is CₑH₂ₑ;
e is zero, 1, 2 or 3;
where in the group CₑH₂ₑ a carbon atom can be replaced by one of the groups -O-, -CO- or -SOᵣ-;
r is zero or 2;
G is a phenylene radical 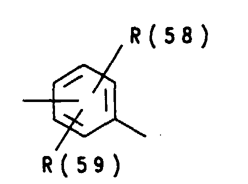 R(58) and R(59)
independently of one another are hydrogen, methyl, F, Cl, CF₃ or -SO₂-R(60);
R(60) is methyl or NR(61)R(62);
R(61) and R(62)
independently of one another are hydrogen or methyl;
or
R(2) is -CF₂R(64), -CF[R(65)][R(66)], -CF(CF₃)[R(65)], -C(CF₃)=CR(65)R(66);
R(64) is alkyl having 1, 2, 3 or 4 carbon atoms or cycloalkyl having 5 or 6 carbon atoms;
R(65) and R(66)
independently of one another are hydrogen or alkyl having 1, 2, 3 or 4 carbon atoms;
or
R(1), R(2) and R(3)
independently of one another are -OR(67) or -NR(67)R(68);
R(67) and R(68)
independently of one another are hydrogen, methyl or ethyl;
or
R(67) and R(68)
together are 4 or 5 methylene groups, of which one CH₂ group can be replaced by oxygen, -NH- or -NCH₃;
R(4) and R(5)
independently of one another are hydrogen, alkyl having 1, 2 or 3 carbon atoms, F, Cl, or -CF₃;
R(6) and R(7)
independently of one another are hydrogen or methyl;
X is oxygen or NR(72)
R(72) is hydrogen or methyl.

5. A compound of the formula I as claimed in claim 1, selected from the group consisting of 4-(3-pyridyloxy)-3-trifluoromethylbenzyloxycarbonylguanidine dihydrochloride, 4- (6-quinaldinyloxy)-3-methylsulfonylbenzyloxycarbonylguanidine dihydrochloride, 41 (6-quinaldinyloxy)-3-trifluoromethylbenzyloxycarbonylguanidine dihydrochloride, 4-isopropyl-3-methylsulfonylbenzyloxycarbonylguanidine hydrochloride, 3-isopropylbenzyloxycarbonylguanidine hydrochloride, 2-chloro-5-trifluoromethylbenzyloxycarbonylguanidine hydrochloride, 4-(6-quinolinyloxy)-3-methylsulfonylbenzyloxycarbonylguanidine dihydrochloride, 3-bromo-5-fluorobenzylaminocarbonylguanidine hydrochloride, 3,5-dimethylbenzylaminocarbonylguanidine hydrochloride, 2-fluorobenzylaminocarbonylguanidine hydrochloride, 3-fluorobenzylaminocarbonylguanidine hydrochloride, 2, 6-difluorobenzylaminocarbonylguanidine hydrochloride, 2,5-difluorobenzylaminocarbonylguanidine hydrochloride, 3-fluoro-5-trifluoromethylbenzylaminocarbonylguanidine hydrochloride, 4-dimethylaminobenzylaminocarbonylguanidine hydrochloride, 3,5-difluorobenzylaminocarbonylguanidine hydrochloride, 3-methylbenzylaminocarbonylguanidine hydrochloride, N-3,5-difluorobenzyl-N-methylaminocarbonylguanidine hydrochloride, 3,5-difluorobenzyloxycarbonylguanidine hydrochloride, 2,5-difluorobenzyloxycarbonylguanidine hydrochloride, 2,3,6-trifluorobenzyloxycarbonylguanidine hydrochloride, N-(3,4-dichlorobenzyl)-N-methylaminocarbonylguanidine hydrochloride, N-(2-chloro-5-trifluoromethylbenzyl)-N-methylaminocarbonylguanidine hydrochloride, N-methyl-N-(3-methylsulfonyl-4-isopropylbenzyl)aminocarbonylguanidine methanesulfonate, N-(4-fluoro-3-trifluoromethylbenzyl)-N-methylaminocarbonylguanidine hydrochloride, 4-isopropyl-3-methylsulfonylbenzyloxycarbonylguanidine hydrochloride, 2-chloro-5-trifluoromethylbenzyloxycarbonylguanidine hydrochloride, 3-isopropylbenzyloxycarbonylguanidine hydrochloride, 4-(6-quinolyloxy)-3-methylsulfonylbenzyloxycarbonylguanidine hydrochloride and 3-trifluoromethyl-4-methoxybenzyloxycarbonylguanidine hydrochloride.

6. A process for preparing a compound of the formula I as claimed in claim 1, which comprises reacting a compound of the formula IV 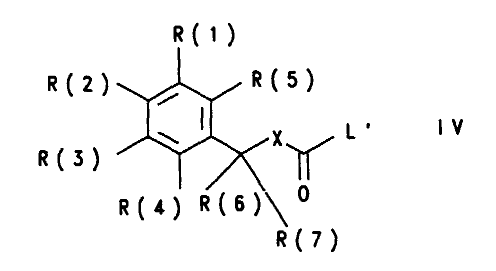 in which R(1) to R(7) and X have the meanings given in claim 1, and in which L' is chlorine, ethoxy, isobutoxy, benzotriazol-1-oxy or 1-imidazolyl,
with guanidine.

7. The use of a compound I as claimed in claim 1 for the production of a medicament for treatment of arrhythmias.

8. The use of a compound I as claimed in claim 1 for the production of a medicament for the treatment or prophylaxis of cardiac infarct.

9. The use of a compound I as claimed in claim 1 for the production of a medicament for the treatment or prophylaxis of angina pectoris.

10. The use of a compound I as claimed in claim 1 for the production of a medicament for the treatment or prophylaxis of ischemic conditions of the heart.

11. The use of a compound I as claimed in claim 1 for the production of a medicament for the treatment or prophylaxis of ischemic conditions of the peripheral and central nervous system and of stroke.

12. The use of a compound I as claimed in claim 1 for the production of a medicament for the treatment or prophylaxis of ischemic conditions of peripheral organs and limbs.

13. The use of a compound I as claimed in claim 1 for the production of a medicament for the treatment of states of shock.

14. The use of a compound I as claimed in claim 1 for the production of a medicament for use in surgical operations and organ transplants.

15. The use of a compound I as claimed in claim 1 for the production of a medicament for the preservation and storage of transplants for surgical measures.

16. The use of a compound I as claimed in claim 1 for the production of a medicament for the treatment of diseases in which cell proliferation is a primary or secondary cause.

17. Medicines comprising an effective amount of a compound I as claimed in one or more of claims 1 to 3.

## Revendications

1. Composé de formule 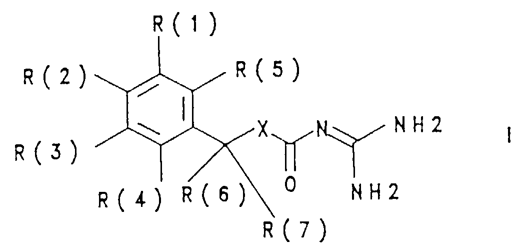 dans laquelle
R(1), R(2) et R(3) représentent, indépendamment les uns des autres, un groupe -Y1 [4-R(8)-phényle], -Y-[3-R(8)-phényle] ou -Y-[2-R(8)-phényle],
le fragment phényle étant dans chaque cas non substitué ou portant 1-2 substituants choisis dans l'ensemble constitué par F, Cl, -CF₃, les groupes méthyle, hydroxy, méthoxy et -NR(96)R(97);
R(96) et R(97) représentant, indépendamment l'un de l'autre, un atome d'hydrogène ou le groupe -CH₃;
Y représente une liaison, CH₂, un atome d'oxygène, -S- ou -NR(9),
R(9) représentant un atome d'hydrogène ou un groupe alkyle ayant 1, 2, 3 ou 4 atomes de carbone;
R(8) représente un groupe SOₐ[NR(98)]_{b}NR(99)R(10),
a étant égal à 1 ou 2,
b étant égal à 0 ou 1,
a + b = 2;
R(98), R(99) et R(10) représentant, indépendamment les uns des autres, un atome d'hydrogène ou un groupe alkyle en C₁-C₈, benzyle, alkylène(C₂-C₈)-NR(11)R(12), alkylène(C₂-C₈)-NR(13)-alkylène(C₂-C₈)-NR(37)R(38) ou alkylène(C₀-C₈)-CR(39)R(40)-CR(41)R(42)-alkylène(C₀-C₈)-NR(43)R(44);
R(11), R(12), R(13), R(37), R(38), R(43) et R(44) représentant, indépendamment les uns des autres, un atome d'hydrogène ou un groupe alkyle en C₁-C₈ ou benzyle;
R(39), R(40), R(41) et R(42) représentant, indépendamment les uns des autres, un atome d'hydrogène ou un groupe alkyle en C₁-C₈ ou alkylène(C₀-C₃)-phényle,
le fragment phényle n'étant pas substitué ou portant de 1 à 3 substituants choisis dans l'ensemble constitué par F, Cl, -CF₃, les groupes méthyle et méthoxy; ou
R(99) et R(10) représentant ensemble 4-6 groupes méthylène, dont un groupe CH₂ peut être remplacé par un atome d'oxygène, -S-, -NH-, -N-CH₃ ou le groupe -N-benzyle;
ou
R(8) représente un groupe
SOₐ[NR(98)]_{b}NR(95)C[=N-R(94)]-NR(93)R(92),
R(92), R(93), R(94) et R(95) représentant, indépendamment les uns des autres, un atome d'hydrogène ou un groupe alkyle ayant 1, 2, 3 ou 4 atomes de carbone;
ou
R(1), R(2) et R(3) représentent, indépendamment les uns des autres, un atome d'hydrogène ou un groupe alkyle en C₁-C₈, alcényle en C₂-C₈ ou -(CH₂)ₘR(14);
m étant 0, 1 ou 2;
R(14) représentant un groupe cycloalkyle en C₃-C₈ ou phényle,
qui n'est pas substitué ou porte de 1 à 3 substituants choisis dans l'ensemble constitué par F et Cl, -CF₃, les groupes méthyle, méthoxy et -NR(15)R(16);
R(15) et R(16) représentant un atome d'hydrogène ou -CH₃;
ou
R(1), R(2) et R(3) représentent, indépendamment les uns des autres, un groupe
-Q-4-[(CH₂)ₖ-CHR(17)-(C=O)R(20)]phényle,
-Q-3-[(CH₂)ₖ-CHR(17)-(C=O)R(20)]phényle ou
-Q-2-[(CH₂)ₖ-CHR(17)-(C=O)R(20)]phényle,
le fragment phényle dans chaque cas étant non substitué ou portant 1 ou 2 substituants choisis dans l'ensemble constitué par F, Cl, -CF₃, les groupes méthyle, hydroxy, méthoxy et -NR(35)R(36),
R(35) et R(36) représentant, indépendamment l'un de l'autre, un atome d'hydrogène ou -CH₃;
Q représente une liaison, un atome d'oxygène, -S- ou -NR(18),
R(18) représentant un atome d'hydrogène ou un groupe alkyle en C₁-C₄;
R(17) représente un groupe -OR(21) ou -NR(21)R(22),
R(21) et R(22) représentant, indépendamment l'un de l'autre,
un atome d'hydrogène ou un groupe alkyle en C₁-C₈, alcanoyle en C₁-C₈, alcoxy-(C₁-C₈)carbonyle, benzyle, benzyloxycarbonyle, ou
R(21) représentant le groupe trityle;
R(20) représente un groupe -OR(23) ou -NR(23)R(24),
R(23) et R(24) représentant, indépendamment l'un de l'autre,
un atome d'hydrogène ou un groupe alkyle en C₁-C₈ ou benzyle;
k est 0, 1, 2, 3 ou 4;
ou
R(1), R(2) et R(3) représentent, indépendamment les uns des autres, un groupe hétéroaryle en C₁-C₉,
qui est relié par C ou N et qui n'est pas substitué ou porte de 1 à 3 substituants choisis dans l'ensemble constitué par F, Cl, CF₃, CH₃, les groupes méthoxy, hydroxy, amino, méthylamino et diméthylamino;
ou
R(1), R(2) et R(3) représentent un groupe -SR(25), -OR(25), -NR(25)R(26), -CR(25)R(26)R(27),
R(25) représentant un groupe -C_{f}H_{2f}-hétéroaryle-(C₁-C₉),
qui n'est pas substitué ou porte de 1 à 3 substituants choisis dans l'ensemble constitué par F, Cl, CF₃, CH₃, les groupes méthoxy, hydroxy, amino, méthylamino et diméthylamino;
f étant 0, 1 ou 2;
R(26) et R(27) étant définis, indépendamment l'un de l'autre, comme R(25), ou représentant un atome d'hydrogène ou un groupe alkyle en C₁-C₄;
ou
R(1), R(2) et R(3) représentent, indépendamment les uns des autres, un groupe hétéroaryl(C₁-C₉)-N-oxyde,
qui est relié par C ou N et qui n'est pas substitué ou porte de 1 à 3 substituants choisis dans l'ensemble constitué par F, Cl, CF₃, CH₃, les groupes méthoxy, hydroxy, amino, méthylamino et diméthylamino;
ou
R(1), R(2) et R(3) représentent, indépendamment les uns des autres, un groupe -SR(28), -OR(28), -NR(28)R(29) ou -CR(28)R(29)R(30),
R(28) représentant un groupe -C_{g}H_{2g}-hétéroaryle-(C₁-C₉)-N-oxyde,
qui n'est pas substitué ou porte de 1 à 3 substituants choisis dans l'ensemble constitué par F, Cl, CF₃, CH₃, les groupes méthoxy, hydroxy, amino, méthylamino et diméthylamino;
g étant 0, 1 ou 2;
R(29) et R(30) étant, indépendamment l'un de l'autre, définis comme R(28), ou représentant un atome d'hydrogène ou un groupe alkyle en C₁-C₄;
ou
R(1), R(2) et R(3) représentent, indépendamment les uns des autres, un atome d'hydrogène, F; Cl, Br, I, -CN, T-(CH₂)ₕ-(CᵢF₂ᵢ₊₁), R(31)SO₁-, R(32)R33)N-CO-, R(34)-CO- ou R(45)R(46)N-SO₂, le groupe perfluoroalkyle étant à chaîne droite ou ramifié;
T représentant une liaison, un atome d'oxygène, -S- ou -NR(47) ;
l étant 0, 1 ou 2;
h étant 0, 1 ou 2;
i étant 1, 2, 3, 4, 5 ou 6;
R(31), R(32), R(34) et R(45) représentant, indépendamment les uns des autres, un groupe alkyle en C₁-C₈, alcényle en C₃-C₆, (CH₂)ₙR(48) ou -CF₃;
n étant 0, 1, 2, 3 ou 4;
R(47) représentant un atome d'hydrogène ou un groupe alkyle ayant 1, 2 ou 3 atomes de carbone;
R(48) représentant un groupe cycloalkyle en C₃-C₇ ou phényle,
qui n'est pas substitué ou porte de 1 à 3 substituants choisis dans l'ensemble constitué par F, Cl, -CF₃, les groupes méthyle, méthoxy et -NR(49)R(50);
R(49) et R(50) représentant un atome d'hydrogène ou un groupe alkyle ayant 1, 2, 3 ou 4 atomes de carbone;
ou
R(32), R(34) et R(45) étant des atomes d'hydrogène;
R(33) et R(46) représentant, indépendamment l'un de l'autre, un atome d'hydrogène ou un groupe alkyle ayant 1, 2, 3 ou 4 atomes de carbone;
ou
R(32) et R(33) ainsi que R(45) et R(46) représentant ensemble 5 ou 6 groupes méthylène, dont un groupe CH₂ peut être remplacé par un atome d'oxygène, -S-, -NH-, -NCH₃ ou le groupe -N-benzyle;
ou
R(1), R(2) et R(3) représentent, indépendamment les uns des autres, un groupe R(51)-A-G-D;
R(51) représentant un radical protonable basique, c'est-à-dire un groupe amino -NR(52)R(53), un groupe amidino R(52)R(53)N-C[=N-R(54)]- ou un groupe guanidino
R(52)R(53)N-C[=N-R(54)]-NR(55)-;
R(52), R(53), R(54) et R(55) représentant, indépendamment les uns des autres, un atome d'hydrogène ou un groupe alkyle ayant 1, 2, 3 ou 4 atomes de carbone;
ou
R(52) et R(53) représentant
un groupe C_{α} H_{2α},
α étant 4, 5, 6 ou 7;
dans le cas où α = 5, 6 ou 7, un atome de carbone du groupe C_{α}H_{2α} pouvant être remplacé par un hétéroatome O ou par un groupe SO_{d} ou NR(56);
ou
R(53) et R(54) ou R(54) et R(55) ou R(52) et
R(55) représentant
un groupe C_{γ}H_{2γ},
γ étant 2, 3, 4 ou 5;
dans le cas où γ = 3, 4 ou 5, un atome de carbone du groupe C_{γ} H_{2γ} pouvant être remplacé par un hétéroatome O ou par SO_{d} ou NR(56) ;
d étant 0, 1 ou 2;
R(56) représentant un atome d'hydrogène ou le groupe méthyle;
ou
R(51) représentant un système cyclique hétéroaromatique basique ayant de 1 à 9 atomes de carbone;
A représentant un groupe CₑH₂ₑ;
e étant 0, 1, 2, 3, 4, 5, 6, 7, 8, 9 ou 10,
dans le groupe CₑH₂ₑ, un atome de carbone pouvant être remplacé par l'un des groupes -O-, -CO- ou SOᵣ-;
r étant 0, 1 ou 2;
G représentant un radical phénylène 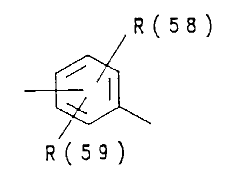 R(58) et R(59) représentant, indépendamment l'un de l'autre, un atome d'hydrogène ou F, Cl, Br, I, ou le groupe méthoxy, CF₃ ou -SOₛ-R(60);
R(60) représentant un groupe méthyle ou NR(61)R(62);
R(61) et R(62) représentant, indépendamment l'un de l'autre, un atome d'hydrogène ou un groupe alkyle ayant 1, 2, 3 ou 4 atomes de carbone;
D représentant CᵥH₂ᵥ-E_{w}-;
v étant 0, 1, 2, 3 ou 4;
E représentant -O-, -CO-, -CH[OR(63)]-, -SOₐₐ- ou -NR(63)-;
w étant 0 ou 1;
aa étant 0, 1 ou 2,
R(63) représentant un atome d'hydrogène ou le groupe méthyle;
ou
R(1), R(2) et R(3) représentent, indépendamment les uns des autres, un groupe -CF₂R(64), -CF[R(65)][R(66)], -CF[(CF₂)_{q}-CF₃)][R(65)], C[(CF₂)ₚ-CF₃]=CR(65)R(66);
R(64) représentant un groupe alkyle ayant 1, 2, 3 ou 4 atomes de carbone, ou cycloalkyle ayant 3, 4, 5, 6 ou 7 atomes de carbone;
R(65) et R(66) représentant, indépendamment l'un de l'autre, un atome d'hydrogène ou un groupe alkyle ayant 1, 2, 3 ou 4 atomes de carbone;
q étant 0, 1 ou 2;
p étant 0, 1 ou 2;
ou
R(1), R(2) et R(3) représentent, indépendamment les uns des autres, un groupe -OR(67) ou -NR(67)R(68);
R(67) et R(68) représentant, indépendamment l'un de l'autre, un atome d'hydrogène ou un groupe alkyle ayant 1, 2, 3, 4, 5 ou 6 atomes de carbone; ou
R(67) et R(68) représentant ensemble 4, 5, 6 ou 7 groupes méthyle, dont un groupe CH₂ peut être remplacé par un atome d'oxygène ou par -S- ou par un groupe -SO₂-, -NH-, -NCH₃ ou -N-benzyle;
R(4) et R(5) représentent, indépendamment l'un de l'autre, un atome d'hydrogène ou un groupe alkyle ayant 1, 2, 3 ou 4 atomes de carbone, F, Cl, -OR(69),
-NR(70)R(71) ou C_{z}F_{2z+1};
R(69), R(70) et R(71) représentant, indépendamment les uns des autres, un atome d'hydrogène ou un groupe alkyle ayant 1, 2 ou 3 atomes de carbone;
z étant 1, 2, 3 ou 4;
R(6) et R(7) représentent, indépendamment l'un de l'autre, un atome d'hydrogène ou un groupe alkyle ayant 1, 2, 3 ou 4 atomes de carbone;
X représente un atome d'oxygène ou NR(72);
R(72) représentant un atome d'hydrogène ou un groupe alkyle ayant 1, 2, 3 ou 4 atomes de carbone;
et sels pharmaceutiquement acceptables d'un tel composé, mais à l'exception des composés dans lesquels les radicaux R(1) à R(7) ainsi que R(72) sont des atomes d'hydrogène.

2. Composé de formule I selon la revendication 1, caractérisé en ce que, dans ce composé,
R(1), R(2) et R(3) représentent, indépendamment les uns des autres, un groupe -Y-[4-R(8)-phényle],
-Y-[3-R(8)-phényle] ou -Y- [2-R(8)-phényle],
le fragment phényle dans chaque cas étant non substitué ou portant un substituant choisi dans l'ensemble constitué par F, Cl, -CF₃, les groupes méthyle, méthoxy et -NR(96)R(97);
R(96) et R(97) représentant, indépendamment l'un de l'autre, un atome d'hydrogène ou -CH₃;
Y représentant une liaison, un atome d'oxygène, -S- ou -NR(9),
R(9) représentant un atome d'hydrogène ou le groupe méthyle;
R(8) représentant un groupe SOₐ[NR(98)]_{b}NR(99)R(10),
a étant égal à 1 ou 2,
b étant égal à 0 ou 1,
a + b = 2;
R(98) représentant un atome d'hydrogène ou un groupe alkyle ayant 1, 2, 3, 4 ou 5 atomes de carbone, ou le groupe benzyle;
R(99) et R(10) représentant, indépendamment l'un de l'autre, un atome d'hydrogène ou un groupe alkyle ayant 1 ou 2 atomes de carbone, le groupe benzyle, un groupe alkylène(C₂-C₃)-NR(11)R(12), alkylène(C₂-C₃)-NR(13)-alkylène(C₂-C₃)-NR(37)R(38) ou alkylène(C₀-C₂)-CR(39)R(40)-CR(41)R(42)-alkylène(C₀-C₂)-NR(43)R(44);
R(11), R(12), R(13), R(37), R(38), R(43) et R(44) représentant, indépendamment les uns des autres, un atome d'hydrogène ou le groupe méthyle ou éthyle;
R(39), R(40), R(41) et R(42) représentant, indépendamment les uns des autres, un atome d'hydrogène ou un groupe alkyle ayant 1, 2, 3 ou 4 atomes de carbone, ou le groupe benzyle; le fragment phényle n'étant pas substitué ou portant un substituant choisi dans l'ensemble constitué par F, Cl, -CF₃, les groupes méthyle et méthoxy;
ou
R(99) et R(10) représentant ensemble 4, 5 ou 6 groupes méthylène, dont un groupe CH₂ peut être remplacé par un atome d'oxygène, -S-, -NH- ou -N-CH₃;
ou
R(8) représentant un groupe SOₐ[NR(98)]_{b}NR(95)-C[=N-R(94)]-NR(93)R(92), R(95) représentant un atome d'hydrogène,
R(92), R(93) et R(94) représentant, indépendamment les uns des autres, un atome d'hydrogène ou un groupe alkyle ayant 1, 2, 3 ou 4 atomes de carbone;
ou
R(1), R(2) et R(3) représentent, indépendamment les uns des autres, un atome d'hydrogène ou un groupe alkyle ayant 1, 2, 3 ou 4 atomes de carbone;
ou
R(1), R(2) et R(3) représentent, indépendamment les uns des autres, un groupe
-Q-4-[(CH₂)ₖ-CH(NR(21)R(22))(C=O)R(20)]phényle,
-Q-3-[(CH₂)ₖ-CH(NR(21)R(22))-(C=O)R(20)]phényle ou
-Q-2-[(CH₂)ₖ-CH(NR(21)R(22))-(C=O)R(20)]phényle,
le radical phényle dans chaque cas n'étant pas substitué ou portant 1 ou 2 substituants choisis dans l'ensemble constitué par F, Cl, -CF₃, les groupes méthyle, hydroxy, méthoxy et -NR(35)R(36),
R(35) et R(36) représentant, indépendamment l'un de l'autre, un atome d'hydrogène ou -CH₃;
Q représentant une liaison ou un atome d'oxygène, -S- ou -NR(18),
R(18) représentant un atome d'hydrogène ou un groupe alkyle en C₁-C₄;
R(21) et R(22) représentant, indépendamment l'un de l'autre, un atome d'hydrogène ou un groupe alkyle en C₁-C₅, alcanoyle en C₁-C₅, alcoxy-(C₁-C₅)carbonyle, benzyle, benzyloxycarbonyle;
ou
R(21) représentant le groupe trityle;
R(20) représentant un groupe -OR(23) ou -NR(23)R(24),
R(23) et R(24) représentant, indépendamment l'un de l'autre, un atome d'hydrogène ou un groupe alkyle en C₁-C₄ ou benzyle;
k étant 0, 1, ou 2;
ou
R(1), R(2) et R(3) représentent, indépendamment les uns des autres, un groupe hétéroaryle en C₁-C₉, qui est relié par C ou N et qui n'est pas substitué ou porte un substituant choisi dans l'ensemble constitué par F, Cl, CF₃, CH₃, les groupes méthoxy et diméthylamino;
ou
R(1), R(2) et R(3) représentent un groupe -SR(25), -OR(25), -NR(25)R(26) , -CR(25)R(26)R(27) ;
R(25) représentant un groupe -C_{f}H_{2f}-hétéroaryle-(C₁-C₉),
qui n'est pas substitué ou porte un substituant choisi dans l'ensemble constitué par F, Cl, CF₃, CH₃, les groupes méthoxy et diméthylamino, f étant 0, 1 ou 2;
R(26) et R(27) étant, indépendamment l'un de l'autre, définis comme R(25) ou représentant un atome d'hydrogène ou le groupe méthyle;
ou
R(1), R(2) et R(3) représentent, indépendamment les uns des autres, un groupe hétéroaryl(C₁-C₉)-N-oxyde, qui est relié par C ou N et qui n'est pas substitué ou porte 1-2 substituants choisis dans l'ensemble constitué par F, Cl, CF₃, CH₃, les groupes méthoxy, hydroxy, amino, méthylamino et diméthylamino;
ou
R(1), R(2) et R(3) représentent, indépendamment les uns des autres, un groupe -SR(28), -OR(28), -NR(28)R(29) ou -CR(28)R(29)R(30) ;
R(28) représentant un groupe C_{g}H_{2g}-hétéroaryl-(C₁-C₉)-N-oxyde,
qui n'est pas substitué ou porte 1-2 substituants choisis dans l'ensemble constitué par F, Cl, CF₃, CH₃, les groupes méthoxy, hydroxy, amino, méthylamino et diméthylamino,
g étant 0 ou 1;
R(29) et R(30) étant, indépendamment l'un de l'autre, définis comme R(28) ou représentant un atome d'hydrogène ou le groupe méthyle;
ou
R(1) et R(3) représentent, indépendamment l'un de l'autre, un atome d'hydrogène, F, Cl, CF₃, R(31)SO₂-,
R(32)R(33) N-CO-, R(34)-CO- ou R(45)R(46)N-SO₂;
R(31) et R(34) représentant, indépendamment l'un de l'autre, le groupe méthyle ou -CF₃;
R(32), R(33), R(45) et R(46) représentant, indépendamment les uns des autres, un atome d'hydrogène ou le groupe méthyle;
ou
R(1), R(2) et R(3) représentent, indépendamment les uns des autres, un groupe R(51)-A-G-D-;
R(51) représentant un groupe -NR(52)R(53), un groupe amidino R(52)R(53)N-C[=N-R(54)]- ou un groupe guanidino R(52)R(53)N-C[=N-R(54)]-NR(55)-;
R(52), R(53), R(54) et R(55) représentant, indépendamment les uns des autres, un atome d'hydrogène ou un groupe alkyle ayant 1, 2, 3 ou 4 atomes de carbone; ou
R(52) et R(53) représentant un groupe C_{α}H_{2α} ,
α étant 4, 5, 6 ou 7,
dans le cas de α = 5, 6 ou 7, un atome de carbone du groupe C_{α}H_{2α} pouvant être remplacé par un hétéroatome 0 ou par un groupe SO_{d} ou NR(56); ou
R(53) et R(54) représentant un groupe C_{γ}H_{2γ} ,
γ étant 2, 3, 4 ou 5,
dans le cas de γ = 3, 4 ou 5, un atome de carbone du groupe C_{γ} H_{2γ} pouvant être remplacé par un hétéroatome O ou par un groupe SO_{d} ou NR(56),
d étant 0 ou 2,
R(56) étant un atome d'hydrogène ou le groupe méthyle;
ou
R(51) représentant le groupe imidazolyle, pyridyle, quinolinyle ou isoquinolinyle;
A représentant un groupe CₑH₂ₑ,
e étant 0, 1, 2, 3, 4 ou 5,
dans le groupe CₑH₂ₑ, un atome de carbone pouvant être remplacé par -O-, -CO- ou -SOᵣ-,
r étant 0 ou 2;
G représentant un radical phénylène 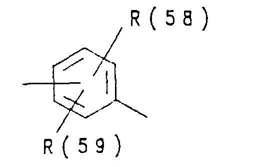 R(58) et R(59) représentant, indépendamment l'un de l'autre, un atome d'hydrogène, le groupe méthyle, F, Cl, CF₃ ou -SO₂-R(60);
R(60) représentant le groupe méthyle ou NR(61)R(62);
R(61) et R(62) représentant, indépendamment l'un de l'autre, un atome d'hydrogène ou le groupe méthyle;
D représentant un groupe -CᵥH₂ᵥ-E_{w}-;
v étant 0, 1, 2, 3 ou 4;
E représentant -O-, -CO-, -CH[OR(63)]-, -SOₐₐ- ou -NR(63)-;
w étant 0 ou 1;
aa étant 0 ou 2,
R(63) représentant un atome d'hydrogène ou le groupe méthyle;
ou
R(2) représente le groupe -CF₂R(64), -CF[R(65)] [R(66)], -CF(CF₃)[R(65)], -C(CF₃)=CR(65)R(66);
R(64) représentant un groupe alkyle ayant 1, 2, 3 ou 4 atomes de carbone, ou cycloalkyle ayant 3, 4, 5, 6 ou 7 atomes de carbone;
R(65) et R(66) représentant, indépendamment l'un de l'autre, un atome d'hydrogène ou un groupe alkyle ayant 1, 2, 3 ou 4 atomes de carbone;
ou
R(1), R(2) et R(3) représentent, indépendamment les uns des autres, un groupe -OR(67) ou -NR(67)R(68);
R(67) et R(68) représentant, indépendamment l'un de l'autre, un atome d'hydrogène ou un groupe alkyle ayant 1, 2 ou 3 atomes de carbone; ou
R(67) et R(68) représentant ensemble 4, 5 ou 6 groupes méthylène, dont un groupe CH₂ peut être remplacé par un atome d'oxygène ou de S, ou par le groupe -SO₂-, -NH- ou -NCH₃;
R(4) et R(5) représentent, indépendamment l'un de l'autre, un atome d'hydrogène ou un groupe alkyle ayant 1, 2 ou 3 atomes de carbone, F, Cl, -OR(69), -NR(70)R(71) ou -CF₃;
R(69), R(70) et R(71) représentant, indépendamment les uns des autres, un atome d'hydrogène ou le groupe méthyle;
R(6) et R(7) représentent, indépendamment l'un de l'autre, un atome d'hydrogène ou le groupe méthyle;
X représente un atome d'oxygène ou NR(72);
R(72) représentant un atome d'hydrogène ou le groupe méthyle;
et sels pharmaceutiquement acceptables d'un tel composé.

3. Composé de formule I selon une ou plusieurs des revendications 1 et 2, caractérisé en ce que, dans ce composé:
R(1), R(2) et R(3) représentent, indépendamment les uns des autres, un groupe -O-[4-R(8)-phényle],
le fragment phényle dans chaque cas étant non substitué ou portant un substituant choisi dans l'ensemble constitué par F, Cl, -CF₃, les groupes méthyle et méthoxy;
R(8) représentant un groupe -SOₐ[NR(98)]_{b}NR(99)R(10);
a étant 1 ou 2;
b étant 0 ou 1;
a + b = 2;
R(98) représentant un atome d'hydrogène ou un groupe alkyle ayant 1, 2, 3 ou 4 atomes de carbone;
R(99) et R(10) représentant, indépendamment l'un de l'autre, un atome d'hydrogène ou un groupe alkyle ayant 1 ou 2 atomes de carbone, benzyle, alkylène(C₂-C₃)-NR(11)R(12), alkylène(C₂-C₃)-NR(13)-alkylène(C₂-C₃)-NR(37)R(38) ou alkylène-C₀-C₂)-CR(39)R(40)-CR(41)R(42)-alkylène-(C₀-C₂)-NR(43)R(44);
R(11), R(12), R(13), R(37), R(38), R(43) et R(44) représentant, indépendamment les uns des autres, un atome d'hydrogène ou le groupe méthyle ou éthyle;
R(39), R(40), R(41) et R(42) représentant, indépendamment les uns des autres, un atome d'hydrogène ou un groupe alkyle ayant 1, 2, 3 ou 4 atomes de carbone ou le groupe benzyle;
le fragment phényle n'étant pas substitué ou portant un substituant choisi dans l'ensemble constitué par F, Cl, -CF₃, les groupes méthyle et méthoxy;
ou
R(99) et R(10) représentant ensemble 4, 5 ou 6 groupes méthylène, dont un groupe CH₂ peut être remplacé par -NH- ou -N-CH₃;
ou
R(8) représentant un groupe
SOₐ[NR(98)]_{b}NR(95)-C[=N-R(94)]-NR(93)R(92);
R(95) représentant un atome d'hydrogène;
R(92), R(93) et R(94) représentant, indépendamment les uns des autres, un atome d'hydrogène ou un groupe alkyle ayant 1, 2, 3 ou 4 atomes de carbone;
ou
R(1), R(2) et R(3) représentent, indépendamment les uns des autres, un atome d'hydrogène ou un groupe alkyle ayant 1, 2, 3 ou 4 atomes de carbone;
ou
R(1), R(2) et R(3) représentent, indépendamment les uns des autres, un groupe
-Q-4-[(CH₂)ₖ-CH(NR(21)R(22))-(C=O)R(20)]phényle, le fragment phényle dans chaque cas étant non substitué ou portant un substituant choisi dans l'ensemble constitué par F, Cl, -CF₃, les groupes méthyle, hydroxy et méthoxy;
Q représentant une liaison ou l'atome d'oxygène;
R(21) et R(22) représentant, indépendamment l'un de l'autre, un atome d'hydrogène ou un groupe méthyle, alcanoyle en C₁-C₅, alcoxy(C₁-C₅)-carbonyle, benzyle ou benzyloxycarbonyle;
R(20) représentant un groupe -OR(23) ou -NR(23)R(24);
R(23) et R(24) représentant, indépendamment l'un de l'autre, un atome d'hydrogène ou un groupe alkyle en C₁-C₄ ou benzyle;
k étant 0, 1 ou 2;
ou
R(1), R(2) et R(3) représentent, indépendamment les uns des autres, un groupe imidazolyle qui est relié par C ou N et qui est non substitué ou porte un substituant choisi dans l'ensemble constitué par F, Cl, CF₃, les groupes CH₃ et méthoxy;
ou
R(1), R(2) et R(3) représentent un groupe -SR(25), -OR(25), -NR(25)R(26), -CR(25)R(26)R(27);
R(25) représentant un groupe hétéroaryle en C₁-C₉ qui est non substitué ou porte un substituant choisi dans l'ensemble constitué par F, Cl, CF₃, CH₃ et le groupe méthoxy;
R(26) et R(27) représentant, indépendamment l'un de l'autre, un atome d'hydrogène ou le groupe méthyle;
ou
R(1), R(2) et R(3) représentent, indépendamment les uns des autres, un groupe -SR(28), -OR(28), -NR(28)R(29) ou -CR(28)R(29)R(30) ;
R(28) représentant un groupe hétéroaryl(C₁-C₉)-N-oxyde,
qui est non substitué ou porte un substituant choisi dans l'ensemble constitué par F, Cl, CF₃, CH₃ et le groupe méthoxy;
R(29) et R(30) représentant, indépendamment l'un de l'autre, un atome d'hydrogène ou le groupe méthyle;
ou
R(1) et R(3) représentent, indépendamment l'un de l'autre, un atome d'hydrogène, F, Cl, CF₃, R(31)SO₂-,
R(32)R(33) N-CO-, R(34)-CO- ou R(45)R(46)N-SO₂;
R(31) et R(34) représentant, indépendamment l'un de l'autre, le groupe méthyle ou -CF₃;
R(32), R(33), R(45) et R(46) représentant, indépendamment les uns des autres, un atome d'hydrogène ou le groupe méthyle;
ou
R(2) représente un groupe R(51)-A-G-D-;
R(51) représentant un groupe -NR(52)R(53), un groupe amidino R(52)R(53)N-C[=N-R(54)]- ou un groupe guanidino R(52)R(53)N-C[=N-R(54)]-NR(55)-;
R(52), R(53), R(54) et R(55) représentant, indépendamment les uns des autres, un atome d'hydrogène ou un groupe alkyle ayant 1, 2, 3 ou 4 atomes de carbone; ou
R(52) et R(53) représentant un groupe C_{α}H_{2α} ,
α étant 4, 5, 6 ou 7,
dans le cas de α = 5, 6 ou 7, un atome de carbone du groupe C_{α}H_{2α} pouvant être remplacé par un hétéroatome 0 ou par un groupe SO_{d} ou NR(56); ou
R(53) et R(54) représentant un groupe C_{γ}H_{2γ} ,
γ étant 2, 3, 4 ou 5,
dans le cas de γ = 3, 4 ou 5, un atome de carbone du groupe C_{γ}H_{2γ} pouvant être remplacé par un hétéroatome O ou par un groupe SO_{d} ou NR(56),
d étant 0 ou 2,
R(56) représentant un atome d'hydrogène ou le groupe méthyle;
ou
R(51) représentant un groupe imidazolyle, pyridyle, quinolinyle ou isoquinolinyle;
A étant CₑH₂ₑ,
e étant 0, 1, 2, 3, 4 ou 5,
dans le groupe CₑH₂ₑ, un atome de carbone pouvant être remplacé par -O-, -CO- ou -SOᵣ-,
r étant 0 ou 2;
G représentant un radical phénylène 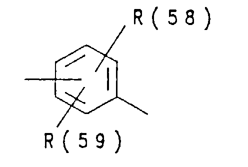 R(58) et R(59) représentant, indépendamment l'un de l'autre, un atome d'hydrogène ou le groupe méthyle, F, Cl, CF₃ ou -SO₂-R(60);
R(60) représentant le groupe méthyle ou NR(61)R(62);
R(61) et R(62) représentant, indépendamment l'un de l'autre, un atome d'hydrogène ou le groupe méthyle;
D représentant un groupe -CᵥH₂ᵥ-E_{w}-;
v étant 0, 1, 2, 3 ou 4;
E étant -O-, -CO-, -CH[OR(63)]-, -SOₐₐ- ou -NR(63)-;
w étant 0 ou 1;
aa étant 0 ou 2,
R(63) représentant un atome d'hydrogène ou le groupe méthyle;
ou
R(2) représente un groupe -CF₂R(64), -CF[R(65)][R(66)], -CF(CF₃)[R(65)], -C(CF₃)=CR(65)R(66);
R(64) représentant un groupe alkyle ayant 1, 2, 3 ou 4 atomes de carbone, ou cycloalkyle ayant 3, 4, 5, 6 ou 7 atomes de carbone;
R(65) et R(66) représentant, indépendamment l'un de l'autre, un atome d'hydrogène ou un groupe alkyle ayant 1, 2, 3 ou 4 atomes de carbone;
ou
R(1), R(2) et R(3) représentent, indépendamment les uns des autres, un groupe -OR(67) ou -NR(67)R(68);
R(67) et R(68) représentant, indépendamment l'un de l'autre, un atome d'hydrogène ou un groupe alkyle ayant 1, 2 ou 3 atomes de carbone; ou
R(67) et R(68) représentant ensemble 4, 5 ou 6 groupes méthylène, dont un groupe CH₂ peut être remplacé par un atome d'oxygène ou par -S-, -SO₂-, -NH- ou -NCH₃;
R(4) et R(5) représentent, indépendamment l'un de l'autre, un atome d'hydrogène ou un groupe alkyle ayant 1, 2 ou 3 atomes de carbone, F, Cl, -OR(69), -NR(70)R(71) ou -CF₃;
R(69), R(70) et R(71) représentant, indépendamment les uns des autres, un atome d'hydrogène ou le groupe méthyle;
R(6) et R(7) représentent, indépendamment l'un de l'autre, un atome d'hydrogène ou le groupe méthyle;
X représente un atome d'oxygène ou NR(72);
R(72) représentant un atome d'hydrogène ou le groupe méthyle.

4. Composé de formule I selon une ou plusieurs des revendications 1 à 3, caractérisé en ce que, dans ce composé:
R(2) représente un groupe -O-[4-R(8)-phényle]:
R(8) représentant un groupe SOₐ[NR(98)]_{b}NR(99)R(10);
a étant 1 ou 2;
b étant 0 ou 1;
a + b = 2;
R(98) représentant un atome d'hydrogène ou un groupe alkyle ayant 1, 2, 3 ou 4 atomes de carbone;
R(99) et R(10) représentant, indépendamment l'un de l'autre, un atome d'hydrogène ou un groupe alkyle ayant 1 ou 2 atomes de carbone, ou un groupe alkylène(C₂-C₃)-NR(11)R(12);
R(11) et R(12) représentant, indépendamment l'un de l'autre, un atome d'hydrogène ou le groupe méthyle ou éthyle;
ou
R(99) et R(10) représentant ensemble 5 ou 6 groupes méthylène, dont un groupe CH₂ peut être représenté par -NH- ou -N-CH₃;
ou
R(8) représentant un groupe
SOₐ[NR(98)]_{b}NR(95)-C[=N-R(94)]-NR(93)R(92);
R(95) représentant un atome d'hydrogène;
R(92), R(93) et R(94) représentant, indépendamment les uns des autres, un atome d'hydrogène ou un groupe alkyle ayant 1, 2, 3 ou 4 atomes de carbone;
ou
R(1), R(2) et R(3) représentent, indépendamment les uns des autres, un atome d'hydrogène ou un groupe alkyle ayant 1, 2, 3 ou 4 atomes de carbone;
ou
R(2) représente un groupe -O-4-[CH₂-CH(NR(21)R(22))-(C=0)R(20)]-phényle;
R(21) et R(22) représentant, indépendamment l'un de l'autre, un atome d'hydrogène ou un groupe méthyle, alcanoyle en C₁-C₅, alcoxy(C₁-C₅)-carbonyle, benzyle ou benzyloxycarbonyle;
R(20) représentant un groupe -OR(23) ou -NR(23)R(24);
R(23) et (24) représentant, indépendamment l'un de l'autre, un atome d'hydrogène ou un groupe alkyle en C₁-C₄;
ou
R(2) représente le groupe imidazolyle qui est relié par C ou N; ou
R(2) représente un groupe -SR(25) ou -OR(25);
R(25) représentant un groupe pyridyle, quinolinyle ou isoquinolinyle, qui dans chaque cas est non substitué ou porte un substituant choisi dans l'ensemble constitué par F, Cl, CF₃, CH₃ et le groupe méthoxy;
ou
R(2) représente un groupe -SR(28) ou -OR(28);
R(28) représentant un groupe pyridyl-N-oxyde, quinolinyl-N-oxyde ou isoquinolinyl-N-oxyde, qui sont chacun non substitués ou substitués par un substituant choisi dans l'ensemble constitué par F, Cl, CF₃, CH₃ et le groupe méthoxy;
ou
R(1) représente un atome d'hydrogène, F, Cl, CF₃, R(31)SO₂- ou R(45)R(46)N-SO₂;
R(31) représentant le groupe méthyle ou -CF₃;
R(45) et R(46) représentant, indépendamment l'un de l'autre, un atome d'hydrogène ou le groupe méthyle;
ou
R(2) représente un groupe R(51)-A-G-O-;
R(51) représentant un groupe -NR(52)R(53);
R(52) et R(53) représentant, indépendamment l'un de l'autre, un atome d'hydrogène ou un groupe alkyle ayant 1 ou 2 atomes de carbone; ou
R(52) et R(53) représentant un groupe C_{α}H_{2α} ,
α étant 5 ou 6;
un atome de carbone du groupe C_{α}H_{2α} pouvant être remplacé par NR(56);
R(56) représentant un atome d'hydrogène ou le groupe méthyle;
ou
R(51) représentant le groupe imidazolyle, pyridyle, quinolinyle ou isoquinolinyle;
A représentant CₑH₂ₑ;
e étant 0, 1, 2 ou 3;
dans le groupe CₑH₂ₑ, un atome de carbone pouvant être remplacé par -O-, -CO- ou -SOᵣ-;
r étant 0 ou 2;
G représentant un radical phénylène 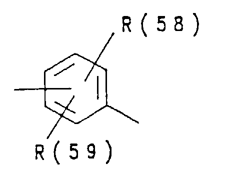 R(58) et R(59) représentant, indépendamment l'un de l'autre, un atome d'hydrogène ou le groupe méthyle, F, Cl, CF₃ ou un groupe -SO₂-R(60);
R(60) représentant un groupe méthyle ou NR(61)R(62);
R(61) et R(62) représentant, indépendamment l'un de l'autre, un atome d'hydrogène ou le groupe méthyle;
ou
R(2) représente un groupe -CF₂R(64), -CF[R(65)][R(66)], -CF(CF₃)[R(65)], -C(CF₃)=CR(65)R(66);
R(64) représentant un groupe alkyle ayant 1, 2, 3 ou 4 atomes de carbone, ou cycloalkyle ayant 5 ou 6 atomes de carbone;
R(65) et R(66) représentant, indépendamment l'un de l'autre, un atome d'hydrogène ou un groupe alkyle ayant 1, 2, 3 ou 4 atomes de carbone;
ou
R(1), R(2) et R(3) représentent, indépendamment les uns des autres, un groupe -OR(67) ou -NR(67)R(68);
R(67) et R(68) représentant, indépendamment l'un de l'autre, un atome d'hydrogène ou le groupe méthyle ou éthyle; ou
R(67) et R(68) représentant ensemble 4 ou 5 groupes méthylène, dont un groupe CH₂ peut être remplacé par un atome d'oxygène ou par -NH- ou -NCH₃;
R(4) et R(5) représentent, indépendamment l'un de l'autre, un atome d'hydrogène ou un groupe alkyle ayant 1, 2, ou 3 atomes de carbone, F, Cl ou -CF₃;
R(6) et R(7) représentent, indépendamment l'un de l'autre, un atome d'hydrogène ou le groupe méthyle;
X représente un atome d'oxygène ou NR(72);
R(72) représentant un atome d'hydrogène ou le groupe méthyle.

5. Composé de formule I selon la revendication 1, choisi dans l'ensemble constitué par les composés suivants:
4-(3-pyridyloxy)-3-trifluorométhylbenzyloxycarbonylguanidine dichlorhydrate,
4-(6-quinaldinyloxy)-3-méthylsulfonylbenzyloxycarbonylguanidine dichlorhydrate,
4-(6-quinaldinyloxy)-3-trifluorométhylbenzyloxycarbonylguanidine dichlorhydrate,
4-isopropyl-3-méthylsulfonylbenzyloxycarbonylguanidine chlorhydrate,
3-isopropylbenzyloxycarbonylguanidine chlorhydrate,
2-chloro-5-trifluorométhylbenzyloxycarbonylguanidine chlorhydrate,
4-(6-quinolinyloxy)-3-méthylsulfonylbenzyloxycarbonylguanidine dichlorhydrate,
3-bromo-5-fluorobenzylaminocarbonylguanidide chlorhydrate,
3,5-diméthylbenzylaminocarbonylguanidide chlorhydrate,
2-fluorobenzylaminocarbonylguanidide chlorhydrate,
3-fluorobenzylaminocarbonylguanidide chlorhydrate,
2,6-difluorobenzylaminocarbonylguanidide chlorhydrate,
2,5-difluorobenzylaminocarbonylguanidide chlorhydrate,
3-fluoro-5-trifluorométhylbenzylaminocarbonylguanidide chlorhydrate,
4-diméthylaminobenzylaminocarbonylguanidide chlorhydrate,
3,5-difluorobenzylaminocarbonylguanidide chlorhydrate,
3-méthylbenzylaminocarbonylguanidide chlorhydrate,
N-3,5-difluorobenzyl-N-méthylaminocarbonylguanidide chlorhydrate,
3,5-difluorobenzyloxycarbonylguanidide chlorhydrate,
2,5-difluorobenzyloxycarbonylguanidide chlorhydrate,
2,3,6-trifluorobenzyloxycarbonylguanidide chlorhydrate,
N-(3,4-dichlorobenzyl)-N-méthylaminocarbonylguanidine chlorhydrate,
N-(2-chloro-5-trifluorométhylbenzyl)-N-méthylaminocarbonylguanidine chlorhydrate,
N-méthyl-N-(3-méthylsulfonyl-4-isopropylbenzyl)aminocarbonylguanidine méthanesulfonate,
N-(4-fluoro-3-trifluorométhylbenzyl)-N-méthylaminocarbonylguanidine chlorhydrate,
4-isopropyl-3-méthylsulfonylbenzyloxycarbonylguanidine chlorhydrate,
2-chloro-5-trifluorométhylbenzyloxycarbonylguanidine chlorhydrate,
3-isopropylbenzyloxycarbonylguanidine chlorhydrate,
4-(6-quinolyloxy)-3-méthylsulfonylbenzyloxycarbonylguanidine chlorhydrate et
3-trifluorométhyl-4-méthoxybenzyloxycarbonylguanidine chlorhydrate.

6. Procédé pour la préparation d'un composé de formule I selon la revendication 1, caractérisé en ce que l'on fait réagir avec de la guanidine un composé de formule IV 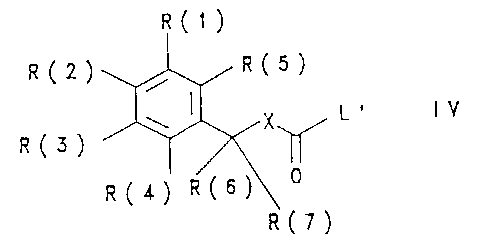 dans laquelle R(1) à R(7) et X ont les significations données dans la revendication 1, et dans laquelle L' représente un atome de chlore ou le groupe éthoxy, isobutoxy, benzotriazol-1-oxy ou 1-imidazolyle.

7. Utilisation d'un composé I selon la revendication 1, pour la fabrication d'un médicament destiné au traitement d'arythmies.

8. Utilisation d'un composé I selon la revendication 1, pour la fabrication d'un médicament destiné au traitement ou à la prophylaxie de l'infarctus du myocarde.

9. Utilisation d'un composé I selon la revendication 1, pour la fabrication d'un médicament destiné au traitement ou à la prophylaxie de l'angine de poitrine.

10. Utilisation d'un composé I selon la revendication 1, pour la fabrication d'un médicament destiné au traitement ou à la prophylaxie d'états ischémiques du coeur.

11. Utilisation d'un composé I selon la revendication 1, pour la fabrication d'un médicament destiné au traitement ou à la prophylaxie d'états ischémiques du système nerveux périphérique et central et de l'apoplexie cérébrale.

12. Utilisation d'un composé I selon la revendication 1, pour la fabrication d'un médicament destiné au traitement ou à la prophylaxie d'états ischémiques d'organes périphériques et des membres.

13. Utilisation d'un composé I selon la revendication 1, pour la fabrication d'un médicament destiné au traitement d'états de choc.

14. Utilisation d'un composé I selon la revendication 1, pour la fabrication d'un médicament destiné à l'utilisation dans des opérations chirurgicales et des greffes d'organes.

15. Utilisation d'un composé I selon la revendication 1, pour la fabrication d'un médicament destiné à la conservation et au stockage de transplants pour des opérations chirurgicales.

16. Utilisation d'un composé I selon la revendication 1, pour la fabrication d'un médicament destiné au traitement de maladies dans lesquelles la prolifération cellulaire représente une cause primaire ou secondaire.

17. Médicament, contenant une quantité efficace d'un composé I selon une ou plusieurs des revendications 1 à 3.
